(19) Europäisches Patentamt
European Patent Office
Office européen des brevets

(11) **EP 1 601 798 B1**

(12) **FASCICULE DE BREVET EUROPEEN**

(45) Date de publication et mention
de la délivrance du brevet:
**01.08.2012 Bulletin 2012/31**

(21) Numéro de dépôt: **04719497.2**

(22) Date de dépôt: **11.03.2004**

(51) Int Cl.:
*C12Q 1/68* (2006.01)

(86) Numéro de dépôt international:
**PCT/FR2004/000586**

(87) Numéro de publication internationale:
**WO 2004/083225 (30.09.2004 Gazette 2004/40)**

(54) **BIOPUCES A SONDES ET LEURS METHODES D'UTILISATION**

SONDEN-BIOCHIPS UND VERFAHREN ZUR VERWENDUNG DAVON

PROBE BIOCHIPS AND METHODS FOR USE THEREOF

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL LT LV MK**

(30) Priorité: **13.03.2003 FR 0303112**

(43) Date de publication de la demande:
**07.12.2005 Bulletin 2005/49**

(73) Titulaire: **Genewave SAS**
**75011 Paris (FR)**

(72) Inventeurs:
• **VALAT, Christophe**
**F-94350 Villiers-Sur-Marne (FR)**
• **LE DOUGUET, Cécile**
**F-92400 Courbevoie (FR)**

(74) Mandataire: **Gallois, Valérie et al**
**Cabinet BECKER & ASSOCIES**
**25, rue Louis Le Grand**
**75002 Paris (FR)**

(56) Documents cités:
EP-A2- 0 745 690    CN-A- 1 363 689
CN-A- 1 373 228    US-A- 5 118 801
US-A- 5 989 823    US-A1- 2002 102 568
US-A1- 2002 110 826    US-A1- 2003 032 051

• WEI FANG ET AL: "Achieving differentiation of single-base mutations through hairpin oligonucleotide and electric potential control." BIOSENSORS & BIOELECTRONICS. ENGLAND 15 AUG 2003, vol. 18, no. 9, 15 août 2003 (2003-08-15), pages 1149-1155, XP002276335 ISSN: 0956-5663
• KABOEV ET AL.: "PCR hot start using primers with the structure of molecular beacons (hairpin-like structure)", NUCLEIC ACIDS RESEARCH, vol. 28, no. 21, 2000, page E94,
• REBOWSKI ET AL.: "Antisense hairpin loop oligonucleotides as inhibitors of expression of mutidrug resistance-associated protein 1", ACTA BIOCHIMICA POLONICA, vol. 48, no. 4, 2001, page 1061-1076,

**Description**

L'invention concerne les domaines d'utilisation des sondes polynucléotidiques non marquées, susceptibles de former des épingles à cheveux, les biopuces contenant de telles sondes ainsi que des méthodes permettant leur utilisation. La présente description décrit également les méthodes permettant de créer de telles sondes ainsi que des biopuces. Plus particulièrement, la description décrit l'utilisation de telles sondes non marquées et biopuces pour la manipulation et l'analyse de séquences polynucléotidiques et éventuellement des molécules qui leurs sont associées. La présente description décrit en outre les méthodes de préparation et d'utilisation de telles sondes et biopuces pour l'analyse de mutations, le séquençage, la détection de variants d'épissage alternatifs, l'analyse de l'expression de gènes, l'analyse des déséquilibres alléliques et des pertes d'heterozygotie, et la détection de tout acide nucléique présent dans les organismes ou résidus issus de ces organismes.

La détermination rapide et précise de l'identité et de la quantité de molécules spécifiques dans un échantillon contenant de nombreuses molécules différentes est d'un intérêt primordial dans les domaines d'application biologique et médicale. De nombreux types de sondes et de systèmes d'analyse ont été créés pour détecter des molécules spécifiques, comme par exemple des sondes et des puces destinées à détecter des variations de séquence des acides nucléiques.

Un tel type de sonde pour détecter des acides nucléiques spécifiques peut être un « interrupteur moléculaire » semblable à celui décrit dans le brevet n° US 5,118,801 fonctionnant sur le principe de l'impossibilité qu'ont les extrémités de la sonde d'interagir entre elles lorsque la portion centrale de la sonde est hybridée avec une séquence cible. Chaque interrupteur moléculaire possède 2 extrémités complémentaires qui ont une longueur d'au moins 10 nucléotides, et une portion centrale longue d'environ 15 à 115 nucléotides. En fonction des conditions du test, la sonde décrite peut adopter une conformation « fermée » ou « ouverte ». Quand la sonde est dans sa conformation fermée, les extrémités de la sonde s'hybrident l'une à l'autre pour former une « tige » avec la séquence centrale formant une « boucle ». Dans la conformation ouverte, la séquence centrale de la sonde est hybridée à une séquence cible complémentaire prédéterminée, pour laquelle la sonde est conçue. Cette conformation ouverte conduit à la dissociation des extrémités de la sonde et à l'impossibilité qu'ont ces extrémités d'interagir l'une avec l'autre. L'une ou les deux extrémités de la sonde décrite contiennent une portion d'acide nucléique fonctionnel sur le plan biologique permettant de générer de façon sélective un signal détectable indiquant l'hybridation de la sonde avec la séquence cible prédéterminée. Une portion préférée est, par exemple, un ARN qui permet une réplication exponentielle grâce à une ARN polymérase ARN dépendante, en présence de nucléotides radioactifs, incorporés dans les produits de réplication.

Un autre type de sondes, communément appelé « molecular beacon » est similaire à celui présenté ci-dessus, et est décrit dans les brevets n° US 5,925,517 et EP 0 745 690 (voir également Tyagi et al., 1996, Nat. Biotechnol. 14 : 303-308 ; Tyagi et al., 1998, Nat. Biotechnol. 16 :49-53 ; Matsuo T. 1998, Biochimica Biophysica Acta. 1379:178-184; Sokol et al. 1998, Proc. Natl. Acad. Sci. USA 95:11538-11543; Leone et al. 1998, Nucleic Acids Res. 26:2150-2155; Piatek et al. 1998, Nat. Biotechnol. 16:359-363; Kostrikis et al. 1998, Science 279:1228-1229; Giesendorf et al. 1998, Clin. Chem. 44 :482-486 ; Marras et al. 1999 Genet. Anal. 14:151-156; Vet et al. 1999, Proc. Natl. Acad. Sci. USA 96 : 6394-6399). Les « molecular beacons » sont des molécules possédant un fluorophore interne quenché dont la fluorescence est restaurée lorsqu'ils se lient à un acide nucléique cible. Ils sont conçus de façon similaire aux interrupteurs moléculaires, la partie boucle de la molécule constituant une séquence sonde complémentaire d'une molécule d'acide nucléique, et la partie tige résultant de l'appariement de séquences « bras » complémentaires, situées aux extrémités de la séquence de la sonde. Un groupement fluorescent est attaché à une extrémité d'un bras. Un groupement quencheur est attaché à l'extrémité de l'autre bras. En l'absence d'une molécule cible, ces deux groupements sont très proches l'un de l'autre, et provoquent le quenching du fluorophore. Quand la sonde est hybridée avec un acide nucléique cible qui lui est parfaitement complémentaire, le « molecular beacon » subit un changement de conformation qui force les bras de la tige à se dissocier, et conduit le fluorophore et le quencheur à s'éloigner l'un de l'autre. Ceci permet de détecter l'hybridation de la sonde avec sa molécule cible au travers de l'apparition d'une fluorescence.

Les systèmes de biopuces sont actuellement largement utilisés pour la détection et la mesure de substances spécifiques dans des échantillons complexes. Dans de tels systèmes, l'identité et la quantité d'une substance cible dans un échantillon sont déterminées par la mesure du niveau d'association de la séquence cible avec les sondes spécifiquement prévues pour cette séquence cible. Dans les technologies de biopuces à acides nucléiques, un jeu d'acides nucléiques sondes, ayant chacun une séquence définie, est immobilisé sur un support solide de façon à ce que chaque sonde occupe une position prédéterminée. Une fois le jeu de sondes immobilisées, la puce est mise en contact avec un échantillon de façon à ce que les séquences complémentaires puissent s'associer à une sonde immobilisée, par exemple en s'hybridant, s'associant ou en se liant à la sonde. Après élimination du matériel non associé, les séquences associées sont détectées et mesurées.

Les technologies des biopuces à ADN ont rendu possible le suivi des niveaux d'expression d'un grand nombre de transcrits génétiques en une seule fois. (voir par ex. : Schena et al., 1995, Science 270 :467-470 ; Lockhart et al., 1996, Nat. Biotechnology 14 :1675-1680 ; Blanchard et al., 1996, Nat. Biotechnology 14:1649; Ashby et al. Brevet U.S.

n° 5,569,588 délivré le 29 Octobre, 1996). La technologie des biopuces a également été utilisée pour séquençer, déterminer les empreintes et cartographier des macromolécules biologiques (Brevets n° US 6,270,961 délivré le 7Aout 2001 ; 6,025,136, délivré le 15 Février 2000 ; 6,018,041 délivré le 25 Janvier 2000 ; 5,871,928 délivré le 16 Février 1999; et 5,695,940, délivré le 9 Décembre 1997). Il existe deux formats principaux de biopuces à ADN. Pour l'un de ces formats, les biopuces à ADN sont préparées par dépôt de fragments d'ADN dont la taille varie de quelques dizaines de bases jusqu'à quelques kilobases sur une surface adéquate (voir par ex. : DeRisi et al., 1996, Nature genetics 14 : 457-460 ; Shalon et al., 1996, Genome Res. 6 :689-645 ; Schena et al., 1995, Proc. Natl. Acad. Sci. U.S.A. 93: 10539-11286; et Duggan et al., 1996, Nature Genetics Supplement 21 :10-14). Par exemple, dans les analyses par « blotting » comme les « dot blot » ou l'hybridation ADN/ADN sur membrane (Southern Blotting), les molécules d'acides nucléiques peuvent être préalablement séparées en utilisant par exemple une séparation selon leur taille sur un gel d'électrophorèse, suivie de leur transfert et de leur immobilisation sur une membrane filtrante telle qu'une membrane de nitrocellulose ou de nylon, puis de leur hybridation avec une séquence marquée unique (voir par ex., Nicoloso, M. et al., 1989, Biochemical and Biophysical Research Communications 159:1233-1241; Vernier, P. et al., 1996, Analytical Biochemistry 235 :11-19). Les biopuces à ADN complémentaire (ADNc) sont préparées par dépôt sur une surface adéquate de produits issus de la réaction de polymérisation en chaîne par la polymérase (polymerase chain reaction, PCR) se présentant sous la forme de fragments d'ADN complémentaires dont la taille varie entre 0,6 à 2,4 kb, provenant d'ADNcs, d'ESTs (Expressed Sequence Tag), etc.. (voir par ex. : DeRisi et al., 1996, Nature Genetics 14:457-460; Shalon et al., 1996, Genome Res. 6 :689-645 ; Schena et al., 1995, Proc. Natl. Acad. Sci. U.S.A. 93:10539-11286; et Duggan et al., 1996, Nature Genetics Supplement 21 :10-14). L'autre format de biopuces à ADN, appelée puces à haute densité d'oligonucléotides contient des milliers d'oligonucléotides complémentaires de séquences définies, occupant des localisations connues sur la surface utilisée. Ces oligonucléotides sont synthétisés *in situ* sur la surface en utilisant par exemple des techniques photo lithographiques (voir par ex. : Fodor et al., 1991, Science 251 :767-773 ; Pease et al., 1994, Proc. Natl. Acad. Sci. U.S.A. 91 :5022-5026 ; Lockhart et al., 1996, Nature Biotechnology 14 :1675-1680 ; Brevets U. S. n° 5,578,832; 5,556,752; 5,510,270; 5,445,934; 5,744,305; et 6,040,138). Les méthodes de fabrication de biopuces utilisant les technologies de jet d'encre pour la synthèse *in situ* d'oligonucléotides sont également bien connues par l'homme de l'art (voir par ex : Blanchard, demande de brevet international WO 98/41531, publiée le 24 Septembre 1998; Blanchard et al., 1996, Biosensors and Bioelectronics, 11 :687-690 ; Blanchard, 1998, dans Synthetic DNA Arrays in Genetic Engineering, Vol.20, J.K. Setlow, Ed., Plenum Press, New York , pages 111-123).

Malgré ces avancées technologiques, il existe toujours un besoin pour des biopuces améliorées et des nouvelles méthodes utiles pour la caractérisation à haut débit de gènes et des produits de ces gènes (par exemple, la détection de mutations ponctuelles comme les « Single Nucleotide Polymorphism » (SNP), les insertions et/ou délétions de nucléotides uniques ou multiples et successifs, le séquençage de gènes, l'analyse de l'expression des gènes, la détection d'épissage alternatif, l'analyse de perte d'hétérozygotie, ainsi que la détection de tout organisme ou résidu d'organismes contenant des nucléotides. Parmi les problèmes rencontrés avec les technologies actuelles de biopuces, il faut mentionner la taille et le positionnement des sondes, l'absence de conditions uniques pour réaliser l'analyse avec toutes les sondes, le besoin de sondes redondantes dues au contenu variable en bases G/C conduisant à des pertes d'espace sur la biopuce, la nécessité de multiples oligonucléotides pour la PCR des séquences détectées, ainsi que la nécessité de marquer chaque séquence cible. La présente invention répond à chacun de ces besoins.

RESUME DE L'INVENTION

La présente invention concerne le kit selon les revendications 1 à 12 et les méthodes selon les revendications 13 à 22.

La présente invention met en jeu des sondes nucléiques susceptibles de former des épingles à cheveux non marquées, des biopuces contenant une pluralité de ces sondes, et des méthodes permettant leur utilisation. Chaque sonde polynucléotidique susceptible de former une épingle à cheveux est constituée d'une séquence d'acide nucléique simple brin non marquée, comprenant une séquence spécifique d'une cible, bordée de chaque coté par des extrémités constituées de deux séquences complémentaires l'une par rapport à l'autre. Chaque sonde peut adopter deux conformations. L'une de ces conformations est dite «fermée» ou en «épingle à cheveux», et est caractérisée par l'hybridation des deux extrémités complémentaires de la sonde, qui forme la « tige », alors que la séquence spécifique de la cible forme une « boucle » (la séquence spécifique de la cible ne peut s'hybrider avec aucune autre partie de la sonde). Cette conformation fermée exclut l'hybridation d'autres séquences sur les bras (extrémités de la sonde formant la tige). La deuxième conformation est dite « ouverte » et est caractérisée par l'hybridation de la séquence cible de la sonde formant la boucle avec la molécule cible, cette hybridation forçant les deux extrémités complémentaires à se dissocier l'une de l'autre. Cette conformation ouverte empêche les deux extrémités complémentaires de s'associer entre elles et permet à d'autres séquences, complémentaires des extrémités, de s'hybrider audites extrémités. L'hybridation de la molécule cible est détectée grâce à l'utilisation d'une molécule « rapporteur » qui est prévue spécifiquement pour s'hybrider avec au moins l'une des extrémités libres de la sonde décrite ici.

Chaque sonde décrite ici peut être prévue pour discriminer une molécule cible spécifique qui présente une variation d'un nucléotide (par exemple, une substitution, une délétion ou une insertion d'un nucléotide), ou des variations de plusieurs nucléotides séparés par de courtes séquences, ou encore des délétions ou des insertions étendues. Chaque sonde en épingle à cheveux décrite ici étant conçue pour s'ouvrir quand elle s'hybride avec la cible pour laquelle elle a été spécifiquement préparée, ce qui permet de distinguer et détecter les cibles. Les capacités de discrimination permettent à de multiples séquences d'être criblées dans une seule solution. Dans un mode préféré de réalisation, une biopuce comprend deux ou plusieurs sondes telles que décrites ici, tous les hybrides parfaits formés entre les séquences spécifiques de la cible des sondes et les molécules cibles ont une température de fusion égale à plus ou moins 4˚C près. De préférence, les températures de fusion des hybrides parfaits formés entre les séquences spécifiques de la cible des sondes telles que décrites ici et les molécules cibles sont égales à 3˚C près. De façon encore plus préférée, les températures de des hybrides parfaits formés entre les séquences spécifiques de la cible des sondes telles que décrites ici et les molécules cibles sont égales à 1˚C près.

La longueur de la séquence spécifique de la cible de chaque sonde décrite ici est comprise entre 6 et 30 nucléotides, de manière préférée entre 10 et 25, de manière encore plus préférée entre 15 et 20 nucléotides. De préférence, chacune des deux extrémités composant la tige de chaque sonde en épingle à cheveux décrite ici est constituée d'une séquence de moins de 10 nucléotides de long, de préférence entre 5 et 9 nucléotides de long.

La description décrit ainsi une sonde nucléique susceptible de former une épingle à cheveux, non marquée, comprenant :

(a) une séquence spécifique d'une cible, de 6 à 30 nucléotides de long ;
(b) un premier bras de moins de 10 nucléotides de long, positionné en 5' de ladite séquence spécifique d'une cible ; et
(c) un deuxième bras de moins de 10 nucléotides de long, positionné en 3' de ladite séquence spécifique d'une cible,

ladite séquence spécifique d'une cible n'est complémentaire d'aucune autre partie de ladite sonde; et en outre, le premier bras et le second bras sont parfaitement complémentaires, l'un avec l'autre.

Dans un mode de réalisation préféré, la séquence spécifique de la cible a une longueur de 10-25 nucléotides, plus particulièrement 15-20 nucléotides. Lorsqu'une molécule cible est hybridée avec la séquence spécifique de la cible, la sonde adopte une conformation « ouverte » et une molécule «rapporteur» peut s'hybrider avec le premier ou le second bras. La molécule «rapporteur» comprend moins de 10 nucléotides parfaitement complémentaires avec la séquence d'acide nucléique du premier bras ou du second bras. La molécule «rapporteur» comprend un marqueur détectable. Le marqueur détectable peut être un analogue de nucléotide, un marqueur fluorescent, de la biotine, de l'imminobiotine, un antigène, un co-facteur, du dinitrophenol, de l'acide lipoïque, un composé oléfinique, un polypeptide, une molécule riche en électrons, un enzyme ou un isotope radioactif. De préférence, le Dtm (différence entre la température de fusion (Tm) de l'hybride parfait formé entre la séquence spécifique de la cible de la sonde et la molécule cible et la température de fusion (Tm) de l'hybride formé entre le premier bras et le second bras de la sonde) est supérieur à 10, plus particulièrement est égale à 15. Alternativement le Dtm est inférieur à 10.

La description décrit également une composition comprenant au moins une sonde telle que décrite ici et au moins une molécule « rapporteur ».

La description décrit également une biopuce de sondes susceptibles de former des épingles à cheveux comprenant :

(a) un support ; et
(b) au moins deux sondes telles que décrites ici.

Dans un mode de réalisation préféré, la sonde est attachée à un support. Facultativement, ladite sonde comprend en outre un espaceur et est attachée audit support par ledit espaceur. Le support peut consister en une surface de verre fonctionnalisée, une surface de plastique fonctionnalisée, une surface métallique fonctionnalisée, une surface conductrice en métal, une surface conductrice en plastique, un support poreux, un métal poreux, une fibre optique, un support dérivé d'une fibre de verre, du dioxyde de silicium, une membrane lipidique fonctionnelle, un liposome ou une membrane de filtration. Plus particulièrement, la surface de plastique fonctionnalisée peut être du polystyrène ; la surface de métal fonctionnalisée peut être du platine, de l'or ou du nickel ; la surface de plastique conductrice peut être un support à base de carbone, ce support pouvant être un polymère ; et, le support poreux peut être du verre. Dans un mode de réalisation particulier, les premiers bras de chaque sonde possèdent une séquence identique. Ainsi, une même molécule «rapporteur» peut s'hybrider avec chaque sonde. De préférence, tous les hybrides parfaits formés entre les séquences spécifiques de la cible des sondes et les molécules cibles ont une température de fusion égale, à plus ou moins 4˚C près, de préférence à plus ou moins 1 ˚C près. De préférence, la différence entre la température de fusion de l'hybride formé entre la séquence spécifique de la cible de chaque sonde et la molécule cible, et la température de fusion pour l'association entre la séquence spécifique de la cible et une molécule pour laquelle la séquence spécifique de la cible de la sonde n'a pas été conçue, est supérieure ou égale à 5˚C, de préférence 8˚C. Dans un mode de réalisation particulier, les Dtm d'au moins deux sondes sont égaux, à plus ou moins 1˚C près.

La présente description décrit également un système d'adressage universel dans lequel une biopuce standardisée constituée de sondes immobilisées en épingle à cheveux telle que décrite ici est utilisée avec des sondes en épingle à cheveux ou linéaires non immobilisées, spécifiques de molécules cibles et permettant la détection de ces molécules cibles. De préférence, les sondes non-immobilisées sont en épingle à cheveux.

La description décrit ainsi un système d'adressage universel non marqué comprenant :

(a) au moins deux premières sondes non marquées comprenant :

(i) une séquence spécifique d'une cible, de 6 à 30 nucléotides de long ;
(ii) une séquence dite étiquette de 10 à 50 nucléotides de long connectée à l'extrémité 5' de la séquence spécifique de la cible,

ladite séquence spécifique d'une cible n'étant complémentaire d'aucune autre partie de ladite sonde non marquée ;
(b) une biopuce telle que décrite ici.

De préférence, lesdites premières sondes non marquées comprennent :

(i) une séquence spécifique d'une cible, de 6 à 30 nucléotides de long ;
(ii) un premier bras de moins de 10 nucléotides de long, positionné en 5' de ladite séquence spécifique d'une cible ;
(iii) un second bras de moins de 10 nucléotides de long, positionné en 3' de ladite séquence spécifique d'une cible ; et
(iv) une séquence dite étiquette de 10 à 50 nucléotides de long connectée au premier bras ou au second bras,

ladite séquence spécifique d'une cible n'étant complémentaire d'aucune autre partie de ladite sonde non marquée ; et en outre, le premier bras et le second bras étant parfaitement complémentaires.

De préférence, tous les premiers bras des premières sondes possèdent une séquence identique. Ainsi, une même molécule «rapporteur» peut s'hybrider avec l'une ou l'autre des premières et secondes sondes.

De préférence, la biopuce comprend au moins deux secondes sondes susceptibles de former une épingle à cheveux comprenant :

(i) une séquence spécifique d'une seconde cible, de 6 à 30 nucléotides de longueur ;
(ii) un second premier bras de ladite seconde sonde de moins de 10 nucléotides de long et positionné en 5' de ladite seconde séquence spécifique de ladite cible;
(iii) un second deuxième bras de ladite seconde sonde de moins de 10 nucléotides de long et positionné en 3' de ladite seconde séquence spécifique de ladite cible ; et
(iv) un espaceur connectant le premier ou le second bras audit support ,

ladite séquence spécifique de ladite cible de la deuxième sonde n'étant complémentaire d'aucune autre partie de ladite seconde sonde; ledit premier bras et ledit second bras de ladite seconde sonde étant parfaitement complémentaires l'un de l'autre ; et en outre, ladite séquence étiquette d'une des premières sondes complétée de la séquence du bras connecté à cette étiquette étant complémentaire de ladite seconde séquence cible d'une des deuxièmes sondes.

La description décrit également des méthodes permettant de préparer des sondes et des biopuces telles que décrites ici.

La description décrit en outre des méthodes d'utilisation de sondes et de biopuces telles que décrites ici. Des aspects particuliers concernent l'analyse de mutations, le séquençage, l'analyse de l'expression des gènes, l'analyse de la perte d'heterozygotie et de déséquilibre allélique, et la détection de tout organisme ou résidu d'organisme contenant des acides nucléiques.

La description décrit ainsi une méthode de détection d'acide nucléique comprenant :

(a) la mise en contact *ex vivo* d'un échantillon d'acide nucléique avec une biopuce comprenant au moins deux sondes telles que décrites ici; et
(b) la détection d'un signal provenant d'au moins une desdites sondes de la biopuce ayant adopté une conformation ouverte à la suite du contact établi lors de l'étape (a).

Elle décrit également une méthode de détection *ex vivo* d'un variant génétique dans un échantillon d'acide nucléique comprenant :

(a) la mise en contact d'un échantillon d'acide nucléique avec une biopuce comprenant au moins deux sondes telles que décrites ici, au moins une sonde de la biopuce étant une sonde spécifique d'un variant génétique, possédant une région boucle parfaitement complémentaire dudit variant génétique, et

(b) la détection d'un signal provenant de la sonde spécifique du variant génétique, le signal détecté à l'étape (b) indiquant la présence du variant génétique dans l'échantillon d'acide nucléique.

De préférence, le variant génétique détecté est un polymorphisme d'un seul nucléotide.

Elle décrit en outre une méthode de détection *ex vivo* de tout organisme contenant des acides nucléiques ou de tout résidu de ces organismes comprenant :

(a) la mise en contact d'un échantillon d'acide nucléique avec une biopuce comprenant au moins deux sondes telles que décrites ici, au moins une sonde étant une sonde spécifique des acides nucléiques d'un organisme ou de résidus de cet organisme ; et

(b) la détection d'un signal provenant de ladite sonde spécifique d'un organisme contenant des acides nucléiques,

le signal détecté dans l'étape (b) indiquant la présence de l'organisme contenant des acides nucléiques ou de résidus de cet organisme.

De préférence, l'organisme contenant des acides nucléiques est un virus ou une bactérie.

La description décrit également une méthode de détection *ex vivo* d'un produit d'épissage alternatif d'un gène dans un échantillon d'acides nucléiques comprenant :

(a) la mise en contact d'un échantillon d'acide nucléique avec une biopuce à sondes comprenant au moins deux sondes telles que décrites ici, au moins une sonde de la biopuce étant une sonde spécifique d'un exon du gène ou de la jonction de deux exons ; et

(b) la détection d'un signal provenant de ladite sonde spécifique de l'exon ou de la jonction de deux exons,

le signal détecté dans l'étape (b) indiquant la présence du produit d'épissage alternatif du gène dans l'échantillon d'acides nucléiques.

De préférence, l'échantillon d'acide nucléique comprend de l'ARNm ou de l'ADNc.

Elle décrit en outre une méthode de séquençage *ex vivo* d'un oligonucléotide comprenant :

(a) la mise en contact d'un échantillon contenant l'oligonucléotide avec une biopuce comprenant au moins deux sondes telles que décrites ici ; et

(b) la détection d'un signal provenant d'au moins une sonde de ladite biopuce ;

le signal détecté dans l'étape (b) étant utilisé pour déterminer la séquence de l'oligonucléotide.

La détection de l'étape (b) des méthodes ci-dessus mentionnées est obtenue en utilisant une molécule «rapporteur» marquée avec un marqueur détectable. L'échantillon de l'étape (a) peut être marqué au préalable avec un marqueur détectable.

La description décrit également une méthode de détection *ex vivo* des déséquilibres alléliques et pertes d'hétérozygoties dans un échantillon d'acide nucléique comprenant :

(a) l'amplification d'au moins une région d'ADN chromosomique de type microsatellite au moyen d'une paire d'amorces à partir d'au moins deux échantillons d'acides nucléiques issus de tissus ou fluides biologiques, au moins un des échantillons provenant de cellules ou de tissus ne présentant pas de déséquilibre allélique ou perte d'hétérozygotie, et chaque tissu ou fluide étant marqué différemment au cours de l'amplification ;

(b) l'élimination de ces amorces après l'amplification ;

(c) la mise en contact desdits produits d'amplification avec une biopuce à sondes comprenant au moins deux sondes telles que décrites ici, dont au moins une sonde de la biopuce étant complémentaire d'une amorce utilisée pour amplifier ladite région d'ADN chromosomique; et

(d) la détection des signaux provenant d'au moins une sonde de ladite biopuce ; les signaux détectés dans l'étape (d) étant utilisés pour déterminer la présence d'un déséquilibre allélique ou d'une perte d'hétérozygotie dans un des échantillons d'acides nucléiques.

La description décrit des kits comprenant des biopuces de sondes telles que décrites ici, pour la détermination de la présence ou de l'absence de diverses molécules dans des échantillons biologiques ou médicaux. Chaque kit contient au moins une biopuce de sondes telle que décrite ici, et un ou plusieurs réactifs additionnels nécessaires à la détection de molécules cibles spécifiques. Le kit peut comprendre en outre un jeu de sondes telles que décrites ici non-immobilisées. Le kit comprend en outre une molécule «rapporteur».

DESCRIPTION DES FIGURES

FIG.1 représente des caractéristiques des sondes en épingle à cheveux.

FIG.2 représente les différentes conformations d'une sonde en épingle à cheveux immobilisée.

FIG.3 représente la façon dont un signal peut être généré par l'hybridation d'une sonde en épingle à cheveux avec la séquence cible.

FIG.4 représente la détection d'une cible avec une sonde en épingle à cheveux et un système de marquage utilisant une polymérase.

FIG.5 représente un système d'adressage universel

FIG.6 représente la séquence d'un ARN messager, d'un variant de cet ARN, et de régions de cet ARNm qui sont des cibles des sondes en épingle à cheveux.

FIG.7 représente les séquences d'acides nucléiques utilisées pour démontrer la façon dont les sondes en épingle à cheveux interagissent avec les cibles et les molécules « rapporteur ».

FIG.8 représente un graphique en barre des intensités de fluorescence après hybridation des séquences cible marquée avec des sondes en épingle à cheveux.

FIG.9 représente un graphique en barre des intensités de fluorescence après hybridation successive d'une séquence cible marquée avec des sondes en épingle à cheveux, puis avec une molécule « rapporteur ».

FIG.10 représente un graphique en barre des intensités de fluorescence après hybridation successive d'une séquence cible marquée avec des sondes en épingle à cheveux, puis avec une molécule « rapporteur », suivi de l'élimination des interactions non spécifiques. »

FIG.11 représente un graphique en barre des intensités de fluorescence après hybridation de plusieurs cibles marquées avec des sondes en épingle à cheveux.

FIG.12 représente un graphique en barre des intensités de fluorescence après hybridation de plusieurs cibles marquées avec des sondes en épingle à cheveux, dans des conditions d'hybridation plus stringentes que pour la FIG. 11.

FIG. 13 représente une méthode d'analyse de la perte d'hétérozygotie, depuis les étapes d'amplifications PCR des échantillons jusqu'à l'hybridation des produits de PCR sur la biopuce.

FIG. 14 représente l'évolution du ratio de discrimination des sondes en épingle à cheveux (Rd) en fonction de la différence de Tm entre tige et boucle (Dtm).

FIG. 15 représente l'hybridation de produits de PCR issu de l'amplification de l'exon 3 du gène PM P22 sur une biopuce en épingle à cheveux.

DESCRIPTION DETAILLEE DE L'INVENTION

La description décrit des sondes susceptibles de former des épingles à cheveux non marquées, des biopuces contenant une pluralité de sondes telles que décrites et concerne des méthodes de préparation et d'utilisation de sondes et biopuces telles que décrites. Cela est décrit en détail et illustré par des exemples dans la section ci-après.

LES SONDES SUSCEPTIBLES DE FORMER DES EPINGLES A CHEVEUX

La description décrit une biopuce de sondes susceptibles de former des épingles à cheveux comprenant :

(a) un support ; et
(b) au moins deux sondes susceptibles de former des épingles à cheveux non marquées, chaque sonde non marquée comprenant :

(i) une séquence spécifique d'une cible, de 6 à 30 nucléotides de longueur;
(ii) un premier bras de moins de 10 nucléotides de long positionné en 5' de la séquence spécifique de la cible ;
(iii) un second bras de moins de 10 nucléotides de long positionné en 3' de la séquence spécifique de la cible ;
(iv) un espaceur connectant le premier ou le second bras audit support ;

la séquence spécifique d'une cible n'étant complémentaire d'aucune autre partie de la sonde non marquée ; et ledit premier bras et ledit second bras étant parfaitement complémentaires, l'un avec l'autre, pour chacune des sondes.

Par « non-marqué » est entendu que la sonde ne comporte pas de marqueur détectable. Par exemple, la sonde ne comprend pas d'analogue de nucléotide, de marqueur fluorescent, de la biotine, de l'imminobiotine, d'antigène, de co-facteur, du dinitrophenol, de l'acide lipoïque, de composé oléfinique, de polypeptide, de molécule riche en électrons, d'enzyme, ou un isotope radioactif permettant la détection directe de la sonde.

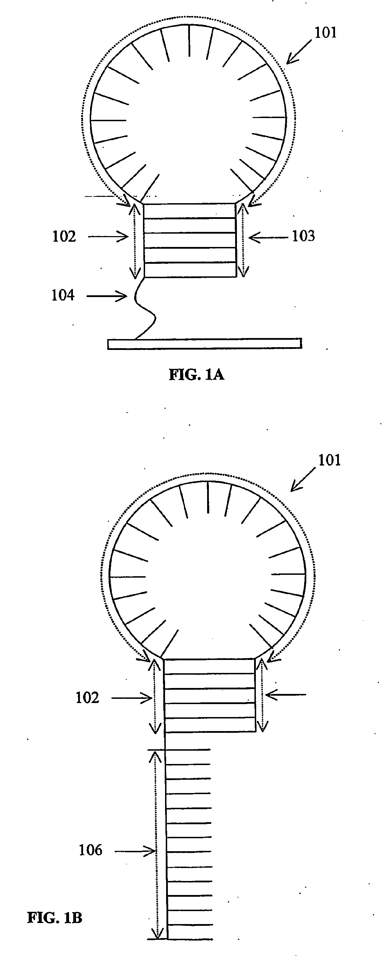

Deux exemples de sondes en épingle à cheveux non marquées telles que décrites dans ici sont illustrés dans les FIGs. 1A et 1B. Dans le cadre de cette description, une sonde en épingle à cheveux est une séquence simple brin d'acide nucléique comprenant une « boucle » 101 dont la longueur de la séquence spécifique d'une cible peut varier de 6 à 30 nucléotides, une longueur préférée variant de 10 à 25 nucléotides, et de façon encore plus préférée de 15 à 20 nucléotides, et deux extrémités, ou « bras » qui encadrent la séquence spécifique de la cible. Un bras 102 est relié à l'extrémité 3' de la séquence spécifique de la cible, et l'autre bras 103 est relié à l'extrémité 5' de la séquence spécifique de la cible. Chaque bras comprend de préférence moins de 10 nucléotides parfaitement complémentaires de ceux de l'autre bras. De façon encore plus préférée, chaque bras comprend 4 à 9 nucléotides, parfaitement complémentaires de ceux de l'autre bras. Idéalement, chaque bras comprend 6-8 nucléotides parfaitement complémentaires de ceux de l'autre bras. Si deux ou plusieurs sondes en épingle à cheveux doivent être utilisées ensemble (par exemple dans une biopuce), toutes les sondes en épingle à cheveux peuvent être conçues pour avoir des bras dont les séquences ont la même composition.

Les sondes en épingle à cheveux telles que décrites ici peuvent également comporter un « espaceur » 104. Dans le cadre de son utilisation, un espaceur correspond à une molécule qui est attachée à l'un des bras de la sonde en épingle à cheveux, et sert de moyen d'immobilisation permanent de la sonde en épingle à cheveux sur un support 105 tel que, par exemple, mais pas uniquement, une lame de verre, pour former une biopuce à sondes en épingle à cheveux. Comme indiqué dans la FIG. 1B, les sondes en épingle à cheveux telles que décrites ici peuvent en outre comporter une séquence « étiquette » 106 correspondant de préférence à 5-50 nucléotides supplémentaires, encore plus préférentiellement de 5-20 nucléotides, et qui peut être utilisée dans un système d'adressage universel. Une telle séquence étiquette peut être utilisée pour « adresser » une sonde en épingle à cheveux à un endroit précis sur une biopuce, grâce à son hybridation avec une séquence immobilisée sur la biopuce qui est parfaitement complémentaire de l'étiquette ou d'une partie de l'étiquette.

CONCEPTION DES SONDES EN EPINGLE A CHEVEUX

Les sondes en épingle à cheveux peuvent être conçues pour une utilisation avec une concentration déterminée en sels, une température déterminée et une concentration déterminée en un ou plusieurs agents chimiques additionnels tels que la formamide, le dimethylsulfoxide, le chlorure de tetramethylammonium, et des détergents. La température fixe à laquelle les sondes en épingle à cheveux peuvent être utilisées, peut être obtenue en calculant d'abord la température de fusion (« Tm ») de la séquence spécifique de la cible et de la molécule cible en utilisant la méthode de calcul de la plus proche Tm dite « nearest neighbor » avec le Logiciel Meltcalc (Voir Schütz, E. et al. 1999, Biotechniques 27 :1218-1224. ; Allawi H.T. et al. 1998, Biochemistry 37(8):2170-9; Allawi H.T. et al. 1997, Biochemistry 36 : 10581-94 ; Peyret N. et al. 1999, Biochemistry 38:3468-77) ou Oligo 6, (Molecular Biology Insights, Inc., cascade, CO).; Meltcalc, (www.meltcalc.de). Meltcalc est un logiciel se présentant sous la forme d'une feuille de tableur, permettant le calcul du point de fusion thermodynamique lors de l'hybridation d'oligonucléotides présentant ou non des mésappariements. Meltcalc donne la température de fusion d'un oligonucléotide en faisant varier les concentrations en sels et en oligonucléotides. La température de fusion préférée de l'hybride formé entre la cible et la séquence spécifique de la cible (« Tm de l'hybride formé entre la séquence spécifique de la cible et la molécule cible ») est de 60°C +/- 10°C pour des concentrations en séquences spécifiques de la cible de 100nM et des concentrations en sels de 100 nM, le sodium (NaCl) et le magnésium (MgCl) étant les sels préférés.

Quand deux ou plusieurs sondes en épingle à cheveux sont conçues pour être utilisées ensemble, il est préférable que chaque Tm de l'hybride formé entre la séquence spécifique de la cible et la molécule cible de la sonde en épingle à cheveux ait une valeur à peu près égale à celles des hybrides parfaits formés entre les séquences spécifiques des cibles et les molécules cibles de toutes les autres sondes en épingle à cheveux. De préférence, la différence de valeur des Tm des hybrides formés entre la séquence spécifique de la cible et la molécule cible est inférieure ou égale à 4°C. De façon encore plus préférée, la différence de valeur des Tm des hybrides formés entre la séquence spécifique de la cible et la molécule cible est inférieure ou égale à 3°C. Toujours de façon encore plus préférée, la différence de valeur des Tm des hybrides formés entre la séquence spécifique de la cible et la molécule cible est inférieure ou égale à 2°C. Idéalement, la différence de valeur des Tm des hybrides formés entre la séquence spécifique de la cible et la molécule cible est inférieure ou égale à 1°C. Des variations de chaque Tm des hybrides formés entre la séquence spécifique de la cible et la molécule cible peuvent être obtenues grâce à plusieurs manipulations de la séquence spécifique de la cible. Par exemple, et sans aucune limitation, la Tm des hybrides formés entre la séquence spécifique de la cible et la molécule cible peut être augmentée ou diminuée en faisant varier le nombre de nucléotides dans la séquence spécifique de la cible. Une augmentation dans le nombre de nucléotides présents dans la séquence spécifique de la cible, par augmentation du nombre de nucléotides qui s'associent à la molécule cible, augmente la Tm des hybrides formés entre la séquence spécifique de la cible et la molécule cible. A l'inverse, une diminution du nombre de nucléotides de la séquence spécifique de la cible diminue la Tm des hybrides formés entre la séquence spécifique de la cible et la molécule cible. D'autres moyens de modifier les Tm des hybrides formés entre la séquence spécifique de la cible et la molécule cible sont décrits ci-dessous.

Une fois qu'une séquence boucle a été conçue, avec une Tm de l'hybride formé entre la séquence spécifique de la cible et la molécule cible dont la valeur est située dans une zone acceptable, les bras peuvent être conçus pour être complémentaires l'un de l'autre, puis ajoutés à la séquence de la boucle. Le contenu en G/C de la tige est de préférence de 80%, avec une valeur préférée de Tm de la tige de 40˚C-80˚C. Une fois que la conception de chaque sonde est terminée, le logiciel mfold (Zuker, M. mfold-2.3. ftp://snark.wustl.edu) est utilisé pour vérifier qu'aucune autre boucle interne additionnelle n'est présente dans la sonde en épingle à cheveux, et que la sonde adopte bien une conformation en épingle à cheveux grace uniquement à l'association des nucléotides de la tige. L'absence d'homologie entre chaque boucle et toute autre séquence nucléotidique, (par exemple produit de PCR autre que celui analysé, cDNA autre que celui analysé) utilisée dans l'analyse, est ensuite vérifiée. L'alignement de toutes les sondes en épingle à cheveux avec chaque produit de PCR qui peut être utilisé dans l'analyse devrait également être effectué en utilisant des outils d'alignement tels que LALIGN (http://fasta.bioch.virginia.edu/fasta/lalign.html) ou LFASTA (http://www. 2.igh.cnrs.fr/fasta/lfasta-query.html).

Pour chaque sonde complémentaire d'une séquence à analyser, la différence entre le Tm de la boucle et celui de la tige de la sonde est calculée selon l'expression : $DTm = Tm\ s - Tm\ I$ avec $Tm\ s = Tm$ de la tige, et $Tm\ I = Tm$ de la boucle, les Tm étant déterminés comme indiqué plus haut. Plus particulièrement, le Tm de la tige correspond à la température de fusion (Tm) de l'hybride formé entre le premier bras et le second bras de la sonde. Le Tm de la boucle correspond à la différence entre la température de fusion (Tm) de l'hybride parfait formé entre la séquence spécifique de la cible de la sonde et la molécule cible.

Dans un mode de réalisation préféré, ce Dtm doit être, pour chaque sonde, supérieur à 10, avec une préférence pour une valeur voisine de 15 (par exemple, entre 12 à 17, de préférence entre 13 et 16 ou entre 14 et 16), par exemple égale à 15. Cependant, pour des applications où la sensibilité du signal doit être favorisée par rapport à la capacité de discrimination des sondes, cette valeur de Dtm peut être inférieure à 10.

Pour un ensemble de sondes devant être utilisées conjointement dans une expérience (plus particulièrement, mise en oeuvre sur une même biopuce), la distribution des valeurs de Dtm pour l'ensemble des sondes doit être de préférence centrée sur une valeur supérieure à 10, de préférence une valeur moyenne de 15 avec un écart type de 5. Cependant, pour des applications où la sensibilité du signal doit être favorisée par rapport à la capacité de discrimination des sondes, cette valeur moyenne de Dtm peut être inférieure à 10, avec un écart type de 5.

Pour deux sondes devant être utilisées conjointement pour analyser deux allèles d'une séquence (polymorphisme ou mutation), les Dtm de ces sondes doivent être les plus proches possibles. Les Dtm de deux sondes utilisées pour analyser deux allèles différents d'une séquence sont identiques, à plus ou moins 2 près, de préférence à plus ou moins 1 près. Ces valeurs de Dtm sont des valeurs préférentielles qui peuvent être acceptables pour des valeurs d'écart <1, ou >2 pour un couple de sonde, dans certains cas où la composition en base des sondes ne permet pas d'obtenir des valeurs de Dtm identiques.

## IMMOBILISATION DES SONDES EN EPINGLE A CHEVEUX ET BIOPUCES

Les sondes en épingle à cheveux peuvent être immobilisées ou incluses sur un support pour réaliser des produits, tels que notamment des biopuces à sondes en épingle à cheveux spécifiques d'une ou plusieurs molécules cibles. Les sondes à épingle à cheveux peuvent aussi être immobilisées sur une gamme de supports, tels que notamment des billes ou des particules de taille inférieure au micron. Chaque sonde à épingle à cheveux spécifique d'une cible peut être identifiée après immobilisation par une propriété physique, chimique ou optique qui différentie chaque sonde en épingle à cheveux des autres. Les sondes peuvent également être directement synthétisées sur une surface solide en utilisant un système bidimensionnel ou le système d'adressage individuel. Pour réaliser l'immobilisation des sondes en épingle à cheveux, ces sondes contiennent au moins un groupement attaché, un «espaceur» tel que décrit précédemment. Les sondes en épingle à cheveux possédant un espaceur peuvent également être immobilisées grâce à un groupement immobilisé sur le support et présentant de l'affinité pour l'espaceur.

La détection des molécules cible est de préférence mesurée à l'aide de biopuces à sondes telles que décrites ici. Une biopuce est un ensemble organisé de sites de liaisons adressables en terme de position sur un support ; par exemple un support solide où chaque sonde en épingle à cheveux est immobilisée à la surface du support. De préférence, les supports utilisés sont notamment des lames de verre modifiées ou fonctionnalisées par silanisation de façon à créer des groupements chimiques adéquats pour l'immobilisation covalente des sondes en épingle à cheveux. Ces groupements peuvent être notamment des amines primaires, des thiols, des carboxyls, ou des aldéhydes. D'autres surfaces utiles pour l'immobilisation peuvent être notamment du dioxyde de silicium, des polymères plastiques comme le polystyrène, ou des supports conducteurs de courant comme des surfaces métalliques ou du carbone vitreux. Le support peut être poreux ou non poreux. Par exemple, les sondes en épingle à cheveux peuvent être immobilisées sur des filtres ou des membranes en nitrocellulose ou en nylon. La description décrit ainsi des sondes en épingle à cheveux non marquées, caractérisée en ce que le support consiste en une surface de verre fonctionnalisée obtenue par silanisation avec un silane porteur d'un groupement permettant une liaison covalente avec des sondes aminées ou thiolées, une surface de plastique fonctionnalisée obtenue par création de groupements fonctionnels permettant une liaison covalente avec des sondes aminées ou thiolées, comme une oxydation chimique ou électrochimique, une surface métallique fonctionnalisée par création de groupements fonctionnels permettant une liaison covalente avec des sondes aminées ou thiolées, comme une oxydation chimique ou électrochimique, une surface conductrice en métal, une surface conductrice en plastique, un support poreux (du verre de préférence), un métal poreux, une fibre optique, un support dérivé d'une fibre de verre, du dioxyde de silicium, une membrane lipidique fonctionnelle, un liposome, ou une membrane de filtration. La surface de plastique fonctionnalisée est de préférence réalisée en polystyrène. La surface de métal fonctionnalisée est de préférence réalisée en platine, en or ou en nickel. La surface de plastique conductrice est de préférence un support à base de carbone. Le support à base de carbone est de préférence un polymère.

De telles méthodes d'attachement des sondes sont bien connues de l'homme de l'art (voir par exemple Cass et al.eds., 1998, Immobilized Biomolecules in Analysis, A practical approach, Ed. Oxford University Press, Great Clarendon Street, Oxford)

Les biopuces à sondes en épingle à cheveux peuvent être faites en utilisant toutes les méthodes connues de l'art antérieur. Cependant, les biopuces produites présentent généralement certaines caractéristiques. Les biopuces à épingle à cheveux décrites ici sont reproductibles en terme de fabrication, permettant la production de multiples copies d'une biopuce donnée, et leur comparaison. L'immobilisation de sondes en épingle à cheveux sur une biopuce peut être obtenue grâce à un transfert de fluide avec un « spotter » conventionnel, ou par transfert électrocinétique, ou par électropolymérisation d'oligonucléotides électroactifs, ou grâce à tout moyen de liaison chimique directe de sondes sur une surface, ou d'inclusion de la sonde dans un support solide, ou grâce à une synthèse *in situ* via des procédés de photolithographie utilisant un jeu préfabriqué de masques (utilisant des bases modifiées adaptées à ce procédé). Les biopuces décrites ici sont réalisées avec des matériaux stables dans les conditions utilisées pour l'hybridation d'acides nucléiques. Les biopuces sont généralement de petite taille, par exemple de 1 cm$^2$ à 25 cm$^2$, de préférence de 1 à 3 cm$^2$. Cependant, des biopuces de taille plus élevée ou plus faible sont également envisagées ici. Des biopuces de taille plus élevée peuvent être préférées pour la détermination simultanée d'un très grand nombre de cibles différentes. Dans la présente description, la densité de sondes en épingle à cheveux présente sur la biopuce peut varier. La densité peut être comprise entre plusieurs (par exemple 3, 10, 30) et jusqu'à 100 sondes différentes (c'est à dire non identiques) par cm$^2$, ou davantage.

Dans cette description, les sondes en épingle à cheveux peuvent être immobilisées sur une surface solide de façon à constituer une biopuce de sondes qui possèdent différentes spécificités pour les séquences cibles. L'immobilisation des sondes est faite de telle façon que les sondes n'inhibent pas la capacité des bras de chaque sonde à s'auto-associer, ou à s'associer avec des molécules cibles ou des réactifs utilisés lors de l'analyse. Comme mentionné précédemment, les sondes décrites ici sont conçues pour adopter deux types de structures conformationnelles en fonction des conditions de l'analyse, et de la présence ou de l'absence de séquences d'acides nucléiques complémentaires. Une des structures est la conformation « fermée » (en épingle à cheveux) pour laquelle les séquences des deux bras sont hybridées ensembles. Cette structure est obtenue dans des conditions d'analyse favorables et en l'absence d'une molécule cible qui s'associe avec la séquence spécifique de la cible de la sonde d'hybridation. La deuxième structure est la conformation « ouverte » pour laquelle les séquences des bras ne sont pas hybridées ensemble. Cette conformation peut être détectée lorsque la séquence spécifique de la cible est associée avec sa cible spécifique. Une méthode préférée pour réaliser une biopuce telle que décrite ici est obtenue par fixation des sondes décrites ici par dépôt direct ou « impression » sur des lames de verre, comme décrit de façon générale par Schena et al. 1995, Science 270 : 467-470. Cette méthode est particulièrement utile pour préparer des biopuces à ADN complémentaire (voir par ex. : DeRisi et al., 1996, Nature genetics 14 :457-460 ; Shalon et al., 1996, Genome Res. 6 :639-645 ; Schena et al., 1995, Proc. Natl. Acad. Sci. U.S.A. 93:10539-11286). D'autres méthodes de fabrication de biopuces par exemple en utilisant des masques peuvent aussi être utilisées (Maskos and Southern, 1992, Nucl. Acids. Res. 20:1679-1684). En principe, et comme mentionné plus haut, tout type de biopuces comme par exemple les « dots blots » pratiqués par hybridation sur une membrane de nylon peut être utilisé (voir Sambrook et al., eds., 1989, Molecular Cloning : A Laboratory Manual, Vols. 1-3, 2nd Ed. Cold Spring Harbor Laboratory, Cold Spring Harbor, New York).

Les biopuces décrites ici peuvent être fabriquées grâce à un dispositif d'impression jet d'encre permettant la synthèse d'oligonucléotides, en utilisant par exemple les méthodes et systèmes décrits par Blanchard dans le brevet international WO 98/41531, publié le 24 Septembre 1998; Blanchard et al. ,1996, Biosensors and Bioelectronics, 11 : 687-690 ; Blanchard, 1998, Synthetic DNA Arrays in Genetic Engineering, Vol. 20, J.K. Setlow, ed., Plenum Press, New York, pages 111-123 ; et le brevet U.S. n° 6,028,189. Plus spécifiquement, les sondes en épingle à cheveux de telles biopuces sont préférentiellement synthétisées par exemple sur une lame de verre, par dépôt en série de bases nucléotidiques individuelles sous la forme de « micro-gouttes » de solvant à haute tension de surface comme le carbonate de polypropylène. Les micro-gouttes ont de petits volumes (par exemple de 100pL ou moins, de préférence de 50pL ou moins) et sont séparées les unes des autres sur la biopuce (en utilisant par exemple des domaines hydrophobes) pour former des puits de tension de surface circulaire, qui définissent les positions des éléments de la biopuce (c.a d., les différentes sondes). Les sondes polynucléotidiques en épingle à cheveux peuvent être attachées de façon covalente à la surface par les extrémités 3' ou 5' du polynucléotide en épingle à cheveux.

<u>MOLECULES CIBLES</u>

Les molécules cibles pouvant être analysées par les méthodes et compositions ici décrites incluent les molécules d'acides nucléiques, en particulier des molécules d'ARN, telles que par exemple des molécules d'ARN messagers (mRNA), des molécules d'ARN ribosomaux (rRNA), des molécules d' ARNc (c'est à dire des molécules d'ARN préparées par transcription *in vitro* à partir de molécules d'ADN complémentaire) et des fragments de ces molécules. Les molécules cibles qui peuvent également être analysées par les méthodes et compositions ici décrites incluent notamment les molécules d'ADN, comme les molécules d'ADN génomique, les molécules d'ADNc, et des fragments de ces molécules incluant des oligonucléotides, des ESTs, des STSs, des séquences microsatellites, etc. D'autres molécules cibles pouvant également être analysées par les méthodes et compositions ici décrites incluent notamment les protéines, les complexes comprenant des protéines, d'autres molécules, et des complexes de ces molécules présentant de l'affinité pour l'ADN, comme par exemple les facteurs de transcription, les protéines du système de réparation de l'ADN et les anticorps anti-ADN (Fang, W. et al. 2000. Anal. Chem. 72 :3280-5 ; Bar-Ziv, R. et al. 2001. P.N.A.S. U.S.A. 98:9068-73; Hamaguchi, N. et al. 2001 Anal. Biochem. 294:126-31). Pour les facteurs de transcription, les sondes en épingle à cheveux pourraient être utilisées pour déterminer quelle séquence présumée d'affinité pour un facteur de transcription peut être conçue dans la boucle d'une sonde en épingle à cheveux et utilisée pour déterminer quelle séquence a la plus grande affinité pour le facteur de transcription.

Les molécules cibles peuvent être de toute origine. Par exemple, les molécules cibles peuvent être des molécules naturelles d'acide nucléique, comme des molécules d'ADN génomique ou extra génomique isolées d'un organisme, ou des molécules d'ARN, comme des molécules d'ARN messager isolées d'un organisme. De façon alternative, les molécules cibles peuvent être synthétisées. Ces molécules cibles peuvent être par exemple des molécules d'acides nucléiques synthétisées à l'aide d'enzymes *in vivo* ou *in vitro*, comme des molécules d'ADNc, des molécules polynucléotidiques synthétisées par PCR, des molécules d'ARN synthétisées par transcription *in vitro,* etc. Les méthodes de PCR sont bien connues de l'homme de l'Art et sont décrites par exemple dans Innis et al., Eds., 1990, PCR Protocols : A Guide to Methods and Applications, Academic Press Inc., San Diego, CA. L'échantillon de molécules cibles peut contenir par exemple des molécules d'ADN, d'ARN, ou des copolymères d'ADN et d'ARN. Les molécules cibles ici décrites peuvent correspondent à des gènes particuliers, à des transcrits de gènes particuliers, ou des fragments particuliers d'un transcrit de gène (par exemple, des séquences de certains ARNm exprimés dans des cellules ou des séquences de certains ADNc dérivés de ces séquences d'ARNm).

Les molécules cibles à analyser peuvent aussi être préparées *in vitro,* à partir d'acides nucléiques extraits de cellules. Par exemple, l'ARN peut être extrait de cellules (notamment l'ARN cellulaire total, la fraction poly(A)$^+$ des ARNm ou des fractions de ces derniers) et l'ARN messager purifié à partir de l'extrait d'ARN total. Les méthodes pour préparer l'ARN total et l'ARN poly(A)$^+$ sont bien connues de l'art antérieur et décrites de façon générale dans Sambrook et al., eds., 1989, Molecular Cloning : A Laboratory Manual, Vols. 1-3, 2nd Ed. Cold Spring Harbor Laboratory, Cold Spring Harbor, New York. L'ARN peut être extrait de cellules présentant différents types d'intérêt, par une lyse des cellules avec du thiocyanate de guanidinium, suivi d'une centrifugation en présence de CsCl, et d'une purification par affinité avec des oligo dT (Chirgwin et al.,1979, Biochemistry 18 :5294-5299). L'ARN peut également être extrait des cellules par une lyse avec du thiocyanate de guanidinium, suivie d'une purification sur des colonnes Rneasy (Qiagen). Les ADNc peuvent alors être synthétisés à partir de l'ARNm purifié en utilisant par exemple des oligo dT ou des amorces aléatoires. Les molécules cibles peuvent être des ARNc préparés à partir d'ARN messagers purifiés ou d'ARN total extraits de cellules. L'ARNc décrit ici se réfère aux ARN complémentaires à l'ARN source. Les ARN extraits peuvent être ensuite amplifiés en utilisant un procédé dans lequel des double brins d'ADNc sont synthétisés à partir des ARNs en utilisant une amorce liée à un promoteur de la polymérase à ARN dans un sens permettant de diriger la transcription d'ARN anti-sens. Les ARNs anti-sens ou ARNsc peuvent alors être transcrit à partir du deuxième brin des ADNsc en utilisant une polymérase à ARN (voir par exemple les brevets US n° 5,891,636 ; 5,716,785 ; 5,545,522 et 6,132,997). Des amorces d'oligo-dT contenant un promoteur de la polymérase à ARN peuvent être utilisées. L'ARN total peut être utilisé comme matériel de départ pour la synthèse d'ARNc. Une amorce d'oligo-dT contenant une séquence du promoteur de la polymérase T7 peut être utilisée pour initier la synthèse du premier brin d'ADNc, et des amorces aléatoires hexamériques peuvent être utilisées pour initier la synthèse du deuxième brin d'ADNc avec la transcriptase inverse MMLV. Cette réaction produit un ADNc double brin contenant le promoteur de la polymérase T7 à son extrémité 3'. Les ADNc double brins peuvent alors être transcrits en ARNc par la polymérase à ARN de T7. La concentration de la cible synthétisée peut varier de 1$\mu$M à 50 nM. La concentration de cibles synthétisées par PCR peut varier de 5 ng/$\mu$L à 50

ng/µL.

Les molécules cibles peuvent être des amorces de PCR ou être comprises dans des amorces de PCR. Ainsi, les sondes décrites ici peuvent permettre la détection d'un produit d'amplification par PCR. Plus particulièrement, cette détection constitue un excellent témoin négatif de PCR. Ces témoins négatifs sont réalisés en amplifiant une séquence en absence d'ADN génomique mais en présence des réactifs nécessaires à la PCR et d'un couple d'amorces dont au moins une porte une séquence parfaitement complémentaire de la séquence spécifique de la cible de la sonde en épingle à cheveux.

Les molécules cibles à analyser par les méthodes et compositions ici décrites peuvent éventuellement être marquées de façon à pouvoir être détectées. Par exemple, des ADNc peuvent être marqués directement, c.a.d. à l'aide d'analogues de nucléotides ou indirectement par exemple en préparant un second brin d'ADNc marqué en utilisant le premier brin comme matrice. Alternativement, l'ADNc double brin peut être transcrit en ARNc et marqué lors de sa transcription. Le marqueur de détection peut être un marqueur fluorescent, fonctionnant par exemple par incorporation d'analogues de nucléotides. D'autres marqueurs pouvant être utilisés ici peuvent inclure notamment de la biotine, de l'imminobiotine, des antigènes, des co-facteurs, du dinitrophenol, de l'acide lipoique, des composés oléfiniques, des polypeptides détectables, des molécules riches en électrons, des enzymes capables de générer un signal détectable en agissant en présence d'un support, et des isotopes radioactifs. Des isotopes radioactifs préférés incluent notamment $^{32}P$, $^{35}S$, $^{14}C$, $^{15}N$ et $^{125}I$. Les molécules fluorescentes utilisables ici incluent notamment la fluorosceine et ses dérivés, la rhodamine et ses dérivés, le rouge texas, la 5' carboxy-fluoresceine (« FAM »), la 2',7'-dimethoxy-4',5'-dichloro-6-carboxy-fluoresceine (« JOE »), la N,N,N',N'-tetramethyl-6-carboxy-rhodamine (« TAMRA »), la 6'carboxy-X-rhodamine (« ROX »), HEX, TET, IRD40, et IRD41. D'autres molécules fluorescentes additionnelles adaptées et utilisables ici incluent en outre : les cyanines (incluant notamment la Cy3, Cy3.5, et Cy5), les molécules BODIPY (incluant notamment BODIPY-FL, BODIPY-TM, BODIPY-630/650 et BODIPY-650/670), et les molécules fluorescentes de la série ALEXA (incluant notamment ALEXA-488, ALEXA-532, ALEXA-546, ALEXA-568, et ALEXA-594), ainsi que toute autre molécule fluorescente connue de l'homme de l'art. Les molécules indicatrices riches en électrons utilisables ici comprennent notamment la ferritine, l'hémocyanine et/ou de l'or colloïdal. Alternativement, les molécules cibles peuvent être marquées en complexant de façon spécifique un premier groupement sur les molécules cibles. Un second groupement lié de façon covalente à une molécule indicatrice, ayant de l'affinité pour le premier groupement peut être utilisé pour détecter les molécules cibles de façon indirecte. Dans une telle configuration, les composés utilisables pour le premier groupement incluent notamment la biotine et l'imminobiotine. Les composés utilisables comme second groupements incluent notamment l'avidine, et la streptavidine. Des molécules fluorescentes capables de s'intercaler dans la molécule d'ADN comme le bromure d'éthidium ou le Sybr Green I peuvent également être utilisées pour suivre l'accroissement de signal fluorescent lors de l'hybridation d'une cible polynucléotidique avec une sonde en épingle à cheveux ou de l'hybridation de la molécule "rapporteur" sur la sonde en épingle à cheveux (voir ci-après). Cet aspect peut être particulièrement utile pour analyser l'hybridation en temps réel.

Cependant, selon un mode préféré de réalisation de l'analyse selon l'invention, les molécules cibles ne sont pas marquées, et les marqueurs détectables décrits précédemment sont liés à une molécule « rapporteur » (voir ci-après).

## METHODES D'HYBRIDATION UTILISABLES AVEC LES SONDES EN EPINGLE A CHEVEUX

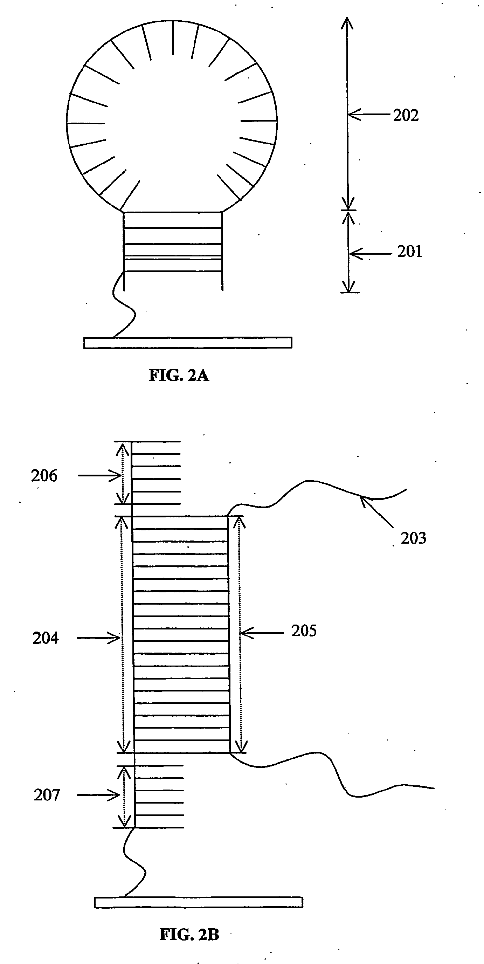

Les sondes en épingle à cheveux décrites ici sont conçues pour adopter lors du criblage de molécules cibles, deux conformations alternatives. Ces deux conformations, la conformation « fermée » et la conformation « ouverte » sont décrites dans la FIG. 2. A la fois les FIG. 2A et FIG. 2B montrent une sonde en épingle à cheveux fixée sur un support. La FIG. 2A montre une sonde en épingle à cheveux dans sa conformation fermée. En l'absence de la cible spécifique d'une sonde en épingle à cheveux, les bras de la sonde s'hybrident ensemble pour former une « tige » 201, alors que la séquence spécifique de la cible forme une « boucle » 202. La sonde ne peut adopter d'autre conformation, car les bras de la sonde en épingle à cheveux ne sont conçus que pour s'hybrider l'un à l'autre. La température fixe préférée de criblage d'une molécule cible avec une sonde en épingle à cheveux est inférieure de 5 à 10°C à celle de l'hybride formé entre la séquence spécifique de la cible et la molécule cible. Cette température peut varier selon la longueur, la position et la présence ou l'absence de mutations supplémentaires dans la structure de la boucle. Une séquence spécifique de la cible plus longue ou plus courte, ainsi qu'une tige plus longue ou plus courte, peuvent également être utilisées pour modifier la température optimale d'hybridation. Lors de l'association avec la molécule cible, la sonde en épingle à cheveux subit un changement de conformation structural. L'association d'une portion de la séquence de la boucle avec sa molécule cible (i.e., l'hybridation de la séquence spécifique de la cible avec sa séquence cible complémentaire d'acide nucléique) contraint la sonde en épingle à cheveux à s'ouvrir.

La compétition entre l'hybridation des bras complémentaires entre eux et la séquence spécifique de la cible avec sa molécule cible est un critère clef pour discriminer une association parfaite entre la séquence spécifique de la cible et sa molécule cible et une association imparfaite avec d'autres molécules cibles. Quand une sonde en épingle à cheveux est mise en présence de la molécule cible pour laquelle elle a été conçue (i.e., correspondant parfaitement), l'interaction entre la séquence spécifique de la cible et la molécule cible conduit à une dissociation de la tige, et à l'apparition de la conformation « ouverte » de la sonde. La FIG. 2B montre une sonde en épingle à cheveux dans une conformation « ouverte » résultant de l'association avec sa molécule cible 203. La séquence spécifique de la cible 204 s'hybride avec une partie 205 de la molécule cible 203. Il est prévu en outre qu'une interaction entre une sonde en épingle à cheveux et une séquence cible soit analysable avec une boucle entièrement hybridée à la molécule cible, ou à une portion de celle-ci. Cette hybridation entre la séquence spécifique de la cible 204 et la molécule cible 203 ne doit pas forcément inclure la totalité de la séquence de la molécule cible 203. Quand la séquence spécifique de la cible 204 est hybridée avec une portion 205 de la molécule cible 203, les bras de la sonde, le bras 206 du coté 5' de la séquence spécifique de la cible, et le bras 207 du coté 3' de la séquence spécifique de la cible, se dissocient et sont accessibles pour interagir avec les molécules «rapporteur».

Durant l'hybridation, l'affinité d'association d'acides nucléiques cibles avec les séquences des sondes dépend à la fois de la similarité des différentes séquences cibles dans l'échantillon, et des conditions de stringence de l'hybridation, notamment, la température d'hybridation, la concentration en sels et la présence d'agents chimiques réduisant l'affinité. La cinétique d'association dépend également des concentrations relatives des différents acides nucléiques présents dans l'échantillon. Pour des sondes polynucléotidiques ciblant (i.e., complémentaires de) des espèces de faible abondance ou ciblant des acides nucléiques ayant un grand degré de similarité de séquences (i.e., homologues), des réactions d'hybridation croisées peuvent avoir un impact significatif en terme de contamination, et fausser les résultats des mesures d'hybridation. La détection des SNP est un exemple où les hybridations croisées posent un problème, puisque la séquence à détecter (plus particulièrement le SNP à analyser) doit être différentiée parmi d'autres séquences n'ayant qu'un seul nucléotide de différence.

Plusieurs approches de l'art antérieur ont été conçues pour réduire les hybridations croisées et peuvent être utilisées dans le cadre des méthodes ici décrites. Les hybridations croisées peuvent être diminuées en régulant à la fois les conditions de stringence pendant l'hybridation et/ou pendant les lavages effectués après l'hybridation. Par exemple, des conditions de lavage très stringentes peuvent être employées pour déstabiliser tous les hybrides formés sauf les duplexes les plus stables, de façon à ce que les signaux détectés correspondent uniquement aux hybrides parfaits entre la séquence spécifique d'une sonde en épingle à cheveux et sa séquence spécifique. Un exemple de condition de stringence très élevée est une hybridation de l'ADN dans du tampon SSC 5X contenant 1% de sodium dodecyl sulfate (SDS), 1mM EDTA à 65°C, et un lavage dans du tampon SSC 0,1X, contenant 0,1% de SDS (Ausubel et al., eds, 1989, Current Protocols in MolecularBiology, Vol.I, Green Publishing Associates, Inc., and John Wiley&Sons, Inc., New York, NY, pages 2.10.3). Ces lavages peuvent être effectués à 5-10°C en dessous de la Tm de l'hybride formé entre la séquence spécifique de la cible et la molécule cible. Des conditions de stringence élevées permettent la détection de variants alléliques d'une séquence nucléotidique, par exemple 1 mésappariement tous les 10-30 nucléotides. Alternativement, des conditions de lavage « modérées » ou de « faible stringence » peuvent également être utilisées. Ces conditions modérées ou de faible stringence sont également bien connues de l'art antérieur (voir par exemple Sambrook J. et al., eds., 1989, Molecular Cloning : A Laboratory Manual, 2nd Ed., Cold Spring Harbor Laboratory Press, Cold Spring Harbor, New York, pages 9.47-9.51 et 11.55-11.61 ; Ausubel et al., eds, 1989, Current Protocols in Molecular Biology, Vol.I, Green Publishing Associates, Inc., and John Wiley&Sons, Inc., New York, pages 2.10.1-2.10.16). Des exemples de conditions de lavage de stringence modérée incluent par exemple un lavage dans du tampon SSC 0,2X, contenant 0,1% de SDS à 42°C (Ausubel et al. 1989, ci-dessus). Des exemples de conditions de lavage de faible stringence incluent par exemple un lavage dans du tampon SSC 5X ou 0,2X, contenant 0,1% de SDS, ou SSC 5X, à température ambiante (Ausubel *et al.*, 1989,ci-dessus).

Les contributions des hybridations croisées aux signaux des sondes en épingle à cheveux peuvent être suivies et supprimées en soustrayant les signaux des sondes de référence adéquates. La contribution des hybridations croisées peut être mesurée par exemple, en utilisant des sondes en épingle à cheveux ne s'hybridant avec aucune des molécules cibles présentes lors de l'analyse. Le signal non spécifique ainsi obtenu peut être soustrait du signal d'hybridation spécifique.

Selon la description, les hybridations croisées peuvent également être diminuées en utilisant des sondes en épingle à cheveux possédant entre elles des différences faibles de Tm de l'hybride formé entre la séquence spécifique de la cible et la molécule cible, c'est-à-dire des différences inférieures ou égales à 1°C. A l'opposé, lorsque les différences de Tm des hybrides formés entre la séquence spécifique de la cible et la molécule cible augmentent, i.e., sont supérieures à 1°C, les hybridations croisées deviennent un facteur plus significatif. Dans ce cas, des approches similaires à celles mentionnées précédemment peuvent être utilisées, le nombre de sondes en épingle à cheveux utilisé pour cribler chaque molécule cible peut être augmenté ou une combinaison de ces approches peuvent être utilisées.

Une ou plusieurs séquences uniques de contrôle peuvent être utilisées et la température peut être déterminée en analysant quelle sonde en épingle à cheveux s'hybride avec la séquence contrôle. Une séquence contrôle peut être complémentaire de 5-10 sondes en épingle à cheveux différentes, différant les unes des autres par la longueur de la séquence spécifique de la cible et donc par leur Tm. Par exemple, une sonde en épingle à cheveux peut être complémentaire de huit nucléotides localisés au milieu de la séquence de contrôle, et avoir une Tm de l'hybride formé entre la séquence spécifique de la cible et la molécule cible de 40˚C. Une deuxième sonde en épingle à cheveux peut être complémentaire de douze nucléotides de la séquence de contrôle, et avoir une Tm de l'hybride formé entre la séquence spécifique de la cible et la molécule cible de 45˚C. D'autres sondes en épingle à cheveux peuvent être complémentaires d'autres régions de la séquence de contrôle et avoir des Tm des hybrides formés entre la séquence spécifique de la cible et la molécule cible différentes. Il devient possible pour un utilisateur de déterminer la température de l'essai sur la base de l'identification de la sonde en épingle à cheveux qui s'hybride avec la séquence contrôle.

La description décrit ainsi une biopuce de sondes telles que décrites ici, caractérisée en ce que tous les hybrides parfaits formés entre les séquences spécifiques de la cible des sondes et les molécules cibles ont une température de fusion égale, à plus ou moins 4˚C près.

Elle décrit également une biopuce de sondes telles que décrites ici, caractérisée en ce que chaque hybride, formé entre la séquence spécifique de la cible de chaque sonde et la molécule cible, a une température de fusion égale à plus ou moins 1 ˚C près aux températures de fusion des hybrides formés entre la séquence spécifique de la cible et la molécule cible de toutes les autres sondes.

Elle décrit en outre, une biopuce de sondes telles que décrites ici, caractérisée en ce que la différence entre chaque température de fusion de l'hybride, formé entre la séquence spécifique de la cible de chaque sonde et la molécule cible, et une seconde température de fusion pour l'association entre la séquence spécifique de la cible et une molécule pour laquelle la séquence spécifique de la cible n'a pas été conçue, est supérieure ou égale à 5˚C. La différence entre chaque température de fusion de l'hybride formé entre la séquence spécifique de la cible de chaque sonde et la séquence cible, et la seconde température de fusion, est notamment supérieure ou égale à 8˚C.

LES MOLECULES «RAPPORTEUR»

La présente invention comprend également l'utilisation d'une ou plusieurs molécules « rapporteur » non immobilisées, marquées ou non, telles que des séquences d'acide nucléique comprenant une longueur égale à celle du bras d'une sonde. L'invention décrit ainsi une biopuce de sondes en épingle à cheveux, caractérisée en ce qu'une molécule «rapporteur» peut s'hybrider avec chaque sonde en épingle à cheveux. Une sonde en épingle à cheveux décrite ici peut être utilisée en conjonction avec des molécules «rapporteur», qui ne peuvent s'hybrider qu'avec la forme ouverte de la sonde, permettant ainsi une différentiation entre les conformations ouvertes et fermées, et générant un signal uniquement lorsqu'une molécule cible s'associe avec la séquence spécifique de la cible d'une sonde en épingle à cheveux.

Des molécules «rapporteur» peuvent être conçues pour avoir une structure tige boucle avec une tige de 4-10 nucléotides, préférentiellement de 6 nucléotides ou moins, et une boucle comprenant entre 12-20 nucléotides, dans laquelle la séquence spécifique de la cible peut être parfaitement complémentaire avec un bras de la sonde en épingle à cheveux.

Des molécules «rapporteur» peuvent être conçues pour avoir une structure linéaire comprenant une séquence parfaitement complémentaire d'un bras de la sonde en épingle à cheveux.

L'invention décrit ainsi une sonde en épingle à cheveux non marquée, caractérisée en ce qu'une molécule cible est hybridée avec la séquence spécifique de la cible, et en ce qu'une molécule «rapporteur» est hybridée avec le premier ou le second bras. La molécule «rapporteur» comprend de préférence moins de 10 nucléotides parfaitement complémentaires avec la séquence d'acide nucléique du premier bras ou du second bras.

Les molécules «rapporteur» peuvent être conçues avec une molécule se fixant sur le petit sillon de l'ADN (« minor groove binder ») attaché à une extrémité de la molécule «rapporteur». Le marquage des molécules «rapporteur» s'effectue de la même façon que celui des séquences cibles (voir précédemment), avec un marquage fluorescent à l'extrémité 3' et/ou 5'.

Une fois que l'échantillon a été mis en contact avec une sonde en épingle à cheveux, ou un jeu de sondes en épingle à cheveux, le bras d'une sonde peut devenir accessible à une molécule «rapporteur» complémentaire, du fait de l'association de la boucle avec sa séquence cible, et un signal peut donc être émis. La molécule «rapporteur» peut être notamment une séquence d'acide nucléique, une séquence de peptide-acide nucléique, un acide nucléique bloqué (« locked nucleic acid, LNA »), une séquence oligopeptidique, une protéine, ou un enzyme couplé à une séquence d'acide nucléique. La molécule « rapporteur » est préférentiellement caractérisée par sa capacité à se lier à un bras de la sonde décrite ici uniquement en conformation « ouverte ». La molécule «rapporteur» contient de préférence au moins une séquence parfaitement complémentaire de l'une des séquences des bras d'une sonde en épingle à cheveux. Lors de l'addition d'une molécule «rapporteur», l'association entre la molécule «rapporteur» et une sonde en épingle à cheveux se produit seulement avec un bras de la sonde qui est dans sa conformation ouverte. Cette structure ouverte est une conséquence de l'association de la boucle de la sonde en épingle à cheveux avec une molécule cible. Pour des sondes en épingle à cheveux immobilisées sur le même support, les sondes peuvent être conçues pour avoir la même séquence de bras, de façon à ce qu'une seule molécule «rapporteur» soit nécessaire pour analyser l'association de toutes les sondes en épingle à cheveux avec leurs molécules cibles spécifiques.

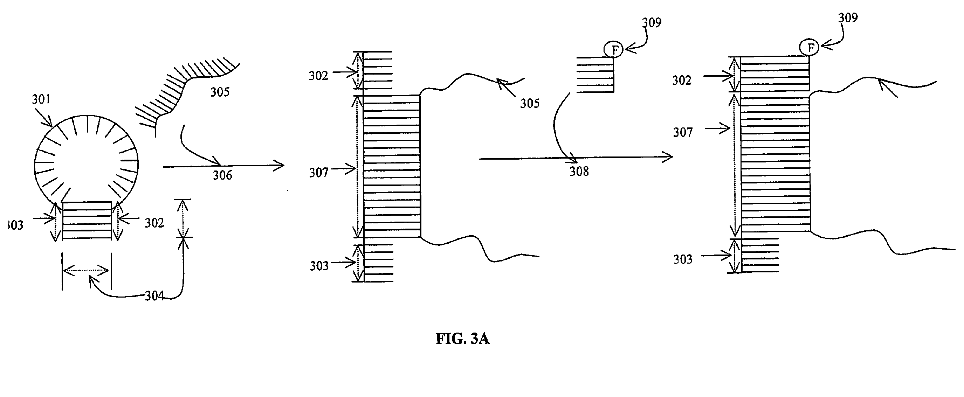

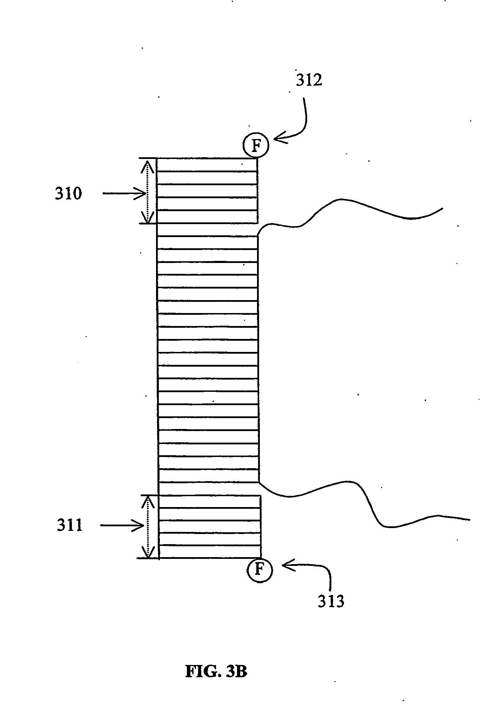

Les FIG.3 montrent comment se fait l'association d'une molécule «rapporteur» avec une sonde décrite ici pour générer un signal lors de l'hybridation de la sonde en épingle à cheveux avec sa molécule cible. La FIG. 3A montre les étapes de l'association d'une molécule «rapporteur» avec une sonde en épingle à cheveux. Tout d'abord, la sonde 301 est dans sa conformation fermée pour laquelle le bras 302 situé à l'extrémité 5' de la séquence spécifique de la cible et le bras 303, situé à l'extrémité 3' de la séquence spécifique de la cible, sont hybridés, formant la tige 304. En présence 306 de la molécule cible 305 (i.e., un acide nucléique) pour laquelle la sonde en épingle à cheveux a été conçue, la séquence spécifique de la cible 307 s'hybride avec la molécule cible 305. Cela conduit la sonde en épingle à cheveux à adopter une conformation ouverte dans laquelle les bras 302 et 303 de la sonde se dissocient l'un de l'autre et deviennent accessibles à la molécule «rapporteur». Lors de l'ajout 308 de la molécule «rapporteur» 309, qui comprend un acide nucléique, à la sonde décrite ici en conformation ouverte, la molécule «rapporteur» 309 peut s'hybrider avec le bras 302 situé à l'extrémité 5' de la séquence spécifique de la cible, car une portion de la molécule «rapporteur» 309 a été conçue pour être le complément du bras 302 situé à l'extrémité 5' de la séquence spécifique de la cible.

Comme chaque sonde en épingle à cheveux possède deux bras, deux molécules «rapporteur» peuvent être conçues et utilisées pour générer un signal lorsqu'une sonde en épingle à cheveux s'hybride avec une séquence cible. La FIG. 3B décrit une sonde telle que décrite ici de conformation ouverte pour laquelle les deux bras 310 et 311 de la sonde en épingle à cheveux sont hybridés avec deux molécules «rapporteur» 312 et 313. Quand deux molécules «rapporteur» sont utilisées et qu'une molécule «rapporteur» est conçue pour contenir une séquence d'acide nucléique complémentaire d'un bras de la sonde en épingle à cheveux, et que l'autre molécule «rapporteur» est conçue pour contenir une séquence d'acide nucléique complémentaire de l'autre bras de la sonde en épingle à cheveux, il peut être nécessaire d'ajouter les molécules «rapporteur» l'une après l'autre, en intercalant une étape de lavage entre les étapes d'ajout de molécules «rapporteur». Ceci est dû au fait que chaque molécule «rapporteur» contient une séquence d'acide nucléique parfaitement complémentaire de celle de l'autre molécule «rapporteur». Si les deux molécules «rapporteur» étaient ajoutées simultanément, ces molécules «rapporteur» pourraient s'hybrider l'une avec l'autre ainsi que sur la sonde en épingle à cheveux.

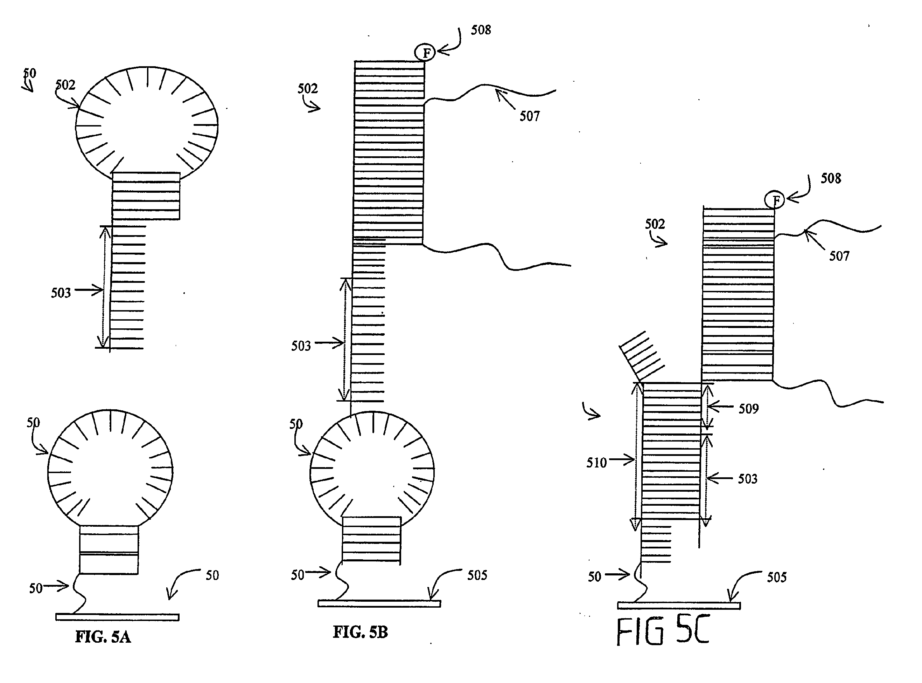

La molécule «rapporteur» comprend un marqueur détectable. De manière préférée, le marqueur détectable est choisi parmi un analogue de nucléotide, un marqueur fluorescent, de la biotine, de l'imminobiotine, un antigène, un co-facteur, du dinitrophenol, de l'acide lipoïque, un composé oléfinique, un polypeptide, une molécule riche en électrons, un enzyme, ou un isotope radioactif. Les molécules «rapporteur» peuvent être des séquences marquées par exemple avec un fluorochrome (référencé par F dans les FIGS. 3A, 3B, 5B, et 5C) ou des séquences non marquées présentant des modifications supplémentaires permettant une augmentation de l'affinité pour leur association avec les bras qui leurs sont complémentaires. Par exemple, cette modification peut être une molécule se fixant sur le petit sillon de l'ADN (DNA minor groove binder) attachée à l'extrémité 3' ou 5' du «rapporteur», ou une modification chimique du «rapporteur» permettant l'établissement d'une liaison covalente entre les brins d'ADN hybridés après application d'un traitement spécifique. Ces modifications peuvent également inclure l'incorporation d'un groupement phosphorethioate dans la chaîne phosphate de la molécule «rapporteur» (Cogoi, S et al. 2001. Biochemistry 40 :1135-43 ; Xodo, L. et al. 1994. Nucleic Acids Res. 22:3322-30).

Les molécules «rapporteur» peuvent également être détectées par leur masse, en utilisant la spectrométrie de masse et l'analyse du temps de vol. Par exemple, des sondes décrites ici, immobilisées sur un support et hybridées avec leurs séquences cibles et des molécules «rapporteur» peuvent être analysées directement par spectrométrie de masse en orientant la source du laser du spectromètre de masse sur une région définie de la surface correspondant à une sonde spécifique, et en collectant la masse et la quantité ou l'abondance relative de chaque molécule «rapporteur». La molécule «rapporteur» peut varier en longueur et en masse, ou peut être liée à plusieurs espèces moléculaires de masse connue, détectable par spectrométrie de masse (Laken, S.J. et al. 1998. Nat. Biotechnol. 16 :1352-6 ; Little, D. P. et al. 1997. Nat. Med. 3:1413-6 ; Little, D. P. 1997. Eur. J. Clin. Chem. Clin. Biochem. 35 :545-8 ; Braun, A. et al. 1997. Clin. Chem. 43:1151-8).

La molécule «rapporteur» peut être couplée à des molécules électrochimiquement actives telles que des dérivés du ferrocène ou du cobaltocène qui génèrent un courant électrique d'oxydo-réduction lorsqu'un courant leur est appliqué (Umek, R.M. et al. 2001. J.Mol.Diagn. 3 :74-84 ; Padeste, C. et al. 2000. Biosens.Bioelectron. 15:431-8; Tsai, W.C. et al. 1995. Analyst 120:2249-54). Les molécules «rapporteur» peuvent aussi être un enzyme capable de générer des espèces électroactives, soit par une activité électrocatalytique, soit par clivage d'un support électrochimiquement inactif en un produit électrochimiquement actif. (Valat, C et al. 2000. Analytica Chimica Acta 404 :187-94 ; Oliver, B. et al. 1997. Anal. Chem. 69 :4688-94 ; Limoges, B. 1996. Anal. Chem. 68 :4141-48; Bourdillon, C. et al. 1996. J. Am. Chem. Soc. 115:12264-69). Pour de telles molécules «rapporteur», des sondes décrites ici peuvent être immobilisées sur des surfaces conductrices comme du dioxyde de silicium, du graphite, du carbone vitreux, des surfaces d'oxyde de Sélenium-Indium, des surfaces métalliques ou des surfaces conductrices à base de polymères plastiques, ou des surfaces non-conductrices présentant des zones individualisées de matériaux conducteurs pré-déposés ou pré-polymérisés. Cette méthode de détection peut utiliser l'électropolymérisation du détecteur ou d'une partie de celui-ci sur la surface comme moyen permettant de détecter un signal sous la forme d'un courant ou d'une variation de potentiel.

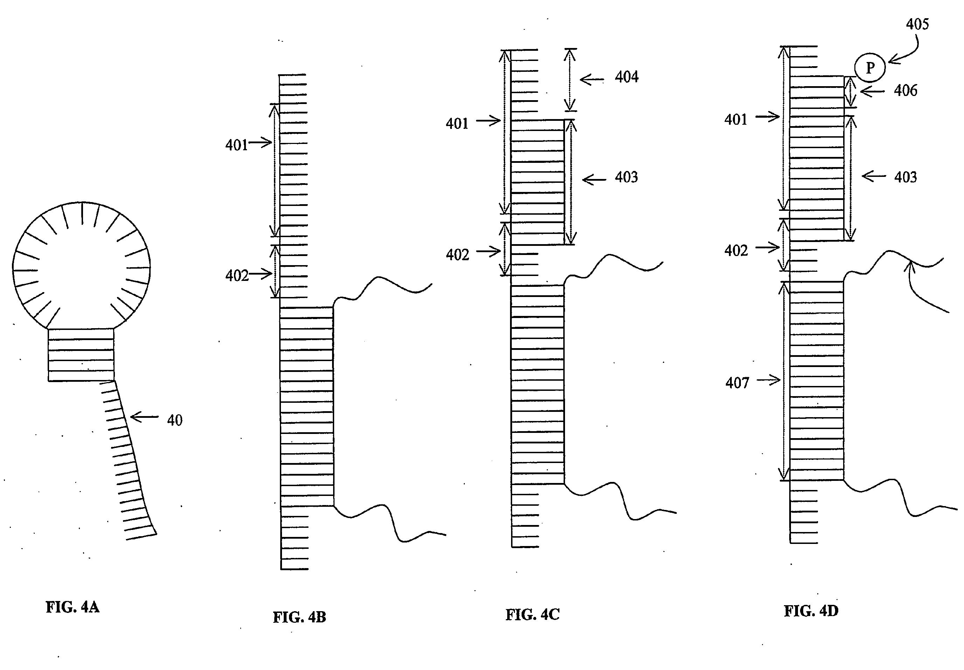

Un autre système «rapporteur» permettant la détection de l'association d'une sonde en épingle à cheveux avec une molécule cible utilise un système de marquage avec une polymérase. La FIG. 4 décrit le principe de fonctionnement de ce système. La FIG 4A montre une sonde en épingle à cheveux dans sa conformation fermée, prolongée dans sa partie 5' par une séquence de 10 à 20 nucléotides, « l'amorce » 401. Les amorces de toutes les sondes en épingle à cheveux qui doivent être utilisées dans une condition de test spécifique sont de préférence conçues pour avoir la même séquence nucléotidique. La description décrit également l'utilisation d'amorces différentes dans une condition de test spécifique pour ajouter un niveau supplémentaire de différentiation entre les signaux générés par hybridation d'une sonde en épingle à cheveux avec sa molécule cible spécifique. Pour chaque amorce utilisée, la séquence nucléique de l'amorce 401 est contrôlée pour vérifier l'absence d'homologie de séquence avec toutes les autres sondes en épingle à cheveux, ainsi que les autres réactifs de la polymérase. De façon préférée, l'amorce a une longueur de 18-20 nucléotides et une température de fusion de 59˚C +/-2˚C. L'amorce 401 est utilisée pour initier l'incorporation d'au moins un nucléotide marqué par une polymérase. Lors de l'association de la séquence spécifique de la cible avec la molécule cible, le bras 5' de la séquence spécifique de la cible ainsi que l'amorce sont accessibles aux réactifs de la réaction de polymérisation. La réaction de polymérisation est effectuée sans dénaturation de l'ADN et à 37-60˚C, selon la polymérase utilisée. Des exemples de polymérases utilisables pour ce système de marquage sont la polymérase I de *E.coli,* fragment de Klenow, les polymérases thermostables Taq, La polymerase à ADN de T4, et toutes les polymérases natives ou modifiées génétiquement, à l'exception des polymérases « hot-start » (Sambrook J. et al., 1989, Molecular Cloning : A Laboratory Manual, 2nd Ed., Cold Spring Harbor Laboratory, Cold Spring Harbor, New York, pages 5.44-5.47 ; Ausubel F.M. et al., 1997, Current Protocols in Molecular Biology, Vol.I, Green Publishing Associates, Inc., and John Wiley&Sons, Inc., New York, pages 3.5.11 et 3.5.12).

La FIG.4B montre une telle sonde en épingle à cheveux dans sa conformation ouverte dans laquelle le bras 402, et l'amorce 401 situés à l'extrémité 5' de la séquence spécifique de la cible sont accessibles pour participer à la réaction avec la polymérase. Une molécule «rapporteur» 403 complémentaire d'une portion du bras 402 et de l'amorce 401 peut alors s'hybrider avec ces séquences. La FIG. 4C montre une telle hybridation entre un bras 402, une amorce 401, et une molécule «rapporteur» 403. La molécule «rapporteur» 403 peut être complémentaire d'une portion ou de la totalité du bras 402. Cependant, la molécule «rapporteur» 403 ne peut être complémentaire de la totalité de l'amorce 401. La portion de la molécule «rapporteur» 403 qui complète la portion du bras 402 et du primer 401 ne doit pas s'associer avec ou avoir des nucléotides qui sont complémentaires d'une portion de l'extrémité 3' du primer 401. De préférence, la taille de la séquence nucléotidique de la molécule «rapporteur» 403 est de 1 à 5 nucléotides plus courte que celle de l'amorce 401 à laquelle elle s'hybride. Cette particularité est représentée par la séquence non hybridée 404 de l'amorce 401, et permet à une polymérase de prolonger la molécule «rapporteur» 403 en ajoutant des nucléotides complémentaires de l'amorce 401. Quand une polymérase 405 prolonge la molécule «rapporteur» 403 (voir FIG. 4D), des nucléotides marqués 406 peuvent être incorporés pour générer un signal permettant de détecter l'hybridation de la séquence spécifique de la cible 407 avec la molécule cible 408.

SYSTEME D'ADRESSAGE UNIVERSEL

La présente description décrit aussi un système dans lequel des sondes en épingle à cheveux non-immobilisées peuvent être utilisées en conjonction avec des sondes en épingle à cheveux immobilisées. Désigné par le terme « système d'adressage universel, ou SAU », ce système de criblage *ex vivo* a pour avantage de mettre en oeuvre une batterie de sondes en épingle à cheveux ou linéaires immobilisés, pré-fabriquées et utilisables avec des sondes en épingle à cheveux non immobilisées fournies par l'utilisateur final, et lui permettant d'éviter la fabrication de la biopuce. Le SAU peut par exemple fournir une biopuce universelle dans laquelle les séquences spécifiques des cibles des sondes en épingle à cheveux immobilisées comprennent un jeu de sondes en épingle à cheveux pré-déterminées conçues pour s'associer avec des sondes en épingle à cheveux ou linéaires non immobilisées possédant une étiquette spécifique. Ceci permet la construction d'une biopuce unique pour toutes les conditions d'analyse. Les acides nucléiques cibles à détecter sont tout d'abord capturés par les séquences spécifiques des cibles des sondes en épingle à cheveux ou linéaires non immobilisées, qui sont, à leur tour, conçues pour s'associer ,via leur séquence étiquette spécifique , aux sondes en épingle à cheveux immobilisées.

Dans une configuration préférée, le SAU comprend deux jeux de sondes en épingle à cheveux. Une sonde en épingle à cheveux, désignée par le terme « première sonde en épingle» n'est pas immobilisée sur un support et possède une séquence étiquette à l'extrémité de l'un de ses bras 3' ou 5'. L'étiquette peut s'hybrider avec une séquence d'acide nucléique simple brin de 6 à 30 nucléotides, ou avec une sonde en épingle à cheveux désignée par le terme « deuxième sonde en épingle» qui est immobilisée sur un support et peut être partiellement ou totalement complémentaire de l'étiquette. Lorsque l'espèce immobilisée est une deuxième sonde en épingle, la séquence spécifique de la cible de la deuxième sonde peut être complémentaire de la séquence étiquette, d'une portion de la séquence étiquette, et/ou d'une portion du bras adjacent à l'étiquette. L'utilisation de deuxièmes sondes qui ont des séquences spécifiques de la cible dirigées contre une portion de l'étiquette et une portion du bras adjacent à l'étiquette permet que seules les premières sondes ayant leur séquence spécifique de la cible associée avec une molécule cible sont capables de s'hybrider avec la deuxième sonde en épingle. Si plusieurs séquences étiquettes sont utilisées avec le SAU, et nécessitent d'être différentiées, une différence d'au moins 2 nucléotides, située au centre de la boucle de la deuxième sonde peut être requise, de façon à ce qu'il y ait suffisamment de différences avec les autres séquences d'étiquettes pour hybrider spécifiquement la séquence spécifique de la cible complémentaire sur les sondes en épingle à cheveux immobilisées.

La description décrit ainsi un système d'adressage universel non marqué comprenant :

(a) au moins deux premières sondes non marquées linéaires comprenant :

(i) une séquence spécifique d'une cible, de 6 à 30 nucléotides de long ;
(ii) une séquence dite étiquette de 10 à 50 nucléotides de long connectée à l'extrémité 5' de la séquence spécifique de la cible,

ladite séquence spécifique d'une cible n'étant complémentaire d'aucune autre partie de ladite sonde non marquée ; et
(b) au moins deux secondes sondes susceptibles de former une épingle à cheveux comprenant :

(i) une séquence spécifique d'une seconde cible, de 6 à 30 nucléotides de longueur ;
(ii) un second premier bras de ladite seconde sonde de moins de 10 nucléotides de long et positionné en 5' de ladite seconde séquence spécifique de ladite cible;
(iii) un second deuxième bras de ladite seconde sonde de moins de 10 nucléotides de long et positionné en 3' de ladite seconde séquence spécifique de ladite cible ; et
(iv) un espaceur connectant le premier ou le second bras audit support ,

ladite séquence spécifique de ladite cible de la deuxième sonde n'étant complémentaire d'aucune autre partie de ladite seconde sonde; ledit premier bras et ledit second bras de ladite seconde sonde étant parfaitement complémentaires l'un de l'autre ; et en outre, ladite séquence étiquette d'une des premières sondes étant complémentaire de ladite seconde séquence cible d'une des deuxièmes sondes.

De préférence, la description décrit ainsi un système d'adressage universel non marqué comprenant :

(a) au moins deux premières sondes non marquées susceptibles de former une épingle à cheveux comprenant :

(i) une séquence spécifique d'une cible, de 6 à 30 nucléotides de long ;
(ii) un premier bras de moins de 10 nucléotides de long, positionné en 5' de ladite séquence spécifique d'une cible ;
(iii) un second bras de moins de 10 nucléotides de long, positionné en 3' de ladite séquence spécifique d'une cible ; et
(iv) une séquence dite étiquette de 10 à 50 nucléotides de long connectée au premier bras ou au second bras,

ladite séquence spécifique d'une cible n'étant complémentaire d'aucune autre partie de ladite sonde non marquée ; et en outre, le premier bras et le second bras étant parfaitement complémentaires, l'un avec l'autre, et
(b) au moins deux secondes sondes susceptibles de former une épingle à cheveux comprenant :

(i) une séquence spécifique d'une seconde cible, de 6 à 30 nucléotides de longueur ;
(ii) un second premier bras de ladite seconde sonde de moins de 10 nucléotides de long et positionné en 5' de ladite seconde séquence spécifique de ladite cible;
(iii) un second deuxième bras de ladite seconde sonde de moins de 10 nucléotides de long et positionné en 3' de ladite seconde séquence spécifique de ladite cible ; et
(iv) un espaceur connectant le premier ou le second bras audit support ,

ladite séquence spécifique de ladite cible de la deuxième sonde n'étant complémentaire d'aucune autre partie de ladite seconde sonde; ledit premier bras et ledit second bras de ladite seconde sonde étant parfaitement complémentaires l'un de l'autre ; et en outre, ladite séquence étiquette d'une des premières sondes complétée de la séquence du bras connecté à cette étiquette étant complémentaire de ladite seconde séquence cible d'une des deuxièmes sondes.

Dans un mode de réalisation préféré, la séquence spécifique de la cible des premières sondes en épingle à cheveux a une longueur de 10 à 25 nucléotides, de manière encore plus préférée de 15 à 20 nucléotides.

La séquence spécifique de la cible des secondes sondes en épingle à cheveux a de manière préférée une longueur de 10 à 25 nucléotides, de manière encore plus préférée de 15 à 20 nucléotides.

La description décrit également un système d'adressage universel non marqué, caractérisé en ce que tous les premiers bras possèdent une séquence identique.

Elle décrit par ailleurs un système d'adressage universel non marqué, caractérisé en ce que tous les premiers bras de la deuxième sonde en épingle à cheveux possèdent une séquence identique.

La description décrit également un système d'adressage universel non marqué, caractérisé en ce qu'une même molécule «rapporteur» peut s'hybrider avec l'une ou l'autre des première et seconde sondes décrites ici.

Le premier bras de la seconde sonde décrite ici et le second bras de la seconde sonde décrite ici peuvent se dissocier l'un de l'autre quand une portion de l'étiquette et une portion du premier ou du deuxième bras connecté à l'étiquette s'associent avec la séquence spécifique de la cible de la seconde sonde.

En particulier, la description décrit un système d'adressage universel non marqué, caractérisé en ce que l'étiquette s'associe avec la séquence spécifique de la cible de la seconde sonde.

La FIG. 5 décrit le fonctionnement d'une des configurations du SAU. Dans cette configuration, la séquence spécifique de la cible de la deuxième sonde en épingle à cheveux est conçue pour s'hybrider avec l'étiquette et une portion du bras adjacent de l'étiquette de la première sonde en épingle à cheveux. FIG. 5A montre le SAU 501, en l'absence de molécule cible. La première sonde en épingle à cheveux 502, qui comprend une étiquette 503, et la deuxième sonde en épingle à cheveux 504, attachée au support 505 par un espaceur 506, sont dans leur conformation fermée. La FIG. 5B montre comment la première sonde 502, adopte une conformation ouverte lors de l'association avec sa cible 507, et une molécule «rapporteur» 508 marquée. La FIG. 5C montre la première sonde en épingle à cheveux 502, avec son étiquette 503, et une portion du bras 509 adjacent à l'étiquette hybridé avec la séquence spécifique de la cible 510 de la deuxième sonde en épingle à cheveux 504. La position de la molécule cible peut être déterminée en détectant la position de la molécule «rapporteur» sur le support.

DETECTION D'ACIDES NUCLEIQUES

La description décrit notamment une méthode de détection d'acide nucléique comprenant :

(a) la mise en contact *ex vivo* d'un échantillon d'acide nucléique avec une biopuce comprenant au moins deux sondes décrites ici ; et

(b) la détection d'un signal provenant d'au moins une desdites sondes de la biopuce ayant adopté une conformation ouverte à la suite du contact établi lors de l'étape (a).

La détection de l'étape (b) est obtenue en utilisant une molécule «rapporteur» marquée avec un marqueur détectable. Le marqueur détectable est de préférence choisi parmi un analogue de nucléotide, un marqueur fluorescent, de la biotine, de l'imminobiotine, un antigène, un co-facteur, du dinitrophenol, de l'acide lipoïque, un composé oléfinique, un polypeptide, une molécule riche en électrons, un enzyme ou un isotope radioactif.

Selon un mode de réalisation préféré, l'échantillon d'acide nucléique mis en oeuvre à l'étape (a) est marqué au préalable avec un marqueur détectable.

De manière préférée, tous les hybrides parfaits formés entre les séquences spécifiques de la cible des sondes, et les molécules cibles, ont une température de fusion égale à plus ou moins 4˚C près, de préférence à plus ou moins 1 ˚C près. La différence entre une température de fusion de l'hybride formé entre la séquence spécifique de la cible et la molécule cible, et une deuxième température de fusion pour l'association entre la séquence spécifique de la cible et une molécule pour laquelle la séquence spécifique n'a pas été conçue pour chaque sonde, est de préférence supérieure ou égale à 5˚C, de manière encore plus préférée supérieure ou égale à 8˚C.

ANALYSE DE MUTATION

La description propose également une méthode pour concevoir et utiliser des biopuces de sondes décrites ici pour analyser des séquences multiples d'ADN pouvant présenter des variations alléliques. Plus particulièrement, la présente description propose un outil pour la détermination des haplotypes sur de court segments d'ADN présentant de multiples variations ponctuelles d'un seul nucléotide sur une séquence d'ADN. Les mutations analysables par cette méthode incluent les transitions (ou substitutions), délétions ou insertions d'un nucléotide, ou les variations multiples séparées par une courte séquence ainsi que les larges délétions et insertions. L'analyse de telles mutations peut nécessiter plus d'une sonde spécifique des allèles. Jusqu'à quatre sondes spécifiques des allèles peuvent être conçues et immobilisées pour l'analyse d'une seule transition, en fonction du degré d'allélisme observé pour chaque variation génétique, et le degré de confiance souhaité pour l'analyse. Le TABLEAU I donne par exemple les séquences en épingle à cheveux qui peuvent être utilisées pour analyser une transition présente dans le codon 142 du gène TCF1, qui code pour le facteur nucléaire hépatique 1 (Linder, T. et al. 1999. Hum. Molec.Genet. 8:2001-8), 142CC Cy5 qui est la molécule cible marquée avec du Cy5 de la sonde en épingle à cheveux StrNCb, et 142CT Cy5, qui est la molécule cible marquée avec du Cy5 de la sonde en épingle à cheveux StrNTb. Les lettres en gras représentent la séquence des boucles, alors que les lettres soulignées indiquent la position du site de mutation. La mutation existant naturellement est une transition C/T, ou G/A sur le brin complémentaire. Les sondes en épingle à cheveux nécessaires pour l'analyse de la présence de cette mutation sont les sondes portant un C, StrNCb, et un T, StrNCT. Les deux autres sondes en épingle à cheveux, StrNGb et StrNAb, peuvent être utilisées mais ne sont pas indispensables.

**TABLEAU I**

| Nom | Séquence | SEQ ID No |
|---|---|---|
| StrNCb | 5'- GCG AGC **CAA CCA GTC CCA CCT GTC** GCT CGC -3' | 1 |
| StrNTb | 5'- GCG AGC **CAA CCA GTT CCA CCT GTC GCT** CGC -3' | 2 |
| StrNGb | 5'- GCG AGC **CAA CCA GTG CCA CCT GTC GCT** CGC -3' | 3 |
| StrNAb | 5'- GCG AGC **CAA CCA GTA CCA CCT GTC GCT** CGC -3' | 4 |
| 142CC CY5 | Cy5 –5' -AGG TGT TGG GAC AGG TGG GAC TGG TTG AGG CCA GTG GTA TCG –3' | 5 |
| 142CT CY5 | Cy5 –5'- AGG TGT TGG GAC AGG TGG AAC TGG TTG AGG CCA GTG GTA TCG- 3' | 6 |

Tous les polymorphismes associés avec la région étudiée sont référencés en utilisant une carte suffisamment précise de la séquence génomique. Pour les variations qui doivent être analysées pour la variation allélique, un jeu d'amorces de PCR est conçu en utilisant les directives générales connues par l'homme de l'art. Pour éviter l'utilisation d'amorces de PCR contenant des variants alléliques et maximiser le rendement et la spécificité des réactions de PCR, une attention particulière est portée au fait de ne pas concevoir de paires d'amorces dans une région génomique contenant des polymorphismes connus. Pour chaque mutation nécessitant une analyse de la variation allélique, au moins deux sondes en épingle à cheveux sont conçues et utilisées. La position de la variation allélique est localisée de la façon la plus proche possible du centre de la séquence spécifique de la boucle (voir 101 dans FIG.1) mais peut varier en fonction des caractéristiques de la séquence. Quand la localisation du variant est positionnée à distance du centre de la boucle, la Tm de l'hybride formé entre la séquence spécifique de la cible et une molécule cible qui ne lui est pas parfaitement complémentaire décroît.

Lors de la conception de sondes en épingle à cheveux devant être utilisées dans la même analyse la modification de la longueur de la séquence spécifique de la cible (voir plus haut) est utilisée, de façon à ce que toutes les Tm des hybrides formés entre la séquence spécifique de la cible et la molécule cible soient égales à 1-4˚C d'écart. Par exemple, la variation allélique à détecter peut être située à 1 à 10 bases de distance du centre de la boucle, de préférence à 1 à 5 bases de distance du centre de la boucle. Une fois les séquences spécifiques de la cible conçues pour toutes les sondes en épingle à cheveux, de façon à avoir une Tm située dans une gamme acceptable (voir plus haut), la Tm de chaque séquence spécifique de la cible lors de l'hybridation avec d'autres allèles (hétéroduplexes) est calculée en utilisant Meltcalc (voir précédemment). La variation de Tm entre les duplexes d'ADN parfaits et imparfaits est maintenue la plus grande possible, et est considérée comme suffisante pour une discrimination correcte quand la Tm de l'hybride formé entre une séquence spécifique de la cible et une molécule cible ne s'associant pas parfaitement, i.e., un hybride possédant au moins un mésappariement d'une base, est au moins de 5˚C inférieure à la Tm de l'hybride formé entre la séquence spécifique de la cible et la molécule cible. De façon encore plus préférée, la Tm de l'hybride formé entre une séquence spécifique de la cible et une molécule cible ne s'associant pas parfaitement est au moins de 8˚C en dessous de la Tm de l'hybride formé entre la séquence spécifique de la cible et la molécule cible.

Les séquences cibles peuvent être de l'ADN génomique, ou des séquences d'ADN marquées ou non marquées amplifiées par PCR. La taille de la séquence analysée en terme de nombre de nucléotides peut être la même que celle de la partie boucle des sondes d'hybridation, mais un nombre plus grand de nucléotide est préféré, sans limitation en terme de taille maximale de la séquence analysée. Chaque brin de ces séquences cibles double brins peut être séparé thermiquement et hybridé en tant qu'espèce individuelle ou sous la forme d'un mélange sur la biopuce à sonde en épingle à cheveux, de façon à ce que seule la séquence parfaitement complémentaire soit hybridée sur sa sonde en épingle à cheveux spécifique immobilisée.

L'abondance relative de chaque allèle détecté dans un échantillon peut être déterminée en mesurant le signal relatif pour chaque sonde spécifique de chaque allèle. Pour une simple transition correspondant à un bi-allélisme unique, deux sondes en épingle à cheveux parmi les quatre possibles (chacune ayant une base de différence correspondant à un allèle) doivent donner un signal pour un échantillon hétérozygote. Cet allèle peut être déterminé par la position des sondes en épingle à cheveux sur la surface solide. Pour un échantillon homozygote, seule l'une des sondes en épingle à cheveux sur les quatre doit donner un signal.

La description décrit ainsi une méthode de détection *ex vivo* d'un variant génétique dans un échantillon d'acide nucléique comprenant :

(a) la mise en contact de l'échantillon avec une biopuce de sondes décrites ici comprenant au moins deux sondes, au moins une sonde de la biopuce étant une sonde décrite ici spécifique d'un variant génétique et possédant une région boucle parfaitement complémentaire du variant génétique, et
(b) la détection d'un signal provenant de ladite sonde en épingle à cheveux du variant génétique,

le signal détecté à l'étape (b) indiquant la présence du variant génétique dans l'échantillon d'acide nucléique.

De préférence, le variant génétique détecté est un polymorphisme d'un seul nucléotide, une délétion ou insertion de une base ou plus, ou une duplication de une base ou plus.

Selon un mode de réalisation préféré, une molécule «rapporteur» peut s'hybrider avec chaque sonde en épingle à cheveux de la biopuce. La détection de l'étape (b) est obtenue en utilisant une molécule «rapporteur» marquée avec un marqueur détectable. L'échantillon de l'étape (a) peut être marqué au préalable avec un marqueur détectable.

SEQUENCAGE

La description décrit également une méthode de séquençage *ex vivo* d'échantillons d'oligonucléotides qui permet d'éviter les étapes de préparations manuelles impliquées par les méthodes de séquençage utilisant une dégradation chimique et une terminaison de chaîne avec des di-déoxy nucléotides. La description décrit la conception et l'utilisation de sondes en épingle à cheveux, et de biopuces à sondes en épingle à cheveux dans lesquelles la séquence spécifique de la cible de chaque sonde contient une séquence identique à une portion au moins d'une autre séquence spécifique de la cible d'une autre sonde immobilisée sur la même biopuce. En fonction de la taille de la séquence à séquencer, les séquences spécifiques de la cible des sondes en épingle à cheveux immobilisées sont en outre conçues pour s'hybrider à toute séquence oligonucléotidique possible dont la taille est au moins égale à la longueur des séquences spécifiques des cibles des sondes en épingle à cheveux. Grâce à cette méthode, une biopuce peut être conçue pour séquencer tout oligonucléotide de taille prédéterminée. La détection des hybridations se produisant entre les portions de l'échantillon et les sondes en épingle à cheveux peut être contrôlée, et la séquence peut être déterminée conformément avec les descriptions faites ici et celles des brevets US n° : 6,270,961 ; 6,025,136 ; 6,018,041 ; 5,871,928 ; et 5,695,940.

La description décrit ainsi une méthode de séquençage *ex vivo* d'un oligonucléotide comprenant :

(a) la mise en contact d'un échantillon contenant l'oligonucléotide avec une biopuce comprenant au moins deux sondes décrites ici ; et
(b) la détection d'un signal provenant d'au moins une desdites sondes de la biopuce ;

le signal détecté dans l'étape (b) étant utilisé pour déterminer la séquence de l'oligonucléotide.

Selon un mode de réalisation préféré de l'invention, une molécule «rapporteur» peut s'hybrider avec chaque sonde en épingle à cheveux de la biopuce. La détection dans l'étape (b) est obtenue en utilisant une molécule «rapporteur» marquée avec un marqueur détectable. L'échantillon de l'étape (a) peut être au préalable marqué avec un marqueur détectable.

ANALYSE DE L'EXPRESSION DES GENES

La description décrit également une méthode pour concevoir et utiliser des sondes et des biopuces de sondes telles que décrites ici pour l'analyse de l'expression des gènes.

Cette application peut être utile pour l'analyse de l'expression différentielle d'acides nucléiques non marqués, pour lesquels un grand nombre de séquences différentes nécessite d'être analysé en terme de quantités relatives des unes par rapport aux autres. Pour chaque gène à analyser, au moins une sonde en épingle à cheveux peut être conçue pour chaque partie correspondant aux exons du gène.

La description décrit également une biopuce de sondes telles que décrites ici comprenant au moins deux sondes immobilisées sur un support. De manière préférée, une structure de tige est identique pour chacune au moins des deux sondes en épingle à cheveux. De manière encore plus préférée, une molécule «rapporteur» peut s'hybrider avec chaque sonde décrite ici de la biopuce.

De façon à suivre les niveaux d'expression d'extraits cellulaires ou d'échantillons tissulaires, les ARNs totaux, les ARNm et les ADNc et ARNc marqués ou non peuvent être utilisés comme séquence cible pour les sondes en épingle à cheveux, ou les biopuces de sondes en épingle à cheveux. Si des ADNc ou des ARNc sont utilisés pour l'analyse d'expression, ces séquences cibles peuvent être marquées par incorporation de marqueurs fluorescents durant la transcription inverse, ou des étapes additionnelles de PCR.

Pour l'analyse d'expression différentielle, plusieurs conditions peuvent être comparées. Ces conditions peuvent être des stress physiologiques, des degrés de différentiation différents, l'activation d'une voie métabolique, ou l'activité transcriptionnelle d'une drogue. Chacun des deux mélanges d'acides nucléiques tels que des ARNm ou des ARN totaux non marqués extraits de cellules ou de tissus, ou des ADNc peuvent par exemple être traités séparément et hybridés avec une biopuce à sondes en épingle à cheveux conçue spécifiquement pour chaque condition ou avec un jeu de premières sondes spécifiquement conçues pour une analyse avec le SAU.

DETECTION DES VARIANTS D'EPISSAGE ALTERNATIF ET CONDITIONS DE MESURE DES QUANTITES RELATIVES

La description décrit également une méthode pour l'analyse des produits d'épissage alternatifs et la détermination de l'abondance relative de chaque produit d'épissage alternatif dans différents états physiologiques du même tissu, ou de l'abondance relative de chaque produit d'épissage alternatif pour un même gène dans plusieurs tissus différents. Cette application peut être utile pour l'analyse de variants multiples de plusieurs gènes dont les produits peuvent interagir dans un schéma défini.

La description décrit ainsi une méthode de détection ex vivo d'un produit d'épissage alternatif d'un gène dans un échantillon d'acides nucléiques comprenant :

(a) la mise en contact de l'échantillon avec une biopuce à sondes telle que décrite ici comprenant au moins deux sondes telles que décrites ici, au moins une desdites sondes de la biopuce étant une sonde spécifique d'un exon du gène ou de la jonction de deux exons ; et
(b) la détection d'un signal provenant de la sonde spécifique de l'exon ou de la jonction de deux exons, le signal détecté indiquant la présence du produit d'épissage alternatif du gène dans l'échantillon d'acides nucléiques.

De préférence l'échantillon d'acides nucléiques comprend de l'ARNm.

Les séquences spécifiques des cibles des sondes en épingle à cheveux sont conçues pour être complémentaires de séquences d'ARNm ou d'ADNc. Plus précisément, elles sont conçues de façon à être complémentaires des différents produits d'épissage déjà connus de chaque séquence cible. Des variants d'épissage contenant des délétions importantes, ou de simples délétions d'un nucléotide peuvent être analysées sur la même biopuce portant des sondes en épingle à cheveux spécifiques de ces variations.

Pour chaque séquence exonique supposée ou connue d'un gène étudié, il est possible de concevoir au moins une sonde en épingle à cheveux spécifique pour détecter la présence ou l'absence de l'exon, de la portion d'exon, ou de la jonction de deux exons dans le produit du gène. La conception des sondes en épingle à cheveux est effectuée de la même façon que pour l'analyse d'expression des gènes, et peut utiliser le SAU pour adresser ces séquences sur un support. De plus, chaque variation en longueur ou en composition de l'ARNm ou de l'ADNc analysé peut être suivi par plusieurs sondes en épingle à cheveux, dont les séquences spécifiques de la cible sont conçues pour s'hybrider avec le centre de la région observée. L'analyse de la perte ou du changement d'un nucléotide entre deux ARNm peut être analysée par exemple en concevant deux sondes en épingle à cheveux ayant un nucléotide de différence localisé dans le centre des séquences spécifiques de la cible.

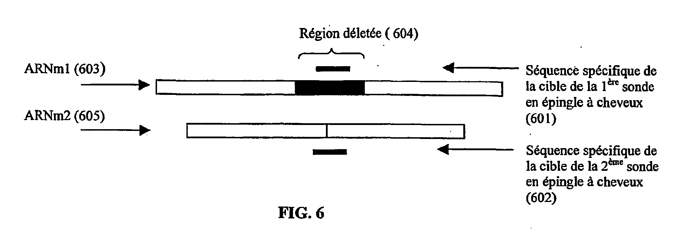

La conception de ces sondes en épingle à cheveux est similaire à celle décrite pour l'analyse de mutation. Pour la détection de délétions importantes dans une séquence d'ARNm, les séquences spécifiques des cibles de deux sondes en épingle à cheveux 601 et 602 peuvent être conçues pour s'hybrider à une séquence d'ARNm (FIG.6). La séquence spécifique de la cible de la première sonde en épingle à cheveux 601 peut être conçue pour s'hybrider à une région 604 d'un ARNm 603 qui peut être délétée et ainsi former un variant d'épissage. La séquence spécifique de la cible de la seconde sonde en épingle à cheveux 602 peut être conçue pour détecter la forme alternative d'épissage 605 en s'hybridant avec les régions 5' et 3' juste en deçà et en delà de la région délétée 604. La conception de ces sondes est similaire à celle des sondes conçues pour l'analyse d'expression. Une biopuce peut alors être utilisée pour rechercher la présence ou l'absence des exons transcrits du même gène.

La procédure pour l'analyse différentielle des variants d'épissage est la même que pour l'analyse d'expression différentielle. Les ARNm issus de deux états physiologiques d'un tissu ou d'un état physiologique de deux tissus, ou de deux tissus différents sont retro-transcrits séparément en ADNc et marqués (un marqueur différent pour chaque état ou tissu). Les ADNc marqués des deux sources sont mélangés et hybridés sur des biopuces de sondes en épingle à cheveux ou hybridés avec les sondes en épingle à cheveux du SAU, puis hybridées sur les biopuces de secondes sondes.

Selon un mode de réalisation préféré de l'invention, une molécule «rapporteur» peut s'hybrider avec chaque sonde en épingle à cheveux de la biopuce. La détection lors de l'étape (b) est obtenue en utilisant une molécule «rapporteur» marquée avec un marqueur détectable. L'échantillon de l'étape (a) peut être marqué au préalable avec un marqueur détectable.

SONDES EN EPINGLES A CHEVEUX POUR LA DETECTION DE MOLECULES D'ORIGINE ETRANGERE

La description décrit également l'utilisation pour la détection de molécules issues de tout organisme contenant des nucléotides, ou résidus issus de ces organismes, tels que des pathogènes, microorganismes, virus, parasites, ou de toute modification trouvée dans les organismes génétiquement modifiés (« OGM ») via la détection de l'ADN, de l'ARN ou de toutes autres molécules s'associant à une sonde en épingle à cheveux. Ici, l'expression « tout résidu d'organisme contenant des nucléotides », signifie toute molécule ou toute molécule résultant de cette molécule, issue d'un organisme contenant des nucléotides qui ait pénétré dans une autre cellule, par exemple un ADN viral dans une cellule infectée, ou un ARNm produit à partir de cet ADN viral. Cela peut s'avérer particulièrement utile dans les situations ou différentes molécules d'origines étrangères doivent être détectées dans le même échantillon, ou pour analyser des variants génétiques dans un échantillon biologique.

La description décrit également une méthode de détection *ex vivo* de tout organisme contenant des acides nucléiques ou de tout résidu de ces organismes comprenant :

(a) la mise en contact d'un échantillon d'acide nucléique avec une biopuce comprenant au moins deux sondes telles que décrites ici, au moins une desdites sondes étant une sonde spécifique des acides nucléiques d'un organisme ou de résidus de cet organisme ; et

(b) la détection d'un signal provenant de ladite sonde spécifique des acides nucléiques d'un organisme,

le signal détecté dans l'étape (b) indiquant la présence de l'organisme contenant des acides nucléiques ou de résidus de cet organisme.

L'organisme contenant des acides nucléiques est de préférence un virus ou une bactérie.

Selon un mode de réalisation préféré de l'invention, une molécule «rapporteur» peut s'hybrider avec chaque sonde en épingle à cheveux de la biopuce. La détection dans l'étape (b) est obtenue en utilisant une molécule «rapporteur» marquée avec un marqueur détectable. L'échantillon de l'étape (a) peut être marqué au préalable avec un marqueur détectable.

De manière préférée, l'ARN total, les ARNm ou les ADNc marqués ou non, ainsi que les ADNc marqués ou non amplifiés par PCR peuvent être utilisés sur des biopuces de sondes en épingle à cheveux pour détecter la présence ou l'absence du pathogène considéré, des microorganismes, des virus ou des OGM. Pour chaque molécule d'origine étrangère à analyser, au moins une sonde en épingle à cheveux est conçue pour au moins une partie exonique des gènes connus pour être exprimés à des niveaux constitutifs dans l'échantillon analysé.

ANALYSE DE DESEQUILIBRE ALLELIQUE ET DE PERTE D'HETEROZYGOTIE

La description décrit également une méthode pour concevoir et utiliser des sondes et des biopuces de sondes telles que décrites ici pour l'analyse de déséquilibre allélique dues à des délétions ou des insertions de fragments chromosomiques et l'analyse de perte d'hétérozygotie.

Cette application peut être utile pour l'analyse de délétions chromosomiques, pour lesquelles les points de cassure du chromosome sont connus ou inconnus. Les points de cassure du fragment d'ADN où se produit la délétion sont souvent inconnus, et parfois spécifique à un individu. (Mateo M. et Al. (1999), Am. J. Path., 154 (5), 1583-1589). Ce type d'altération génétique se retrouve dans des pathologies cancéreuses, comme le cancer de la prostate, du sein, certaines formes de cancer du colon, et certains lymphomes (Larsson C.M. et Al. (2001), Molecular Diagnosis., 6 (3), 181-188).

La description décrit la comparaison des séquences génomiques de cellules saines (cellules lymphocytaires du sang, par exemple), avec celles de cellules présumées tumorales (par exemple cellules issues de biopsies ou de liquides comme l'urine, ou liquide céphalo-rachidien, sécrétions) en utilisant comme moyen d'analyse une biopuce de sondes telle que décrite ici, et une méthode d'analyse différentielle.

Cette méthode d'analyse consiste à amplifier par PCR en utilisant les méthodes connues de l'homme de l'art, des séquences microsatellites, et à hybrider les produits d'amplification sur une biopuce telle que décrite ici. Ces séquences microsatellites sont des séquences répétées, hautement polymorphiques, présentes à intervalles réguliers dans le génome humain, et flanquées de séquences permettant le positionnement d'amorces de PCR (Goldstein, D. and C. Schlotterer, eds. 1999. Microsatellites: Evolution and Applications. Oxford University Press). Les séquences microsatellite à amplifier sont choisies dans la zone putative de délétion, de façon à être amplifiées lorsque la délétion est absente, et à ne pas générer de produits de PCR lorsque la délétion est présente (pour la sélection des séquences microsatellites et leurs amorces de PCR : Genome Database, http://www.gdb.org). Les séquences microsatellites des cellules saines et présumées tumorales sont individuellement amplifiées et marquées en utilisant des amorces de PCR marquées différemment pour chacun des types, sain ou tumoral. Une amplification multiple des marqueurs microsatellites (PCR multiplex) est préférée. De une à plusieurs séquences microsatellites peuvent être ainsi amplifiées en fonction de la pathologie et de l'analyse à réaliser. Un marquage préféré est obtenu avec l'amplification des cellules saines avec une amorce de PCR marquée à la Cy3 et l'amorce complémentaire non marquée, et l'amplification des cellules tumorales avec une amorce de PCR marquée à la Cy5 et l'amorce complémentaire non marquée. Les produits de PCR de chacun des marqueurs microsatellite, marqués à la Cy3 pour les cellules saines, et à la Cy5 pour les cellules tumorales sont alors traités pour éliminer les amorces de PCR n'ayant pas été incorporées lors de l'amplification. Plusieurs méthodes d'élimination des amorces sont envisagées, avec une préférence pour un traitement à l'exonucléase I et la phosphatase alcaline de crevette (exo/sap), selon le protocole fourni par exemple dans le kit exo/sap ExoSAP-IT (USB Corporation, 26111 Miles Road, Cleveland Ohio 44128, USA).

A l'issu de ce traitement, les produits d'amplification des séquences microsatellites sont réunis en une seule solution, et hybridés sur une biopuce de sondes telle que décrite ici. Cette étape de rassemblement des produits d'amplification des séquences microsatellite peut également se faire avant le traitement d'élimination des amorces. La biopuce décrite ici comporte au moins une sonde. Cette sonde est au moins partiellement complémentaire de l'une des amorces de PCR utilisées lors de l'amplification de la séquence microsatellite, et de façon préférée, parfaitement complémentaire d'une partie ou de la totalité de l'amorce. Plusieurs types de sondes peuvent être utilisés sur la biopuce décrite ici, ou de façon préférée des sondes en épingle à cheveux décrites ici modifiées par ajout d'un espaceur, ou encore des sondes du système du SAU. La séquence de la sonde de la méthode préférée est obtenue en suivant les critères de la présente description, notamment lorsque plusieurs sondes sont utilisées avec plusieurs séquences microsatellite à analyser, la Tm de tous les hybrides formés entre les séquences cibles des sondes et les séquences microsatellites qui leur sont complémentaires, est égale à plus ou moins 4 ˚C près, de préférence, plus ou moins 1 ˚C près. De même, lorsque plusieurs sondes sont utilisées sur une biopuce, un des bras de toutes les sondes dans la méthode préférée peuvent avoir la même séquence. Lorsque plusieurs séquences microsatellites doivent être analysées, si cela nécessite la présence sur la biopuce de plusieurs sondes, celles-ci peuvent être de plusieurs types, c'est-à-dire des sondes linéaires et/ou des sondes en épingle à cheveux, et/ou des sondes du système SAU.

Dans la méthode préférée, des sondes du système SAU sont utilisées pour incorporer des témoins négatifs d'amplification lors de la PCR des séquences microsatellites. Ces témoins négatifs sont réalisés en amplifiant une séquence microsatellite en absence d'ADN génomique des cellules saines ou tumorales, mais en présence des réactifs nécessaires à la PCR, et d'un couple d'amorces dont au moins une constitue une première sonde linéaire du SAU qui possède une séquence étiquette parfaitement complémentaire de la séquence spécifique de la cible de la deuxième sonde immobilisée.

Lors de l'hybridation d'un produit de PCR issus de l'amplification d'une séquence microsatellite des cellules saines et présumées tumorales sur une sonde de la biopuce, la sonde de la biopuce s'hybride avec les séquences des amorces de PCR, présentes aux extrémités des produits de PCR des cellules saines et tumorales. Le marquage différentiel en Cy3 et Cy5 des amplicons issus de ces cellules conduit à la production pour chaque sonde sur la puce d'un signal composé d'une émission pour la Cy3 due à l'hybridation de la sonde avec un produit de PCR issu de cellules saines, et d'une émission pour la Cy5, due à l'hybridation de la sonde avec un produit de PCR issu de cellules présumées tumorales. En mesurant la part relative d'émission pour chacun des marqueurs, on en déduit la quantité relative de produits de PCR spécifiques d'une séquence microsatellite issue de cellules tumorales et saines. Les séquences microsatellites analysées n'étant pas amplifiées lorsqu'une délétion se produit dans la région chromosomique d'où est issu le microsatellite, on observe en conséquence une diminution de l'émission en Cy3 (cellule présumée tumorale), par rapport à l'émission en Cy5 (cellule saine) lorsque cette délétion a lieu.

Dans cette méthode, l'analyse de la quantité relative de produits de PCR pour l'amplification d'une séquence microsatellite est obtenue en comparant les valeurs d'émission des signaux en Cy5 et Cy3 après hybridation, avec une courbe de calibration interne. Cette courbe de calibration est obtenue par amplification et hybridation compétitive sur les sondes de la biopuce décrite ici, de l'ADN de marqueurs microsatellite non délétés, localisés sur le chromosome Y (marqueurs d'hétérozygotie) et d'autres marqueurs situés sur d'autres chromosomes, et non délétés (marqueurs d'homozygotie). Dans la méthode préférée, une amplification de ces marqueurs est effectuée sur les deux types d'échantillons (cellules saines et présumées tumorales) en même temps que l'amplification des autres marqueurs microsatellite, en respectant le marquage différentiel avec la Cy3 et la CY5, comme décrit plus haut. Les ADNs utilisé pour amplifier les marqueurs d'hétérozygotie et d'homozygotie peuvent provenir d'une extraction de l'ADN à partir de cellules saines et tumorales de l'individu ou de préparations extemporanées de ces ADN à partir d'individu de sexe mâle, de génotypes connus (ne contenant pas de délétions pour ces marqueurs) pour les marqueurs microsatellites testés dans cette amplification.

Alternativement, la courbe de calibration peut être obtenue en amplifiant par PCR deux ou plus marqueurs microsatellites non délétés dans la pathologie considérée. Ces marqueurs sont amplifiés en présence d'une dilution en série des ADN extraits de chacun des deux types cellulaires comparés (cellules saines, et présumées tumorales), et en même temps que les autres marqueurs microsatellite. La courbe de calibration est établie, après réalisation de l'analyse en reportant sur un graphe ayant en abscisse l'intensité de fluorescence en Cy3 et en ordonnée l'intensité de fluorescence en Cy5, les valeurs respectives de ces deux fluorescences pour chacune des sondes de la biopuce correspondant aux marqueurs de calibration.

L'analyse de chacun des produits de PCR en terme de présence ou d'absence d'un déséquilibre allélique se fait en reportant sur ce même graphe les valeurs de fluorescence en Cy5 et Cy3 obtenues après hybridation des produits d'amplification des séquences satellites sur la biopuce.

La description décrit également des Kits de biopuces telles que décrites ici, de sondes et réactifs y compris réactifs de RT-PCR pour l'analyse de déséquilibres alléliques dans les :

- Cancers de la prostate, du rein, de la vessie
- cancers du sein
- cancers du colon
- cancers du poumon
- Analyse de la présence de délétions importantes dans des maladies héréditaires comme la Myophatie de Duchenne) et maladies acquises.

COMMENTAIRES DES FIGURES:

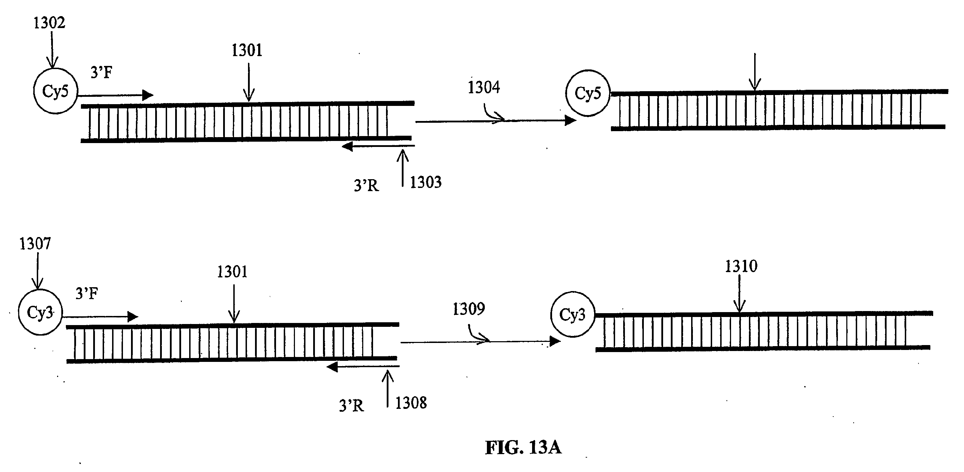

FIG. 13A : L'amorce de PCR 1302, marquée à son extrémité 5' par une molécule fluorescente (CY5), est localisée en 3' du brin sens de la séquence microsatellite 1301, et est utilisée avec l'amorce 1303, non marquée, positionnée en 3' du brin antisens de la séquence microsatellite 1301, pour amplifier cette séquence microsatellite dans les échantillons d'ADN génomiques extraits de cellules saines 1304 , et produire le produit de PCR 1305.

L'amorce de PCR 1307, marquée à son extrémité 5' par une molécule fluorescente (CY3), est localisée en 3' du brin sens de la séquence microsatellite 1301, et est utilisée avec l'amorce 1308, non marquée, positionnée en 3' du brin antisens de la séquence microsatellite 1301, pour amplifier cette séquence microsatellite dans les échantillons d'ADN génomiques extraits de cellules présumées tumorales 1309, et produire le produit de PCR 1310.

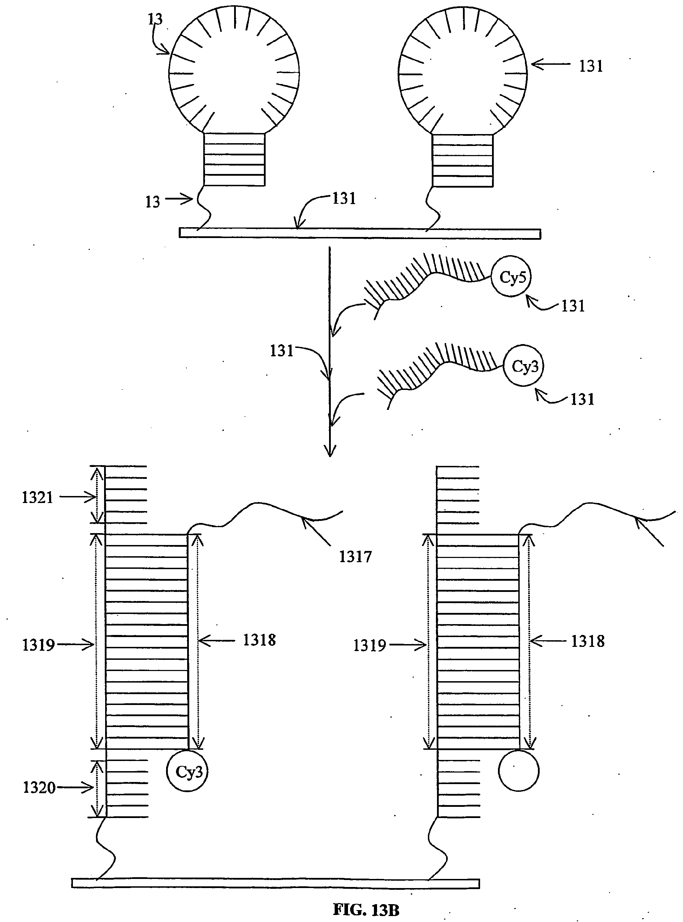

FIG. 13B: La biopuce de sonde est mise en contact 1315 avec le mélange contenant les produits de PCR issus des étapes d'amplification 1304 et 1309 de la FIG3A. La biopuce de sonde est représentée avec deux sondes en épingle à cheveux 1311 et 1314, identiques, toutes deux immobilisées sur le support 1312 via un espaceur 1313. La séquence spécifique de la cible 1319 de ces deux sondes est parfaitement complémentaire de la séquence des amorces de PCR sens, 1318 incorporées dans les produits de PCR 1316 et 1317. Le produit de PCR 1316 est marqué à la Cy5, et le produit de PCR 1317 à la Cy3. Le produit de PCR 1316 est obtenu par dénaturation du produit de PCR 1305 (FIG. 3A), et le produit de PCR 1317, par dénaturation du produit de PCR 1310 (FIG.3A).

KITS DE BIOPUCES EN EPINGLE A CHEVEUX

La description décrit également la fourniture de kits pour détecter la présence de molécules cibles dans un échantillon. De tels kits comprennent typiquement deux ou plus composants nécessaires à la réalisation de l'analyse. De tels composants peuvent inclure une biopuce de sondes en épingle à cheveux, et un ou plusieurs réactifs additionnels nécessaires pour détecter des molécules cibles sélectionnées. Alternativement, le kit peut comprendre un jeu de sondes en épingle à cheveux immobilisées et/ou non immobilisées ainsi que des réactifs tels que ceux utilisés pour le SAU. Un kit préféré comprend la biopuce de sondes en épingle à cheveux, un jeu de sondes en épingle à cheveux non-immobilisées et/ou une molécule «rapporteur» et un ou plus réactifs additionnels. Un kit autre préféré comprend la biopuce de sondes linéaires, un jeu de sondes en épingle à cheveux non-immobilisées et/ou une molécule «rapporteur» et un ou plus réactifs additionnels.

EXEMPLES : HYBRIDATIONS D'ADN CIBLE AVEC DES SONDES EN EPINGLE A CHEVEUX IMMOBILISEES

Les exemples qui suivent ne sont présentés que pour illustrer la présente invention, et ne doivent pas être interprétés comme des limitations de l'invention de quelque manière que ce soit. Les exemples présentés ci-dessous décrivent l'utilisation de biopuces à sondes en épingle à cheveux pour la détection de séquences cibles.

LES MOLECULES D'ACIDES NUCLEIQUES

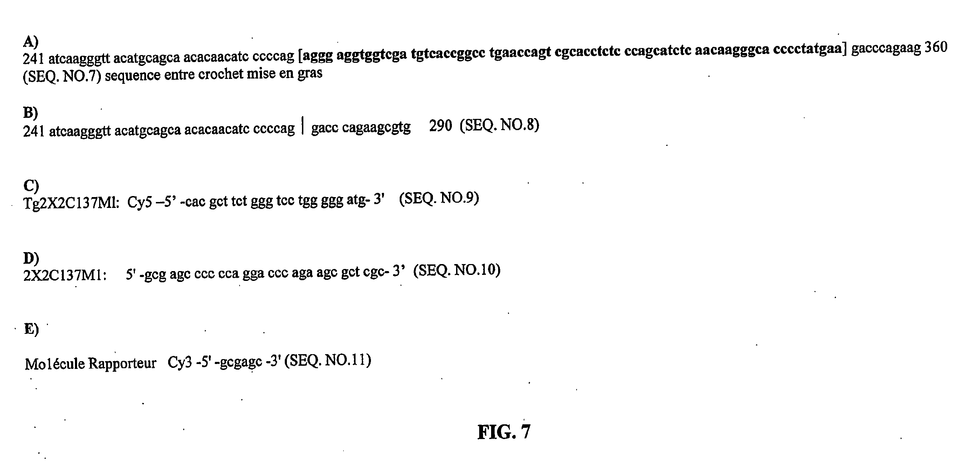

La mutation du codon 137 dans l'exon 2 du gène TCF2 est une délétion de 75 paires de bases qui a pour résultat l'apparition d'un phénotype diabétique de type II, associé à un dysfonctionnement rénal grave (Linder, T. et al. 1999. Hum. Molec.Genet. 8:2001-8). La séquence cible utilisée dans cet exemple correspond à la région 266 à 290 de la séquence délétée du codon 137 dans l'exon 2 du gène TCF2. La FIG.7A montre la position de la mutation du codon 137 de l'exon 2. La partie en gras et entre crochet comprend les bases 276 à 350, qui sont délétées chez les individus mutants. La FIG.7B montre la séquence de l'exon 2 portant la mutation.

La séquence cible Tg2X2C137M1, utilisée dans cet exemple, FIG.7C, est la séquence complémentaire ou brin négatif de la séquence en gras de la FIG.7B. La séquence spécifique de la sonde en épingle à cheveux 2X2C137M1, montrée dans la FIG.7D, a été conçue en sélectionnant 18 nucléotides dans le centre de la séquence cible mutante, parfaitement complémentaire de la séquence cible. La molécule «rapporteur» conçue pour cette analyse apparaît sur la FIG.7E et possède une température de fusion de 23˚C en présence d'une solution de sodium à 1M, et d'une concentration de la solution d'oligonucléotides de 1μM. La tige de la sonde en épingle à cheveux 2X2C137M1 a une Tm de 72˚C. La séquence spécifique de la cible des sondes en épingle à cheveux a été dessinée pour avoir une Tm de l'hybride formé entre la séquence spécifique de la cible et la molécule cible de 60˚C ± 2˚C en utilisant le logiciel Meltcalc, avec les paramètres fixés à 100 mM en terme de sodium, et pour une concentration d'oligonucléotides de 100mM (la Tm du duplex Tg2X2C137M1/2X2C137M1 = 60,8˚C).

Le logiciel Meltcalc a été utilisé pour prédire les températures de fusion lors des hybridations d'oligonucléotides. Ce logiciel permet d'ajuster une Tm en jouant sur les variations en concentration saline et en DMSO, ainsi qu'en oligonucléotides. Les sondes en épingle à cheveux de ces exemples ont été conçues en utilisant des concentrations de 0,1mM en sel de sodium, et 0,1mM en oligonucleotides. L'association sur eux même des bras de la sonde en épingle à cheveux a été vérifiée avec mfold, en utilisant les paramètres suivants : température d'association 37˚C, 1 M en sodium, pas de magnésium, correction de type oligomère, 5% de suboptimalité, et un nombre maximum d'association modélisée de 50. Toutes les sondes en épingle à cheveux rentrées dans mfold ont une tige continue longue de 6 nucléotides au maximum, avec une Tm de 65-72˚C déterminée avec les paramètres ci dessus. La Tm des hybrides parfaits formés entre les molécules cibles et les séquences spécifiques des cibles sauvages et mutantes est de 60˚C, avec une ΔTm d'au moins 5˚C entre les hybridations parfaites et imparfaites, et une Tm de 65-72˚C pour toutes les tiges.

## IMMOBILISATION DES SONDES EN EPINGLE A CHEVEUX

Toutes les sondes en épingle à cheveux, synthétisées avec un espaceur aminé à leur extrémité 5' ont été déposées sur des lames de verre activées au N-Hydroxy-succinimide (« NHS »), (NoAb Diagnostics, Mississauga, Ontario, Canada) dans un tampon de dépôt fourni avec les lames, pendant 45 minutes à 30˚C dans une chambre humidifiée. Les groupements NHS restants ont été désactivés par réaction, à température ambiante pendant 30 minutes, avec une solution de saturation fournie également avec les lames. Les lames ont été alors lavées dans du tampon citrate salin à pH7, comprenant 0,1 M en NaCl, et rincées avec de l'eau milliQ.

## HYBRIDATION DES MOLECULES CIBLES

Chaque biopuce de sondes immobilisées a été traitée avec 20μL d'une solution à 400nM de molécule cible, Tg2X2C137M1, marquée à la Cy5, dans du tampon SSC 6X contenant 50% de formamide. Après le dépôt d'une lamelle sur chaque lame, la solution de molécule cible a été laissée à hybrider pendant 1 heure à température ambiante. Chaque biopuce a alors été rincée pendant cinq minutes dans du SSC 4X, puis rincée pendant 2 minutes dans du SSC 0,1X. Chaque lame a alors été analysée dans un scanner à biopuce (Axon Instruments, Inc., Union City, California, USA) en utilisant les longueurs d'ondes d'excitation et d'émission du Cy5 et Cy3.

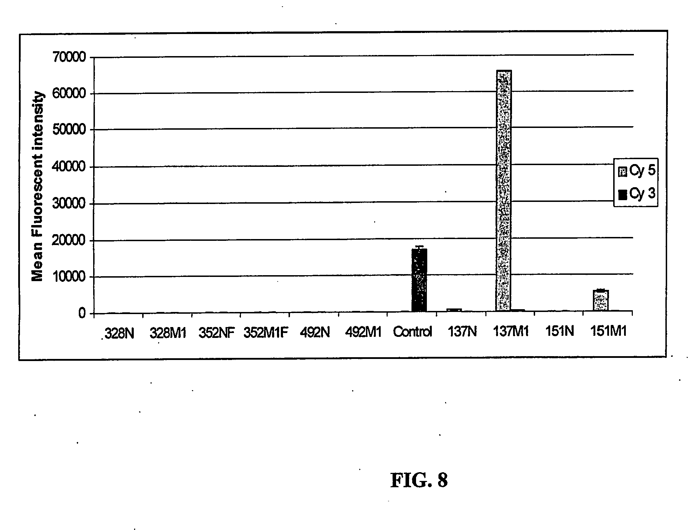

La FIG. 8 montre les intensité de fluorescence de Cy5 et Cy3 après hybridation d'une molécule cible marquée à la Cy5. Chaque valeur est une moyenne des mesures de 5 répliques pour chaque sonde en épingle à cheveux. Les dix sondes en épingle à cheveux utilisées dans ce criblage sont conçues pour analyser cinq mutations différentes présentes dans différents exons du gène TCF2. Seul la sonde 2X2C137M1 (« 137 M1 » dans la FIG.8) est parfaitement complémentaire de la cible Tg2X2C137M1. Le contrôle est une séquence simple brin linaire marquée en 5' à la Cy3 et modifiée par un espaceur aminé à son extrémité 3'. Ce contrôle est utilisé pour positionner les dépôts, et pour contrôler la qualité du dépôt. Le signal en Cy5 associé à l'hybridation de la molécule cible marquée Cy5 est observé essentiellement sur les dépôts correspondant aux séquences parfaitement complémentaires de la cible, la boucle de la sonde en épingle à cheveux 2X2C137M1.

## HYBRIDATION DE LA MOLECULE «RAPPORTEUR»

A la suite de l'hybridation avec la molécule cible, chaque biopuce à sondes en épingle à cheveux immobilisées a été traitée avec 20μL d'une solution 1M de molécule «rapporteur» marquée à la Cy3 dans un tampon SSC 6X contenant 50% de formamide. Après dépôt d'une lamelle sur chaque lame, la solution de molécule «rapporteur» a été laissée à hybrider pendant 2 heure à température ambiante. Chaque lame a été ensuite lavée deux fois pendant cinq minutes dans du tampon SSC 4X, puis rincée pendant 2 minutes dans du SSC 0,1X.Chaque lame a alors été analysée dans un scanner à biopuce en utilisant les longueurs d'ondes d'excitation et d'émission du Cy5 et Cy3.

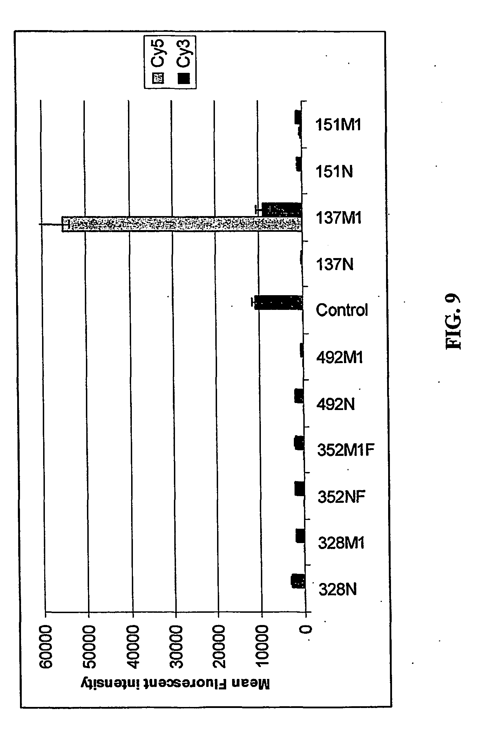

La FIG. 9 montre les intensités de fluorescence moyennes de Cy5 et Cy3 calculée sur la base des mesures effectuées sur 5 répliques, après hybridation avec la molécule «rapporteur» marquée à la Cy3. Les signaux Cy3 associés à l'hybridation de la molécule «rapporteur» marquée à la Cy3, s'observent essentiellement sur les points où la séquence cible Tg2X2C137M1 marquée à la Cy5, est hybridée avec la séquence qui lui est parfaitement complémentaire, i.e. la boucle de la sonde en épingle à cheveux 2X2C137M1. Les résultats montrent que la molécule «rapporteur» se fixe préférentiellement sur les sondes ouvertes, et que ces sondes ouvertes ne peuvent être observées que lorsque les molécules cibles sont hybridées avec les sondes en épingle à cheveux contenant la séquence spécifique de la cible.

SPECIFICITE DES SONDES EN EPINGLE A CHEVEUX

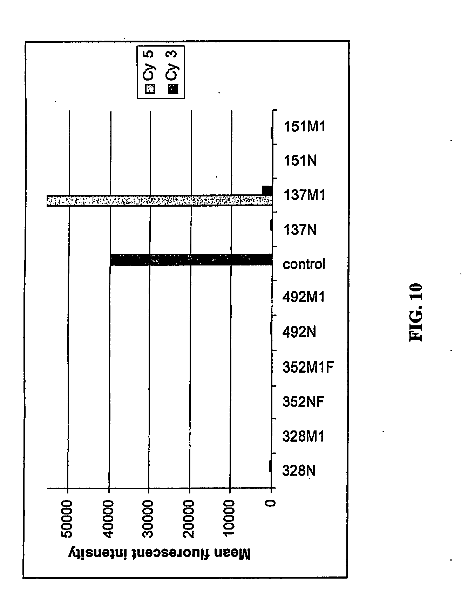

Les interactions non spécifiques entre les sondes en épingle à cheveux et les séquences cibles peuvent être réduites en faisant varier les conditions de l'hybridation. Dans la FIG. 8, on observe une interaction non spécifique évidente entre la cible marquée CY5, et la sonde 151M1. Dans la FIG. 9, une interaction non spécifique évidente est observée entre la molécule «rapporteur» et toutes les sondes en épingle à cheveux, à l'exception de la sonde 2X2C137M1. Une des façons de supprimer les interactions non spécifiques est l'ajout d'étapes de lavages. La FIG. 9 montre qu'avec des lavages supplémentaires, l'interaction non spécifique entre la séquence cible marquée à la CY5, et la sonde en épingle à cheveux 151M1 est réduite. Une autre méthode de diminuer les interactions non spécifiques est d'utiliser des conditions plus stringentes. La FIG. 10 montre comment un lavage plus stringent de 15 à 30 minutes avec du tampon SSC 4X à 28˚C, qui est la valeur de la Tm du «rapporteur» +5˚C, supprime la plupart des interactions non spécifiques entre la molécule «rapporteur» et toutes les sondes en épingle à cheveux, à l'exception de la sonde en épingle à cheveux 2X2C137M1.

DISCRIMINATION D'UN NUCLEOTIDE AVEC LES SONDES EN EPINGLE A CHEVEUX

Le TABLEAU II montre les séquences de seize sondes en épingle à cheveux qui ont été conçues pour analyser les mutations des gènes de la lipase hépatique (« LIPC »), de la Cholestérol ester transférase (« CEPT »), de la lipoprotéine lipase (« LPL »), et de TCF1. Sont résumés dans le TABLEAU II le nom de chaque sonde en épingle à cheveux, les correspondances avec les noms des gènes et les codons, la séquence de chaque sonde en épingle à cheveux, et le nombre et le type de mésappariements que la sonde en épingle à cheveux présente lorsqu'elle est hybridée avec les molécules cibles. Les séquences spécifiques de la cible des sondes en épingle à cheveux sont soit parfaitement complémentaires de la séquence cible spécifiée, soit ne sont pas parfaitement complémentaires et présentent un ou deux mésappariements, ou ne sont pas du tout complémentaires de la molécule cible, ce cas étant indiqué par «- ». Les sondes en épingle à cheveux ont été conçues en sélectionnant une séquence de boucle de 18 à 24 nucléotides complémentaires de la séquence cible comportant un polymorphisme d'un nucléotide. Tous les variants de SNP ont été positionnés au centre de la boucle (caractères gras) avec de 9 à 11 nucléotides bordant la position polymorphique. La Tm de chaque boucle sauvage ou mutante hybridée avec sa séquence cible a été fixé à 60˚C $\pm$ 2˚C en utilisant le logiciel Meltcalc avec les paramètres de concentration fixés à 100mM en sodium, et 100mM en oligonucléotides. La séquence des sondes en épingle à cheveux a été ajustée en ajoutant et/ou en retirant un ou plus nucléotide à chaque extrémité de la boucle pour atteindre la Tm de 60˚C, avec une variation de la température de fusion entre hétéroduplexes et homoduplexes d'au moins 5˚C.

L'immobilisation des sondes en épingle à cheveux a été effectuée comme décrit plus haut. Chaque biopuce de sondes en épingle à cheveux immobilisées a été traitée avec 20µL d'une solution de tampon de sodium 1M, contenant 400nM de cible M19 marquée à la Cy3, et 400nM de cible 142CC marquée à la Cy5, pendant une heure dans du tampon SSC 6X, à 20˚C, et sous une lamelle de verre. La biopuce a alors été rincée pendant 5 minutes dans du SSC 4X, et ensuite lavée dans du SSC 0,1X pendant deux minutes. La biopuce a alors été analysée comme indiqué plus haut en utilisant les longueurs d'ondes d'excitation et d'émission de Cy5 et Cy3, et en utilisant un facteur de photomultiplication (« PMT ») de 600×600 pour chaque fluorochrome.

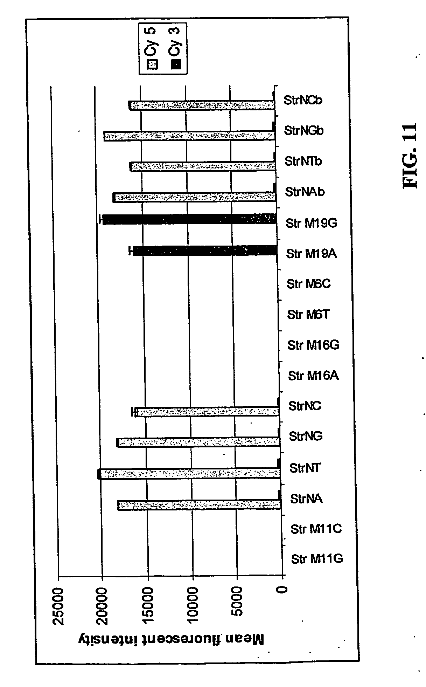

La FIG. 11 montre les intensités de fluorescence moyennes de Cy5 et Cy3 calculées sur la base des mesures de 5 répliques, après hybridation des séquences cibles marquées à l'aide de CY5 et Cy3. Le signal en Cy3 correspond à l'hybridation de la cible M19, et le signal en Cy5 correspond à l'hybridation de la cible 142 CC. Seules les sondes en épingle à cheveux partiellement ou totalement complémentaires des séquences cibles ont été capables de générer un signal lors de l'hybridation des séquences cibles dans des conditions de faible stringence. Pour discriminer les héteroduplexes des homoduplexes, chaque biopuce a été lavée pendant 20 minutes dans du tampon SSC 6X, porté à 50˚C, puis lavées avec du SSC 6X, et de nouveau scannée aux longueurs d'ondes de fluorescence de la CY5 et Cy3, avec un PMT de 600x600, pour les deux fluorochromes.

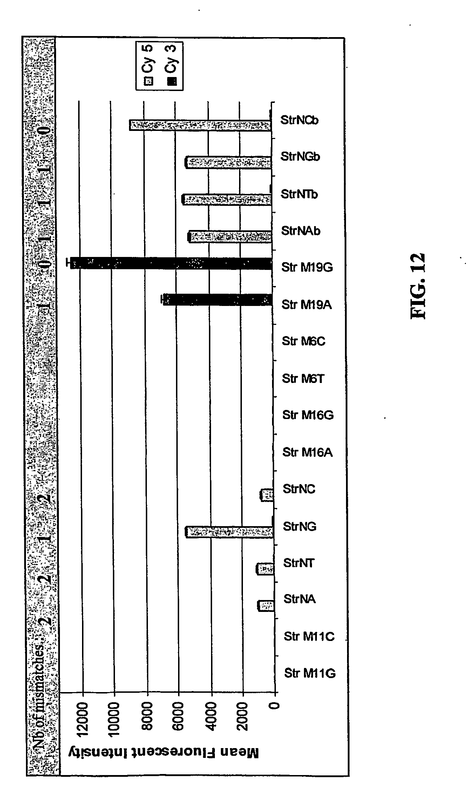

La FIG. 12 montre les intensités de fluorescence moyennes en Cy5 et Cy3 calculées sur la base des mesures effectuées pour 5 répliques, après avoir soumis chaque biopuce à une étape de lavage dans des conditions plus stringente. Les sondes en épingle à cheveux parfaitement complémentaires des cibles marquées, StrM19G, et StrNCb ont le niveau d'hybridation le plus élevé. Les sondes en épingle à cheveux ayant un seul mésappariement ont un signal d'hybridation plus faible mais intermédiaire, alors que les sondes présentant deux mésappariements ont le signal d'hybridation le plus faible. Cette différentiation lors de l'hybridation est obtenue grâce à une plus grande stabilité des hybrides parfaits formés entre les sondes en épingle à cheveux et les séquences cibles.

<u>TABLEAU II</u>

| Gène / Numéro de Codon <u>dans</u> <u>le</u> gène | <u>Nom</u> de la sonde | Séquence | Mésappariement avec la cible | SEQ ID No |
|---|---|---|---|---|
| LPL / 477 S447XC/G | StrM11C | 5'-GCG AGC **GAA TAA GAA GTA GGC TGG TGA GC** GCT CGC-3' | - | 12 |
| | StrM11G | 5' -GCG AGC **GAA TAA GAA GTA GGC TGG TGA GC** GCT CGC- 3' | - | 13 |
| LPL / 188 Glyl88Glu G/A | StrM16G | 5' -GCG AGC **CAC CAG AGG GTC CCC TGG** GCT CGC- 3' | - | 14 |
| | StrM16A | 5' -GCG AGC **CAC CAG AGA GTC CCC TGG** GCT CGC- 3' | - | 15 |
| LIPC / 480 C-480T C/T | StrM6C | 5' -GCG AGC **TTT TGA CAG GGG GTG AAG G** GCT CGC- 3' | - | 16 |
| | StrM6T | 5' -GCG AGC **TTT TGA CAG GGG GTG AAG G** GCT CGC- 3' | - | 17 |
| CETP IV / WIAF-10949 G/A | StrM 19G | 5. -GCG AGC **CCG AGT CCG TCC AGA GCT** GCT CGC- 3' | cible M19 : Complémentarité parfaite | 17 |
| | StrM 19A | 5' -GCG AGC **CCG AGT CCA TCC AGA GCT** GCT CGC- 3' | cible M19 : 1 mésappariement C/A | 19 |

(suite)

| Gène / Numéro de Codon dans le gène | Nom de la sonde | Séquence | Mésappariement avec la cible | SEQ ID No |
|---|---|---|---|---|
| TCF1 / 142 | StrNCb | Voir Tableau I | cible 142CC : Complémentarité parfaite | 1 |
| | StrNTb | Voir Tableau I | cible 142CC : 1 mésappariement T/G | 2 |
| | StrNGb | Voir Tableau I | cible 142CC : 1 mésappariement G/G | 3 |
| | StrNAb | Voir Tableau I | cible 142CC : 1 mésappariement A/G | 4 |
| | StrNC | 5' -GCG AGC **CAA CCA CTC CAC CTG TC** GCT CGC- 3' | cible 142CC: 1 mésappariement T/C | 20 |
| | StrNT | 5' -GCG AGC **CAA CCA TTC CAC CTG TC** GCT CGC- 3' | cible 142CC: 2 mésappariements T/G et T/C | 21 |
| | StrNG | 5' -GCG AGC **CAA CCA GTC CAC CTG TC** GCT CGC- 3' | cible 142CC: 2 mésappariements G/G et T/C | 22 |
| | StrNA | 5' -GCG AGC **CAA CCA ATC CAC CTG TC** GCT CGC- 3' | cible 142CC: 2 mésappariements G/G et T/C | 23 |

EXEMPLE DE CONCEPTION DE SONDES EN EPINGLE A CHEVEUX

Les sondes répertoriées dans le tableau III, ont été conçues pour analyser des mutations présentes chez des individus affectés de la maladie de Charcot Marie Tooth.

La maladie de Charcot Marie Tooth est la maladie héréditaire du système nerveux périphérique la plus fréquente. De nature hétérogène (multigénique), elle se traduit par une cohorte de manifestation neuropathiques motrices et sensorielles (http://molgen-www.uia.ac.be/CMTMutations/CMT.cfm).

Au niveau moléculaire, 14 gènes sont connus actuellement pour être impliqués, via leurs mutations, dans l'apparition des neuropathies de Charcot Marie Tooth. Parmi ceux ci, les gènes de la protéine 22 de la myéline périphérique (PMP22), de la protéine zéro de la myéline (MPZ), et de la connexine 32 (GJB1), dont les mutations sont dominantes, sont utilisés dans cet exemple pour mettre au point des sondes permettant l'analyse de 5 mutations, répertoriées ci dessous :

| Nom Mutation | Type | Gène |
|---|---|---|
| C42R T>C | Substitution T>C | PMP22 |
| W140R T>C | Substitution T>C | PMP22 |
| T124M C>T | Substitution C>T | MPZ |
| V113F G>T | Substitution G>T | MPZ |
| S26L C>T | Substitution C>T | GJB1 |

La conception de ces sondes a été faite selon les étapes indiquées dans la section conception des sondes dans la description détaillée de l'invention.

Ces étapes sont :

1- Détermination de la séquence des boucles des sondes mutantes et sauvages, et calcul des Tm des hybrides parfaits et imparfaits avec le logiciel Meltcalc.

2- Ajout des bras des sondes (tiges), vérification de la structure des épingles à cheveux et calcul du Tm des épingles à cheveux avec Mfold.

3- Vérification de l'alignement des sondes sur leurs cibles respectives, et de l'absence d'homologie entre épingles à cheveux dessinés et les cibles non complémentaires (Blastn).

L'ensemble des sondes dessinées pour analyser ces mutations sont répertoriées dans le tableau III. Ces sondes ont été utilisées pour concevoir une biopuce, suivant la procédure suivante :

Chaque sonde a été synthétisée avec un groupement aminé en 5' (Sigma Genosys, UK). Ces sondes sont déposées par spotting (robot microgrid II Biorobotics) sur des lames de verre activées (Genescore, France). Lors du dépôt, une liaison covalente est établie entre le support de verre et l'extrémité aminé de la sonde. Cinq répliques de chaque dépôt ont été effectués.

Les biopuces ainsi constituées ont été utilisées pour hybrider un ensemble de cibles oligonucléotidiques (tableau IV) parfaitement complémentaires des sondes indiquées dans le tableau III et marquées par une molécule fluorescente (Cyanine 3) à leur extrémité 5' (Sigma Genosys).

**Tableau III**

| Sondes Epingles à Cheveux | 5'-3' | SEQ ID No |
|---|---|---|
| Neu1S26LW6 | GCGAGCAGTATGGCTCTCGGTCATGCTCGC | 24 |
| Neu1S26LM6 | GCGAGCAGTATGGCTCTTGGTCATGCTCGC | 25 |
| Neu4V113FW2 | GCGAGCGCTCCATTGTCATACACAAGCTCGC | 26 |
| Neu4V113FM2 | GCGAGCGCTCCATTTTCATACACAAGCTCGC | 27 |
| Neu4T124MW3 | GCGAGCCAATGGCACGTTCACTTGCTCGC | 28 |
| Neu5C42RW1 | GCGAGCCAGAACTGTAGCACCGCTCGC | 29 |
| Neu5C42RM1 | GCGAGCCAGAACCGTAGCACCGCTCGC | 30 |
| Neu6W140RW4 | GCGACGTCCTGGCCTGGGTGCGTCGC | 31 |
| Neu6W140RM4 | GCGACGTCCTGGCCCGGGTGCGTCGC | 32 |

Deux séries d'expériences ont été réalisées sur deux biopuces différentes. Sur la première biopuce, l'ensemble des cibles correspondant aux allèles sauvages (WT, tableau IV (a)) ont été hybridées. Sur la deuxième biopuce, l'ensemble des cibles correspondant aux allèles mutants (MT, tableau IV (b)) ont été hybridées.

**Tableau IV (a)**

| Cibles WT | 5'-3' | SEQ ID No |
|---|---|---|
| CiS26LW | ATGAAGATGACCGAGAGCCATACTCGGCCA | 33 |
| CiV113FW | TCTAGGTTGTGTATGACAATGGAGCCATCC | 34 |
| CiT124MW | CGTCACAAGTGAACGTGCCATTGTCACTGT | 35 |
| CiC42RW | GAAGAGGTGCTACAGTTCTGCCAGAG | 36 |
| CiW140RW | GAAGGCCACCCAGGCCAGGATGTAGG | 37 |

**Tableau IV (b)**

| Cibles MT | 5'-3' | SEQ ID No |
|---|---|---|
| CiS26LM | ATGAAGATGACCAAGAGCCATACTCGGCCA | 38 |

(suite)

| Cibles MT | 5'-3' | SEQ ID No |
|-----------|-------|-----------|
| CiV113FM | TCTAGGTTGTGTATGAAAATGGAGCCATCC | 39 |
| CiC42RM | GAAGAGGTGCTACGGTTCTGCCAGAG | 40 |
| CiW140RM | GAAGGCCACCCGGGCCAGGATGTAGG | 41 |

Pour chacune de ces hybridations, les conditions étaient : 100 nM en cibles dans du tampon SSC 6X, 1M NaCl, hybridés 1 h RT, 20μL de cible par puce. Valeur moyenne de 5 réplicats, scanning sur scanner GST lumonics, laser et PMT à 500X500.

Les valeurs médianes de fluorescence après scanning ont été analysées dans chaque série d'expériences pour les sondes sauvages et mutantes, et utilisées pour calculer le ratio de discrimination de chaque sonde (Rd).

Le ratio de discrimination des sondes (Rd) est défini par :

Rd = Intensité de fluorescence hybride parfait/ Intensité de fluorescence hybride imparfait

Avec intensité de fluorescence hybride parfait = intensité mesuré lors de l'hybridation de la cible parfaitement complémentaire de l'épingle à cheveux considérée (hybridation par exemple de la cible CiS26LW sur l'épingle à cheveux Neu1S26LW6, ou hybridation de la cible CiS26LM sur l'épingle à cheveux Neu1S26LM6) et intensité de fluorescence hybride imparfait = intensité mesurée lors de l'hybridation de la cible différent d'une base (hybridation par exemple de la cible CiS26LW sur l'épingle à cheveux Neu1S26LM6, ou hybridation de la cible CiS26LM sur l'épingle à cheveux Neu1S26LW6).

Le ratio de discrimination (Rd) a la signification suivante :

par exemple un Rd de 10 pour une épingle à cheveux donnée signifie que l'hybridation d'une cible parfaitement complémentaire de cette épingle à cheveux donnera un signal 10 fois plus élevé que l'hybridation d'une autre cible différent d'une base de la première.

Ces valeurs de Rd sont reportées dans le tableau V, ainsi que la valeur du Tm des boucles, et des tiges de chaque épingle à cheveux, calculées.

Le paramètre Dtm est également calculé pour chacune de ces sondes, avec Dtm = Tm tige - Tm boucle. Ce paramètre traduit la « force relative » de discrimination des sondes. Plus le Dtm est grand (valeur de Tm de la tige élevé, et valeur de Tm de la boucle faible), plus la capacité à discriminer deux séquences d'homologie élevée sera importante.

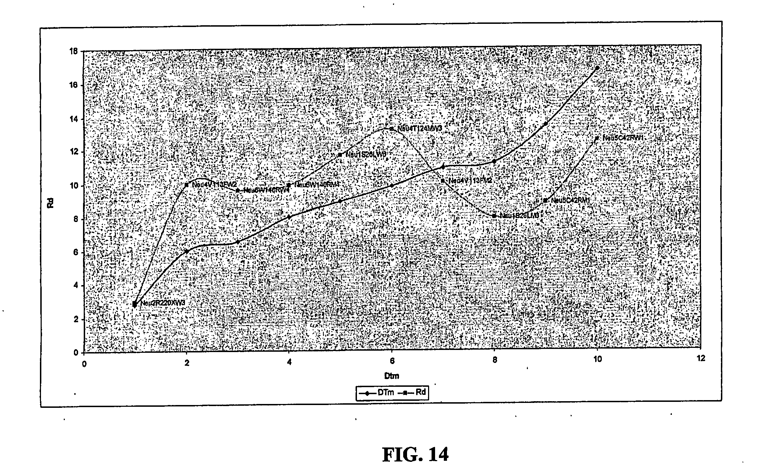

L'ensemble de ces valeurs ont été portées dans un graphique (FIG. 14) qui montre la relation entre l'évolution de la valeur du Dtm et celle du ratio de discrimination Rd.

**Tableau V**

| Nom | Tm boucle | Tm tige | DTm | Rd |
|-----|-----------|---------|-----|-----|
| Neu4V113FW2 | 63,1 | 69,1 | 6 | 9,95 |
| Neu6W140RW4 | 67,3 | 73,8 | 6,5 | 9,61 |
| Neu6W140RM4 | 63,6 | 71,6 | 8 | 9,9 |
| Neu1S26LW6 | 65 | 73,9 | 8,9 | 11,65 |
| Neu4T124MW3 | 62,8 | 72,6 | 9,8 | 13,16 |
| Neu4V113FM2 | 61,1 | 72 | 11 | 10,04 |
| Neu1 S26LM6 | 62,7 | 73,9 | 11 | 7,93 |
| Neu5C42RM1 | 60 | 73,4 | 13 | 8,76 |
| Neu5C42RW1 | 56,7 | 73,4 | 17 | 12,48 |

Cette relation entre Rd et Dtm peut être utilisée pour sélectionner lors de la conception des sondes, des boucles et des tiges de taille et de composition adaptées à l'analyse que l'on souhaite effectuer. Ainsi par exemple, si un Rd de 10 est souhaité pour l'ensemble des sondes à concevoir, la valeur de Dtm qui servira de critère pour la conception des sondes sera de 9.

EXEMPLE DE RESULTAT D'ANALYSE DE MUTATIONS SUR UN ECHANTILLON D'ADN GENOMIQUE HUMAIN

Cet exemple porte plus particulièrement sur l'analyse de l'allèle sauvage de la mutation W140R du gène PMP22 (exon 3) impliqué dans la maladie de Charcot Marie Tooth.

L'ensemble des sondes en épingle à cheveux dont la séquence est donnée dans le tableau III a été immobilisé sur une biopuce.

L'ADN génomique de l'exon 3 du gène PMP22 a été amplifié par PCR en utilisant un couple d'amorces dont une était marquée à son extrémité 5' par une molécule de cyanine 3 fluorescente.

La PCR a été effectuée de la façon suivante : 100 ng d'ADN extrait d'un individu sain ont été utilisés avec un kit de PCR (High fidelity expand PCR, Roche) en suivant le protocole fourni, et les amorces de PCR spécifiques pendant 40 cycles. Un contrôle négatif (puit de PCR sans ADN génomique) a été également inclus lors de la PCR.

Les produits de PCR ont été contrôlés par migration sur gel d'agarose pour vérifier la taille du produit de PCR ainsi que l'absence de contamination dans le contrôle négatif. L'ADN amplifié a été quantifié après purification sur microcolonne (Qiaquick, Qiagen) par adsorption UV à 260 nm.

1,4 $\mu$g d'ADN marqué à la Cyanine 3 issus de la PCR de l'exon 3 du gene PMP22 ont été hybridés 1 heure à température ambiante sur la biopuce portant les sondes en épingle à cheveux.

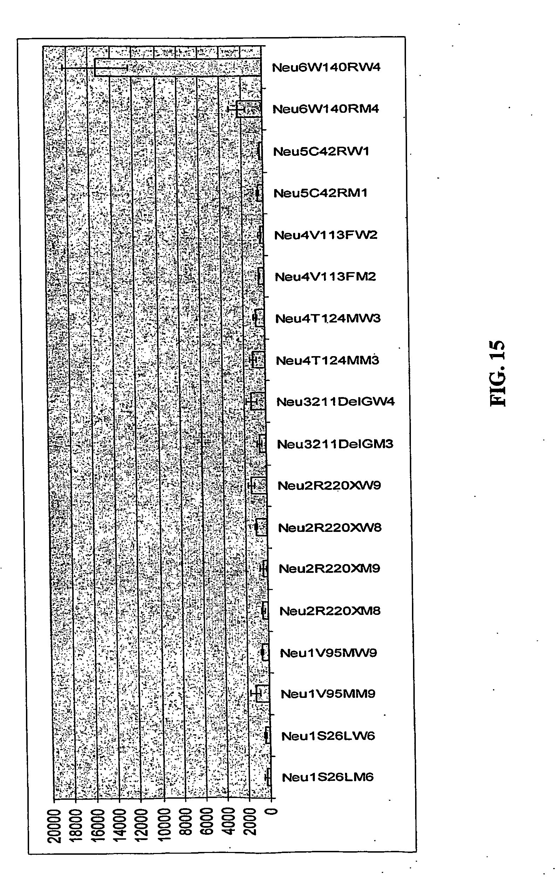

Après hybridation, la biopuce a été scannée et les résultats ont été reportés dans la FIG. 15.

Ces résultats montrent que les produits de PCR sont essentiellement hybridés sur les sonde qui leur sont complémentaires, ou différent d'une base (sondes Neu6W140RM4 et Neu6W140RW4). Le Rd est de 6,61 (signal de la sonde WT/MT).

Le génotype donné par cette analyse est homozygote sauvage pour la mutation W140R, ce qui correspond au phénotype « sain » de l'individu testé.

REFERENCES CITEES

Leur citation ne doit pas être interprétée comme une reconnaissance du fait qu'elles appartiennent à l'état de l'art antérieur de la présente invention.

**Revendications**

1. Kit comprenant

   (i) une biopuce de sondes comprenant un support et au moins deux sondes non marquées, attachées au support, comprenant :

      (a) une séquence spécifique d'une cible, de 6 à 30 nucléotides de long ;
      (b) un premier bras de moins de 10 nucléotides de long, positionné en 5' de la séquence spécifique d'une cible ; et
      (c) un deuxième bras de moins de 10 nucléotides de long, positionné en 3' de la séquence spécifique d'une cible,

   la séquence spécifique de la cible n'étant complémentaire d'aucune autre partie de la sonde; le premier bras et le second bras étant parfaitement complémentaires l'un avec l'autre; les premiers bras desdits au moins deux sondes possédant une séquence identique et les séquences spécifiques de la cible desdites au moins deux sondes étant différentes ;
   (ii) une molécule « rapporteur » comprenant un marqueur détectable et moins de 10 nucléotides parfaitement complémentaires avec la séquence d'acide nucléique du premier ou du second bras de la sonde, ladite molécule « rapporteur » étant capable de s'hybrider avec le premier ou le second bras de la sonde lorsqu'une molécule cible est hybridée avec la séquence spécifique de la cible et que la sonde adopte une conformation « ouverte » ; et
   (iii) un ou plusieurs réactifs additionnels.

2. Kit selon la revendication 1, **caractérisée en ce que** la séquence spécifique de la cible a une longueur de 10-25 nucléotides, de préférence de 15-20 nucléotides.

3. Kit selon l'une des revendications 1 à 2, **caractérisée en ce que** le Dtm (différence entre la température de fusion (Tm) de l'hybride parfait formé entre la séquence spécifique de la cible de la sonde et la molécule cible et la température de fusion (Tm) de l'hybride formé entre le premier bras et le second bras de la sonde) est supérieur à 10, de préférence égal à 15.

4. Kit selon l'une des revendications 1 à 2, **caractérisée en ce que** le Dtm (différence entre la température de fusion (Tm) de l'hybride parfait formé entre la séquence spécifique de la cible de la sonde et la molécule cible et la température de fusion (Tm) de l'hybride formé entre le premier bras et le second bras de la sonde) est inférieur à 10.

5. Kit selon l'une des revendications 1 à 4, **caractérisée en ce que** ladite sonde comprend en outre un espaceur et est attachée audit support par ledit espaceur.

6. Kit selon l'une des revendications 1 à 5, **caractérisée en ce que** le support consiste en une surface de verre fonctionnalisée, une surface de plastique fonctionnalisée, une surface métallique fonctionnalisée, une surface conductrice en métal, une surface conductrice en plastique, un support poreux, un métal poreux, une fibre optique, un support dérivé d'une fibre de verre, du dioxyde de silicium, une membrane lipidique fonctionnelle, un liposome, une membrane de filtration ou des billes ou particules de taille inférieure au micron.

7. Kit selon l'une des revendications 1 à 6, **caractérisée en ce qu'**une même molécule «rapporteur» peut s'hybrider avec chaque sonde.

8. Kit selon l'une des revendications 1 à 7, **caractérisée en ce que** tous les hybrides parfaits formés entre les séquences spécifiques de la cible des sondes et les molécules cibles ont une température de fusion égale, à plus ou moins 4°C près, de préférence à plus ou moins 1 °C près.

9. Kit selon l'une des revendications 1 à 8, **caractérisée en ce que** la différence entre la température de fusion de l'hybride formé entre la séquence spécifique de la cible de chaque sonde et la molécule cible, et la température de fusion pour l'association entre la séquence spécifique de la cible et une molécule pour laquelle la séquence spécifique de la cible de la sonde n'a pas été conçue, est supérieure ou égale à 5°C, de préférence supérieure ou égale à 8°C.

10. Kit selon l'une des revendications 1 à 9, **caractérisée en ce que** les Dtm d'au moins deux sondes (différence entre la température de fusion (Tm) de l'hybride parfait formé entre la séquence spécifique de la cible de la sonde et la molécule cible et la température de fusion (Tm) de l'hybride formé entre le premier bras et le second bras de la sonde) sont égaux, à plus ou moins 1 ˚C près.

**11.** Kit selon l'une des revendications 1 à 10, comprenant en outre un jeu de sondes non marquées et non immobilisées comprenant :

(a) une séquence spécifique d'une cible, de 6 à 30 nucléotides de long ;
(b) un premier bras de moins de 10 nucléotides de long, positionné en 5' de la séquence spécifique d'une cible ; et
(c) un deuxième bras de moins de 10 nucléotides de long, positionné en 3' de la séquence spécifique d'une cible,

la séquence spécifique de la cible n'étant complémentaire d'aucune autre partie de la sonde; le premier bras et le second bras étant parfaitement complémentaires l'un avec l'autre.

**12.** Kit selon l'une des revendications 1 à 11, **caractérisée en ce que** le marqueur détectable est un analogue de nucléotide, un marqueur fluorescent, de la biotine, de l'imminobiotine, un antigène, un co-facteur, du dinitrophenol, de l'acide lipoïque, un composé oléfinique, un polypeptide, une molécule riche en électrons, un enzyme ou un isotope radioactif.

**13.** Méthode de détection d'acide nucléique comprenant :

(a) la mise en contact *ex vivo* d'un échantillon d'acides nucléiques avec une biopuce telle que définie dans le kit selon l'une des revendications 1 à 12; et
(b) la détection d'un signal provenant d'au moins une sonde de la biopuce ayant adopté une conformation ouverte à la suite du contact établi lors de l'étape (a) à l'aide d'une molécule « rapporteur » telle que définie dans le kit selon l'une des revendications 1 à 12.

**14.** Méthode de détection *ex vivo* d'un variant génétique dans un échantillon d'acide nucléique comprenant :

(a) la mise en contact de l'échantillon avec une biopuce telle que définie dans le kit selon l'une des revendications 1 à 12, **caractérisée en ce qu'**au moins une sonde de la biopuce est une sonde spécifique d'un variant génétique, et
(b) la détection d'un signal provenant de la sonde spécifique du variant génétique à l'aide d'une molécule « rapporteur » telle que définie dans le kit selon l'une des revendications 1 à 12, le signal détecté à l'étape (b) indiquant la présence du variant génétique dans l'échantillon d'acide nucléique.

**15.** Méthode selon la revendication 14, **caractérisée en ce que** le variant génétique détecté est un polymorphisme d'un seul nucléotide.

**16.** Méthode de détection *ex vivo* de tout organisme contenant des acides nucléiques ou de tout résidu de ces organismes comprenant :

(a) la mise en contact d'un échantillon d'acide nucléique avec une biopuce telle que définie dans le kit selon l'une des revendications 1 à 12, au moins une desdites sondes étant une sonde spécifique d'acides nucléiques d'un organisme ou de résidus de cet organisme ; et
(b) la détection d'un signal provenant de la sonde spécifique des acides nucléiques d'un organisme à l'aide d'une molécule « rapporteur » telle que définie dans le kit selon l'une des revendications 1 à 12,

le signal détecté dans l'étape (b) indiquant la présence de l'organisme contenant des acides nucléiques ou de résidus de cet organisme.

**17.** Méthode selon la revendication 16, **caractérisée en ce que** l'organisme contenant des acides nucléiques est un virus ou une bactérie.

**18.** Méthode de détection *ex vivo* d'un produit d'épissage alternatif d'un gène dans un échantillon d'acides nucléiques comprenant :

(a) la mise en contact de l'échantillon avec une biopuce telle que définie dans le kit selon l'une des revendications 1 à 12, **caractérisée en ce qu'**au moins une sonde de la biopuce est une sonde en épingle à cheveux spécifique d'un exon du gène ou d'une jonction de deux exons ; et

(b) la détection d'un signal provenant de la sonde spécifique de l'exon ou de la jonction de deux exons à l'aide d'une molécule « rapporteur » telle que définie dans le kit selon l'une des revendications 1 à 12, le signal détecté dans l'étape (b) indiquant la présence du produit d'épissage alternatif du gène dans l'échantillon d'acides nucléiques.

**19.** Méthode selon la revendication 18, **caractérisée en ce que** l'échantillon d'acide nucléique comprend de l'ARNm ou de l'ADNc.

**20.** Une méthode de séquençage *ex vivo* d'un oligonucléotide comprenant :

(a) la mise en contact d'un échantillon contenant l'oligonucléotide avec une biopuce telle que définie dans le kit selon l'une des revendications 1 à 12; et

(b) la détection d'un signal provenant d'au moins une sonde de la biopuce à l'aide d'une molécule « rapporteur » telle que définie dans le kit selon l'une des revendications 1 à 12 ;

le signal détecté dans l'étape (b) étant utilisé pour déterminer la séquence de l'oligonucléotide.

**21.** Méthode selon l'une des revendications 13 à 20, dans laquelle l'échantillon de l'étape (a) est marqué au préalable avec un marqueur détectable.

**22.** Méthode de détection *ex vivo* des déséquilibres alléliques et pertes d'hétérozygoties dans un échantillon d'acide nucléique comprenant :

(a) l'amplification d'au moins une région d'ADN chromosomique de type microsatellite au moyen d'une paire d'amorces à partir d'au moins deux échantillons d'acides nucléiques issus de tissus ou fluides biologiques, au moins un des échantillons provenant de cellules ou de tissus ne présentant pas de déséquilibre allélique ou perte d'hétérozygotie, et chaque tissu ou fluide étant marqué différemment au cours de l'amplification ;

(b) l'élimination de ces amorces après l'amplification ;

(c) la mise en contact desdits produits d'amplification avec une biopuce telle que définie dans le kit selon l'une des revendications 1 à 12, dont au moins une sonde de la biopuce étant complémentaire d'une amorce utilisée pour amplifier ladite région d'ADN chromosomique; et

(d) la détection des signaux provenant d'au moins une sonde de ladite biopuce à l'aide d'une molécule « rapporteur » telle que définie dans le kit selon l'une des revendications 1 à 12 ;

les signaux détectés dans l'étape (d) étant utilisés pour déterminer la présence d'un déséquilibre allélique ou d'une perte d'hétérozygotie dans un des échantillons d'acides nucléiques.

**Claims**

**1.** A kit comprising

(i) a probe biochip comprising a substrate and at least two unlabelled probes attached to substrate, said probes comprising:

(a) a target-specific sequence that is from 6 to 30 nucleotides in length;

(b) a first arm that is less than 10 nucleotides in length and is 5' of the target specific sequence; and

(c) a second arm that is less than 10 nucleotides in length and is 3' of the target specific sequence,

the target-specific sequence being not complementary with any other portion of the probe; the first arm and the second arm being perfectly complementary to each other; the first arms of said at least two probes having an identical sequence and the target-specific sequence of said at least two probes being different;

(ii) a "reporter" molecule comprising a detectable marker and less than 10 nucleotides which are perfectly complementary with the nucleic acid sequence of the first arm or the second arm of the probe, said "reporter" molecule being able to hybridize to the first arm or the second arm of the probe when a target molecule is hybridized with the target-specific sequence and the probe adopts an "open" conformation; and

(iii) one or more additional reagents.

2. The kit according to claim 1, wherein the target-specific sequence is from 10 to 25 nucleotides in length, preferably from 15 to 20 nucleotides in length.

3. The kit according to claim 1 or 2, wherein the Dtm (the difference between melting temperature (Tm) of perfect hybrid formed upon association of the target-specific sequence of the probe with the target molecule and melting temperature (Tm) of hybrid formed by association of the first arm and the second arm of the probe) is greater than 10, preferably equal to 15.

4. The kit according to claim 1 or 2, wherein the Dtm (the difference between melting temperature (Tm) of perfect hybrid formed upon association of the target-specific sequence of the probe with the target molecule and melting temperature (Tm) of hybrid formed by association of the first arm and the second arm of the probe) is lower than 10.

5. The kit according to any one of claims 1 to 4, wherein said probe further comprises a linker, and is attached to substrate by mean of said linker.

6. The kit according to any one of claims 1 to 5, wherein the substrate consists of a functionalized glass surface, a functionalized plastic surface, a functionalized metal surface, a conductive metal surface, a conductive plastic surface, a porous substrate, a porous metal, an optical fiber, a glass fiber derived substrate, silicon dioxide, a functional lipidic membrane, a liposome, a filtration membrane or beads or sub-micron particles.

7. The kit according to any one of claims 1 to 6, wherein one "reporter" molecule can hybridize with each probe.

8. The kit according to any one of claims 1 to 7, wherein all perfect hybrids formed upon association of target-specific sequences of probes with the target molecules have equal melting temperature, within a range of 4°C, preferably within a range of 1°C.

9. The kit according to any one of claims 1 to 8, wherein the difference between melting temperature of hybrid formed upon association of the target-specific sequence of each probe with the target molecule, and melting temperature of hybrid formed upon association of the target-specific sequence with a molecule for which the target specific sequence is not designed is greater or equal to 5°C, preferably greater or equal to 8°C.

10. The kit according to any one of claims 1 to 9, wherein the Dtm of at least two probes (the difference between melting temperature (Tm) of perfect hybrid formed upon association of the target-specific sequence of the probe with the target molecule and melting temperature (Tm) of hybrid formed by association of the first arm and the second arm of the probe) are equal, within a range of 1°C.

11. The kit according to any one of claims 1 to 10, further comprising a set of non-immobilized unlabelled probes comprising:

(a) a target-specific sequence that is from 6 to 30 nucleotides in length;
(b) a first arm that is less than 10 nucleotides in length and is 5' of the target specific sequence; and
(c) a second arm that is less than 10 nucleotides in length and is 3' of the target specific sequence,

the target-specific sequence being not complementary with any other portion of the probe; the first arm and the second arm being perfectly complementary to each other.

12. The kit according to any one of claims 1 to 11, wherein the detectable marker is a nucleotide analog, a fluorescent label, biotin, imminobiotin, an antigen, a cofactor, dinitrophenol, lipoic acid, an olefinic compound, a polypeptide, an electron-rich molecule, an enzyme, or a radioactive isotope.

13. A method of nucleic acid detection comprising:

(a) contacting *ex vivo* a nucleic acid sample with a biochip as defined in the kit according to any one of claims 1 to 12; and
(b) detecting a signal from at least one probe of the biochip which has assumed an open conformation following contacting in step (a) using a reporter molecule as defined in the kit according to any one of claims 1 to 12.

**14.** A method of detecting a genetic variant *ex vivo* in a nucleic acid sample comprising:

(a) contacting the sample with a biochip as defined in the kit according to any one of claims 1 to 12, wherein at least one probe of the biochip is a probe specific of a genetic variant, and
(b) detecting a signal from the probe specific of the genetic variant using a reporter molecule as defined in the kit according to any one of claims 1 to 12, the signal detected in step (b) indicating the presence of the genetic variant in nucleic acid sample.

**15.** The method according to claim 14, wherein the detected genetic variant is a single nucleotide polymorphism.

**16.** A method of detecting *ex vivo* any nucleic acid containing organism or a remnant thereof comprising:

(a) contacting the nucleic acid sample with a biochip as defined in the kit according to any one of claims 1 to 12, at least one of said probes being specific for nucleic acid of an organism or a remnant thereof; and
(b) detecting a signal from the probe specific for a nucleic acid of an organism using a reporter molecule as defined in the kit according to any one of claims 1 to 12,

the signal detected in step (b) indicating the presence of the nucleic acid containing organism or a remnant thereof.

**17.** The method according to claim 16, wherein the nucleic acid containing organism is a virus or a bacterium.

**18.** A method of detecting *ex vivo* an alternative splice product of a gene in a nucleic acid sample comprising:

(a) contacting the sample with a biochip as defined in the kit according to any one of claims 1 to 12, wherein at least one probe of the biochip is a hairpin probe specific for an exon of the gene or specific for a junction of two exons; and
(b) detecting a signal from the specific for an exon of the gene or specific for a junction of two exons using a reporter molecule as defined in the kit according to any one of claims 1 to 12, the signal detected in step (b) indicating the presence of the alternative splice product of the gene in the nucleic acid sample.

**19.** The method according to claim 18, wherein the nucleic acid sample comprises mRNA, or cDNA.

**20.** A method of *ex vivo* sequencing an oligonucleotide comprising:

(a) contacting the sample containing the oligonucleotide with a biochip as defined in the kit according to any one of claims 1 to 12; and
(b) detecting a signal from at least one probe of the biochip using a reporter molecule as defined in the kit according to any one of claims 1 to 12;

the signal detected in step (b) being used for determining the sequence of the oligonucleotide.

**21.** The method according to any one of claims 13 to 20, wherein the sample in step (a) is already labelled with a detectable marker.

**22.** A method of detecting allelic imbalances and loss of heterozygosity *ex vivo* in a nucleic acid sample comprising:

(a) amplifying at least one chromosomal DNA region of microsatellite type, using a pair of primers from at least two nucleic acid samples from biological fluids or tissues, wherein at least one of the samples is from cells or tissues having no allelic imbalance or loss of heterozygosity, and each tissue or fluid is differentially labelled during amplification;
(b) eliminating said primers after amplification;
(c) contacting said amplification products with a biochip as defined in the kit according to any one of claims 1 to 12, at least one probe of the biochip being complementary to a primer used for amplifying said chromosomal DNA region; and
(d) detecting the signals from at least one probe of said biochip using a reporter molecule as defined in the kit according to any one of claims 1 to 12,

the signals detected in step (d) being used to determine the presence of an allelic imbalance or loss of heterozygosity in one of the nucleic acid samples.

**Patentansprüche**

1. Kit, umfassend

   (i) einen Sonden-Biochip, umfassend einen Träger und wenigstens zwei nicht markierte Sondern, die an den Träger gebunden sind und umfassen:

   (a) eine zielspezifische Sequenz mit einer Länge von 6 bis 30 Nukleotiden;
   (b) einen ersten Arm mit einer Länge von wenigstens 10 Nukleotiden, der 5' zur zielspezifischen Sequenz positioniert ist; und
   (c) einen zweiten Arm mit einer Länge von wenigstens 10 Nukleotiden, der 3' zur zielspezifischen Sequenz positioniert ist,

   wobei die zielspezifische Sequenz zu keinem anderen Abschnitt der Sonde komplementär ist; der erste Arm und der zweite Arm zueinander perfekt komplementär sind; die ersten Arme besagter wenigstens zwei Sonden eine identische Sequenz besitzen und die zielspezifischen Sequenzen besagter wenigstens zwei Sonden verschieden sind;
   (ii) ein "Reportermolekül", umfassend einen nachweisbaren Marker und wenigstens 10 Nukleotide, die zu der Nukleinsäure-Sequenz des ersten Arms oder des zweiten Arms der Sonde perfekt komplementär sind, wobei besagtes "Reportermolekül" in der Lage ist, mit dem ersten oder zweiten Arm der Sonde zu hybridisieren, wenn ein Zielmolekül mit der zielspezifischen Sequenz hybridisiert ist und die Sonde eine "offene" Konformation einnimmt; und
   (iii) ein oder mehrere weitere Reagenzien.

2. Kit gemäß Anspruch 1, **dadurch gekennzeichnet, dass** die zielspezifische Sequenz eine Länge von 10 bis 25 Nukleotiden, vorzugsweise von 15 bis 20 Nukleotiden, besitzt.

3. Kit gemäß einem der Ansprüche 1 bis 2, **dadurch gekennzeichnet, dass** die Dtm (Differenz zwischen der Schmelztemperatur (Tm) des perfekten Hybrids, das zwischen der zielspezifischen Sequenz der Sonde und dem Zielmolekül gebildet wird, und der Schmelztemperatur (Tm) des Hybrids, das zwischen dem ersten Arm und dem zweiten Arm der Sonde gebildet wird) größer als 10, vorzugsweise gleich 15, ist.

4. Kit gemäß einem der Ansprüche 1 bis 2, **dadurch gekennzeichnet, dass** die Dtm (Differenz zwischen der Schmelztemperatur (Tm) des perfekten Hybrids, das zwischen der zielspezifischen Sequenz der Sonde und dem Zielmolekül gebildet wird, und der Schmelztemperatur (Tm) des Hybrids, das zwischen dem ersten Arm und dem zweiten Arm der Sonde gebildet wird) kleiner als 10 ist.

5. Kit gemäß einem der Ansprüche 1 bis 4, **dadurch gekennzeichnet, dass** besagte Sonde außerdem einen Spacer umfasst und an besagten Träger mithilfe besagten Spacers gebunden ist.

6. Kit gemäß einem der Ansprüche 1 bis 5, **dadurch gekennzeichnet, dass** der Träger aus einer funktionalisierten Glasoberfläche, einer funktionalisierten Kunststoffoberfläche, einer funktionaliserten Metalloberfläche, einer leitenden Metalloberfläche, einer leitenden Kunststoffoberfläche, einem porösen Träger, einem porösen Metall, einer optischen Faser, einem Träger, der von einer Glasfaser abgeleitet ist, Siliciumdioxid, einer funktionellen Lipidmembran, einem Liposom, einer Filtrationsmembran oder Kügelchen oder Partikeln mit einer Größe kleiner als 1 μ bestellt.

7. Kit gemäß einem der Ansprüche 1 bis 6, **dadurch gekennzeichnet, dass** ein gleiches "Reportermolekül" mit jeder Sonde hybridisieren kann.

8. Kit gemäß einem der Ansprüche 1 bis 7, **dadurch gekennzeichnet, dass** alle perfekten Hybride, die zwischen den zielspezifzschen Sequenzen der Sonden und den Zielmolekülen, gebildet werden, eine gleiche Schmelztemperatur, bis zu etwa plus oder minus 4°C, vorzugsweise bis zu etwa plus oder minus 1 °C, besitzen.

9. Kit gemäß einem der Ansprüche 1 bis 8, **dadurch gekennzeichnet, dass** die Different zwischen der Schmelztemperatur des Hybrids, das zwischen der zielspezifischen Sequenz jeder Sonde und dem Zielmolekül gebildet wird, und der Schmelztemperatur für die Assoziation zwischen der zielspezifischen Sequenz mit einem Molekül, für das die zielspezifische Sequenz der Sonde nicht konstruiert worden ist, größer oder gleich 5°C, vorzugsweise größer oder gleich 8°C, ist.

10. Kit gemäß einem der Ansprüche 1 bis 9, **dadurch gekennzeichnet, dass** die Dtm von wenigstens zwei Sonden (Differenz zwischen Schmelztemperatur (Tm) des perfekten Hybrids, das zwischen der zielspezifischen Sequenz der Sonde und dem Zielmolekül gebildet wird, und der Schmelztemperatur (Tm) des Hybrids, das zwischen dem ersten Arm und dem zweiten Arm der Sonde gebildet wird) gleich, bis zu etwa plus oder minus 1°C, sind.

11. Kit gemäß einem der Ansprüche 1 bis 10, umfassend außerdem einen Satz von nicht markierten und nicht immobilisierten Sonden, umfassend

   (a) eine zielspezifische Sequenz mit einer Länge von 6 bis 30 Nukleotiden;
   (b) einen ersten Arm mit einer Länge von wenigstens 10 Nukleotiden, der 5' zur zielspezifischen Sequenz positioniert ist; und
   (c) einen zweiten Arm mit einer Länge von wenigstens 10 Nukleotiden, der 3' zur zielspezifischen Sequenz positioniert ist,

   wobei die zielspezifische Sequenz zu keinem anderen Abschnitt der Sonde komplementär ist; der erste Arm und der zweite Arm zueinander perfekt komplementär sind.

12. Kit gemäß einem der Ansprüche 1 bis 11, **dadurch gekennzeichnet dass** der nachweisbare Marker ein Nukleotid-Analogon, ein Fluoreszenz-Marker, Biotin, Imminobiotin, ein Antigen, ein Cofaktor, Dinitrophenol, Liponsäure, eine Olefinverbindung, ein Polypeptid, ein elektronenreiches Molekül, ein Enzym oder ein radioaktives Isotop ist.

13. Verfahren zum Nachweis von Nukleinsäure, umfassend:

   (a) Inkontaktbringen *ex vivo* einer Probe von Nukleinsäuren mit einem Biochip, wie er in dem Kit gemäß einem der Ansprüche 1 bis 12 definiert ist; und
   (b) Nachweis eines Signals, das von wenigstens einer Sonde des Biochips stammt, die eine offene Konformation nach dem bei Schritt (a) erfolgten Kontakt eingenommen hat, mithilfe eines "Reportermoleküls", wie es in dem Kit gemäß einem der Ansprüche 1 bis 12 definiert ist.

14. Verfahren zum Nachweis einer Genvariante *ex vivo* in einer Nukleinsäure-Probe, umfassend:

   (a) Inkontaktbringen der Probe mit einem Biochip, wie er in dem Kit gemäß einem der Ansprüche 1 bis 12 definiert ist, **dadurch gekennzeichnet, dass** wenigstens eine Sonde des Biochips eine für eine Genvariante spezifische Sonde ist, und
   (b) Nachweis eines Signals, das von der für die Genvariante spezifschen Sonde stammt, mithilfe eines "Reportermoleküls", wie es in dem Kit gemäß einem der Ansprüche 1 bis 12 definiert ist, wobei das in Schritt (b) nachgewiesen Signal ein Hinweis auf das Vorliegen der Genvariante in der Nukleinsäure-Probe ist.

15. Verfahren gemäß Anspruch 14, **dadurch gekennzeichnet, dass** die nachgewiesene Genvariante ein Einzel-Nukleotid-Polymorphismus ist.

16. Verfahren zum Nachweis *ex vivo* eines beliebigen Organismus, der Nukleinsäuren enthält, oder eines beliebigen Rests dieser Organismen, umfassend:

   (a) Inkontaktbringen einer Nukleinsäure-Probe mit einem Biochip, wie er in dem Kit gemäß einem der Ansprüche 1 bis 12 definiert ist, wobei wenigstens eine besagter Sonden eine Sonde ist, die für Nukleinsäuren eines Organismus oder von Resten dieses Organismus spezifisch ist; und
   (b) Nachweis eines Signal, das von der Sonde stammt, die für Nukleinsäuren eines Organismus spezifisch ist, mithilfe eines "Reportermoleküls", wie es in dem Kit gemäß einem der Ansprüche 1 bis 12 definiert ist,

   wobei das in Schritt (b) nachgewiesene Signal ein Hinweis auf das Vorhandensein des Organismus, der Nukleinsäuren enthält, oder von Resten dieses Organismus ist.

17. Verfahren gemäß Anspruch 16, **dadurch gekennzeichnet, dass** der Organismus, der Nukleinsäuren enthält, ein Virus oder ein Bakterium ist.

**18.** Verfahren zum Nachweis eines alternativen Spleißprodukts eines Gens *ex vivo* in einer Probe von Nukleinsäuren, umfassend:

(a) Inkontaktbringen der Probe mit einem Biochip, wie er in dem Kit gemäß einem der Ansprüche 1 bis 12 definiert ist, **dadurch gekennzeichnet, dass** wenigstens eine Sonde des Biochips eine Haarnadelsonde ist, die für ein Exon des Gens oder eine Verbindung von zwei Exons spezifisch ist; und
(b) Nachweis eines Signals, das von der Sonde stammt, die für das Exon oder die Verbindung von zwei Exons spezifisch ist, mithilfe eines "Reportermoleküls", wie es in dem Kit gemäß einem der Ansprüche 1 bis 12 definiert ist, wobei das in Schritt (b) nachgewiesene Signal ein Hinweis auf das Vorliegen des alternativen Spleißprodukts des Gens in der Probe von Nukleinsäuren ist.

**19.** Verfahren gemäß Anspruch 18, **dadurch gekennzeichnet, dass** die Nukleinsäure-Probe mRNA oder cDNA umfasst.

**20.** Ein Verfahren zur Sequenzierung eines Oligonukleotid *ex vivo*, umfassend:

(a) Inkontaktbringen einer Probe, die das Oligonukleotid enthält, mit einem Biochip, wie er in dem Kit gemäß einem der Ansprüche 1 bis 12 definiert ist; und
(b) Nachweis eines Signals, das von wenigstens einer Sonde des Biochips stammt, mithilfe eines "Reportermoleküls", wie es in dem Kit gemäß einem der Ansprüche 1 bis 12 definiert ist;

wobei das in Schritt (b) nachgewiesen Signal für die Bestimmung der Sequenz des Oligonukleotids verwendet wird.

**21.** Verfahren gemäß einem der Ansprüche 13 bis 20, in dem die Probe von Schritt (a) zuvor mit einem nachweisbaren Marker markiert wird.

**22.** Verfahren zum Nachweis von allelischen Ungleichgewichten und Heterozygotieverlusten ex vivo in einer Nukleinsäure-Probe, umfassend:

(a) Amplifikation wenigstens einer chromosomaler Mikrosatelliten-DNA-Region von wenigstens zwei Nukleinsäure-Proben, die aus Geweben oder biologischen Flüssigkeiten stammen, mithilfe eines Primer-Paares, wobei wenigstens eine der Proben von Zellen oder Geweben stammt, die kein allelisches Ungleichgewicht oder keinen Heterozygotieverlust aufweisen, und wobei jedes Gewebe oder jede Flüssigkeit im Verlauf der Amplifikation unterschiedlich markiert wird;
(b) Entfernen dieser Primer nach der Amplifikation;
(c) Inkontaktbringen besagter Amplifikationsprodukte mit einem Biochip, wie er in dem Kit gemäß einem der Ansprüche 1 bis 12 definiert ist, wobei darunter wenigstens eine Sonde des Biochips zu einem Primer komplementär ist, der für die Amplifikation besagter chromosomaler DNA-Region verwendet wird; und
(d) Nachweis der Signale, die von wenigstens einer Sonde besagten Biochips stammen, mithilfe eines "Reportermoleküls", wie es in dem Kit gemäß einem der Ansprüche 1 bis 12 definiert ist;

wobei die in Schritt (d) nachgewiesenen Signale für die Bestimmung des Vorliegens eines allelischen Ungleichgewichts oder eines Heterozygotieverlustes in einer der Nukleinsäure-Proben verwendet werden.

FIG. 1A

FIG. 1B

**FIG. 2A**

**FIG. 2B**

**FIG. 3A**

**FIG. 3B**

**FIG. 4A**

**FIG. 4B**

**FIG. 4C**

**FIG. 4D**

40

401

402

403

404

405

406

407

EP 1 601 798 B1

FIG. 5A

FIG. 5B

FIG 5C

Région déletée ( 604)

ARNm1 (603)

ARNm2 (605)

Séquence spécifique de la cible de la 1$^{ère}$ sonde en épingle à cheveux (601)

Séquence spécifique de la cible de la 2$^{ème}$ sonde en épingle à cheveux (602)

**FIG. 6**

**A)**

241 atcaagggtt acatgcagca acacaacatc ccccag [aggg aggtggtcga tgtcaccggcc tgaaccagt cgcacctctc ccagcatctc aacaagggca cccctatgaa] gacccagaag 360
(SEQ. NO.7) sequence entre crochet mise en gras

**B)**

241 atcaagggtt acatgcagca acacaacatc ccccag | gacc cagaagcgtg    290  (SEQ. NO.8)

**C)**

Tg2X2C137Ml: Cy5 –5' -cac gct tct ggg tcc tgg ggg atg- 3'    (SEQ. NO.9)

**D)**

2X2C137Ml:    5' -gcg agc ccc cca gga ccc aga agc gct cgc- 3'  (SEQ. NO.10)

**E)**

Molécule Rapporteur   Cy3 -5' -gcgagc -3' (SEQ. NO.11)

**FIG. 7**

FIG. 8

EP 1 601 798 B1

FIG. 9

**FIG. 10**

FIG. 11

**FIG. 12**

**FIG. 13A**

EP 1 601 798 B1

**FIG. 13B**

FIG. 14

EP 1 601 798 B1

FIG. 15

68

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 5118801 A
- US 5925517 A
- EP 0745690 A
- US 5569588 A, Ashby
- US 6270961 B
- US 6025136 A
- US 6018041 A
- US 5871928 A
- US 5695940 A
- US 5578832 A
- US 5556752 A

- US 5510270 A
- US 5445934 A
- US 5744305 A
- US 6040138 A
- WO 9841531 A
- US 6028189 A
- US 5891636 A
- US 5716785 A
- US 5545522 A
- US 6132997 A
- FR 0303112

### Littérature non-brevet citée dans la description

- **TYAGI et al.** *Nat. Biotechnol.,* 1996, vol. 14, 303-308
- **TYAGI et al.** *Nat. Biotechnol.,* 1998, vol. 16, 49-53
- **MATSUO T.** *Biochimica Biophysica Acta,* 1998, vol. 1379, 178-184
- **SOKOL et al.** *Proc. Natl. Acad. Sci. USA,* 1998, vol. 95, 11538-11543
- **LEONE et al.** *Nucleic Acids Res.,* 1998, vol. 26, 2150-2155
- **PIATEK et al.** *Nat. Biotechnol.,* 1998, vol. 16, 359-363
- **KOSTRIKIS et al.** *Science,* 1998, vol. 279, 1228-1229
- **GIESENDORF et al.** *Clin. Chem.,* 1998, vol. 44, 482-486
- **MARRAS et al.** *Genet. Anal.,* 1999, vol. 14, 151-156
- **VET et al.** *Proc. Natl. Acad. Sci. USA,* 1999, vol. 96, 6394-6399
- **SCHENA et al.** *Science,* 1995, vol. 270, 467-470
- **LOCKHART et al.** *Nat. Biotechnology,* 1996, vol. 14, 1675-1680
- **BLANCHARD et al.** *Nat. Biotechnology,* 1996, vol. 14, 1649
- **DERISI et al.** *Nature genetics,* 1996, vol. 14, 457-460
- **SHALON et al.** *Genome Res.,* 1996, vol. 6, 689-645
- **SCHENA et al.** *Proc. Natl. Acad. Sci. U.S.A.,* 1995, vol. 93, 10539-11286
- **DUGGAN et al.** *Nature Genetics,* 1996, 10-14

- **NICOLOSO, M. et al.** *Biochemical and Biophysical Research Communications,* 1989, vol. 159, 1233-1241
- **VERNIER, P. et al.** *Analytical Biochemistry,* 1996, vol. 235, 11-19
- **DERISI et al.** *Nature Genetics,* 1996, vol. 14, 457-460
- **DUGGAN et al.** *Nature Genetics,* 1996, vol. 21, 10-14
- **FODOR et al.** *Science,* 1991, vol. 251, 767-773
- **PEASE et al.** *Proc. Natl. Acad. Sci. U.S.A.,* 1994, vol. 91, 5022-5026
- **LOCKHART et al.** *Nature Biotechnology,* 1996, vol. 14, 1675-1680
- **BLANCHARD et al.** *Biosensors and Bioelectronics,* 1996, vol. 11, 687-690
- **BLANCHARD.** Synthetic DNA Arrays in Genetic Engineering. Plenum Press, 1998, vol. 20, 111-123
- **SCHÜTZ, E. et al.** *Biotechniques,* 1999, vol. 27, 1218-1224
- **ALLAWI H.T. et al.** *Biochemistry,* 1998, vol. 37 (8), 2170-9
- **ALLAWI H.T. et al.** *Biochemistry,* 1997, vol. 36, 10581-94
- **PEYRET N. et al.** *Biochemistry,* 1999, vol. 38, 3468-77
- Immobilized Biomolecules in Analysis, A practical approach. Oxford University Press, 1998
- **SHALON et al.** *Genome Res.,* 1996, vol. 6, 639-645
- **MASKOS ; SOUTHERN.** *Nucl. Acids. Res.,* 1992, vol. 20, 1679-1684

- **SAMBROOK et al.** Molecular Cloning : A Laboratory Manual. Cold Spring Harbor Laboratory, 1989, vol. 1-3
- **FANG, W. et al.** *Anal. Chem.,* 2000, vol. 72, 3280-5
- **BAR-ZIV, R. et al.** *P.N.A.S. U.S.A.,* 2001, vol. 98, 9068-73
- **HAMAGUCHI, N. et al.** *Anal. Biochem.,* 2001, vol. 294, 126-31
- PCR Protocols : A Guide to Methods and Applications. Academic Press Inc, 1990
- Molecular Cloning : A Laboratory Manual. Cold Spring Harbor Laboratory, 1989, vol. 1-3
- **CHIRGWIN et al.** *Biochemistry,* 1979, vol. 18, 5294-5299
- Current Protocols in MolecularBiology. Green Publishing Associates, Inc., and John Wiley&Sons, Inc, 1989, vol. I, 2.10.3
- Molecular Cloning : A Laboratory Manual. Cold Spring Harbor Laboratory Press, 1989, 9.47-9.5111.55-11.61
- Current Protocols in Molecular Biology. Green Publishing Associates, Inc., and John Wiley&Sons, Inc, 1989, vol. I, 2.10.1-2.10.16
- **COGOI, S et al.** *Biochemistry,* 2001, vol. 40, 1135-43
- **XODO, L. et al.** *Nucleic Acids Res.,* 1994, vol. 22, 3322-30
- **LAKEN, S.J. et al.** *Nat. Biotechnol.,* 1998, vol. 16, 1352-6
- **LITTLE, D. P. et al.** *Nat. Med.,* 1997, vol. 3, 1413-6
- **LITTLE, D. P.** *Eur. J. Clin. Chem. Clin. Biochem.,* 1997, vol. 35, 545-8
- **BRAUN, A. et al.** *Clin. Chem,* 1997, vol. 43, 1151-8
- **UMEK, R.M. et al.** *J.Mol.Diagn.,* 2001, vol. 3, 74-84
- **PADESTE, C. et al.** *Biosens.Bioelectron.,* 2000, vol. 15, 431-8
- **TSAI, W.C. et al.** *Analyst,* 1995, vol. 120, 2249-54
- **VALAT, C et al.** *Analytica Chimica Acta,* 2000, vol. 404, 187-94
- **OLIVER, B. et al.** *Anal. Chem.,* 1997, vol. 69, 4688-94
- **LIMOGES, B.** *Anal. Chem.,* 1996, vol. 68, 4141-48
- **BOURDILLON, C. et al.** *J. Am. Chem. Soc.,* 1996, vol. 115, 12264-69
- **SAMBROOK J. et al.** Molecular Cloning : A Laboratory Manual. Cold Spring Harbor Laboratory, 1989, 5.44-5.47
- **AUSUBEL F.M. et al.** Current Protocols in Molecular Biology. Green Publishing Associates, Inc., and John Wiley&Sons, Inc, 1997, vol. I, 3.5.11, 3.5.12
- **LINDER, T. et al.** *Hum. Molec.Genet.,* 1999, vol. 8, 2001-8
- **MATEO M. et al.** *Am. J. Path.,* 1999, vol. 154 (5), 1583-1589
- **LARSSON C.M. et al.** *Molecular Diagnosis.,* 2001, vol. 6 (3), 181-188
- Microsatellites: Evolution and Applications. Oxford University Press, 1999

SEQUENCE LISTING

<110> VALAT, CHRISTOPHE

<120> BIOPUCES A SONDES ET LEURS METHODES D'UTILI

<130> B0175wo

<150> FR 03 03112
<151> 2003-03-13

<160> 41

<170> PatentIn version 3.1

<210> 1
<211> 30
<212> DNA
<213> Artificial sequence

<220>
<223> hairpin probe

<220>
<221> stem_loop

<222> (1)..C6)
<223>

<220>
<221> probe_bind
<222> (7)..(24)
<223>

<220>
<221> stem_loop
<222> (25)..(30)
<223>

<400> 1
gcgagccaac cagtcccacc tgtcgctcgc          30

<210> 2
<211> 30
<212> DNA
<213> Artificial Sequence

<220>
<223> hairpin probe

<220>
<221> stem_loop
<222> (1)..(6)
<223>

<220>
<221> probe_bind
<222> (7)..(24)
<223>

<220>
<221> stem_loop
<222> (25)..(30)
<223>

<400> 2
gcgagccaac cagttccacc tgtcgctcgc          30

<210> 3
<211> 30
<212> DNA
<213> Artificial Sequence

<220>
<223> hairpin probe

<220>
<221> stem_loop
<222> (1)..(6)
<223>

<220>
<221> probe_bind
<222> (7)..(24)

<223>

<220>
<221> stem_loop
<222> (25)..(30)
<223>

<400> 3
gcgagccaac cagtgccacc tgtcgctcgc          30

<210> 4
<211> 30
<212> DNA
<213> Artificial Sequence

<220>
<223> hairpin probe

<220>
<221> stem_loop
<222> (1)..(6)
<223>

<220>
<221> probe_bind
<222> (7)..(24)
<223>

<220>
<221> stem_loop
<222> (25)..(30)
<223>

<400> 4
gcgagccaac cagtaccacc tgtcgctcgc          30

<210> 5
<211> 42
<212> DNA
<213> Artificial Sequence

<220>
<223> oligonucleotide target

<400> 5
aggtgttggg acaggtggga ctggttgagg ccagtggtat cg          42

<210> 6
<211> 42
<212> DNA
<213> Artificial Sequence

<220>
<223> oligonucleotide target

<400> 6
aggtgttggg acaggtggaa ctggttgagg ccagtggtat cg          42

<210> 7
<211> 120
<212> DNA
<213> Homo sapiens

<400> 7

```
atcaagggtt acatgcagca acacaacatc ccccagaggg aggtggtcga tgtcaccggc      60

ctgaaccagt cgcacctctc ccagcatctc aacaagggca cccctatgaa gacccagaag     120
```

<210> 8
<211> 50
<212> DNA
<213> Homo sapiens

<400> 8
atcaagggtt acatgcagca acacaacatc ccccaggacc cagaagcgtg          50

<210> 9
<211> 24
<212> DNA
<213> Artificial sequence

<220>
<223> oligonucleotide target

<400> 9
cacgcttctg ggtcctgggg gatg 24

<210> 10
<211> 30
<212> DNA
<213> Artificial Sequence

<220>
<223> hairpin probe

<220>
<221> stem_loop
<222> (1)..(6)
<223>

<220>
<221> stem_loop
<222> (25)..(30)
<223>

<220>
<221> probe_bind
<222> (7)..(24)
<223>

<400> 10
gcgagccccc caggacccag aagcgctcgc          30

<210> 11
<211> 6

<212> DNA
<213> Artificial sequence

<220>
<223> probe

<400> 11
gcgagc          6

<210> 12
<211> 35
<212> DNA
<213> Artificial sequence

<220>
<223> hairpin probe

<220>
<221> stem_loop
<222> (1)..(6)
<223>

<220>
<221> stem_loop
<222> (30)..(35)
<223>

<220>
<221> probe_bind
<222> (7)..C29)
<223>

<400> 12
gcgagcgaat aagaagtagg ctggtgagcg ctcgc          35

<210> 13
<211> 35
<212> DNA
<213> Artificial sequence

<220>
<223> hairpin probe

<220>
<221> stem_loop
<222> (1)..(6)
<223>

<220>
<221> stem_loop
<222> (30)..(35)
<223>

<220>
<221> probe_bind
<222> (7)..(29)
<223>

<400> 13
gcgagcgaat aagaagtagg ctggtgagcg ctcgc        35


<210> 14
<211> 30
<212> DNA
<213> Artificial sequence


<220>
<223> hairpin probe


<220>
<221> stem_loop
<222> (1)..(6)
<223>


<220>
<221> stem_loop
<222> (25)..(30)
<223>


<220>
<221> probe_bind
<222> (7)..(24)
<223>


<400> 14
gcgagccacc agagggtccc ctgggctcgc        30


<210> 15
<211> 30
<212> DNA
<213> Artificial sequence


<220>
<223> hairpin probe


<220>
<221> stem_loop
<222> (1)..(6)
<223>


<220>
<221> stem_loop
<222> (25)..(30)
<223>


<220>
<221> probe_bind
<222> (7)..(24)
<223>


<400> 15
gcgagccacc agagagtccc ctgggctcgc        30


<210> 16
<211> 31
<212> DNA

<213> Artificial sequence

<220>
<223> hairpin probe

<220>
<221> stem_loop
<222> (1)..(6)
<223>

<220>
<221> stem_loop
<222> (26)..(31)
<223>

<220>
<221> probe_bind
<222> (7)..(25)
<223>

<400> 16
gcgagctttt gacagggggt gaagggctcg c          31

<210> 17
<211> 31
<212> DNA
<213> Artificial Sequence

<220>
<223> hairpin probe

<220>
<221> stem_loop
<222> (1)..(6)
<223>

<220>
<221> stem_loop
<222> (26)..(31)
<223>

<220>
<221> probe_bind
<222> (7)..(25)
<223>

<400> 17
gcgagctttt gacagggggt gaagggctcg c          31

<210> 18
<211> 30
<212> DNA
<213> Artificial sequence

<220>
<223> hairpin probe

<220>

<221> stem_loop
<222> (1)..(6)
<223>

<220>
<221> stem_loop _
<222> (25)..(30)
<223>

<220>
<221> probe_bind
<222> (7)..(24)
<223>

<400> 18
gcgagcccga gtccgtccag agctgctcgc     30

<210> 19
<211> 30
<212> DNA
<213> Artificial Sequence

<220>
<223> hairpin probe

<220>
<221> stem_loop
<222> (1)..(6)
<223>

<220>
<221> stem_loop
<222> (25)..(30)
<223>

<220>
<221> probe_bind
<222> (7)..(24)
<223>

<400> 19
gcgagcccga gtccatccag agctgctcgc     30

<210> 20
<211> 29
<212> DNA
<213> Artificial sequence

<220>
<223> hairpin probe

<220>
<221> stem_loop
<222> (1)..(6)
<223>

<220>
<221> stem_loop

<222> (24)..(29)
<223>

<220>
<221> probe_bind
<222> (7)..(23)
<223>

<400> 20
gcgagccaac cactccacct gtcgctcgc          29

<210> 21
<211> 29
<212> DNA
<213> Artificial sequence

<220>
<223> hairpin probe

<220>
<221> stem_loop
<222> (1)..(6)
<223>

<220>
<221> stem_loop
<222> (24)..(29)
<223>

<220>
<221> probe_bind
<222> (7)..(23)
<223>

<400> 21
gcgagccaac cattccacct gtcgctcgc          29

<210> 22
<211> 29
<212> DNA
<213> Artificial Sequence

<220>
<223> hairpin probe

<220>
<221> stem_loop
<222> (1)..(6)
<223>

<220>
<221> stem_loop
<222> (24)..(29)
<223>

<220>
<221> probe_bind
<222> (7)..(23)

<223>

<400> 22
gcgagccaac cagtccacct gtcgctcgc     29

<210> 23
<211> 29
<212> DNA
<213> Artificial sequence

<220>
<223> hairpin probe

<220>
<221> stem_loop
<222> (1)..(6)
<223>

<220>
<221> stem_loop
<222> (24)..(29)
<223>

<220>
<221> probe_bind
<222> (7)..(23)
<223>

<400> 23
gcgagccaac caatccacct gtcgctcgc     29

<210> 24
<211> 30
<212> DNA
<213> Artificial sequence

<220>
<223> hairpin probe

<400> 24
gcgagcagta tggctctcgg tcatgctcgc     30

<210> 25
<211> 30
<212> DNA
<213> artificial sequence

<220>
<223> hairpin probe

<400> 25
gcgagcagta tggctcttgg tcatgctcgc     30

<210> 26
<211> 31
<212> DNA
<213> artificial sequence

<220>
<223> hairpin probe

<400> 26
gcgagcgctc cattgtcata cacaagctcg c          31

<210> 27
<211> 31
<212> DNA
<213> artificial sequence

<220>
<223> hairpin probe

<400> 27
gcgagcgctc cattttcata cacaagctcg c          31

<210> 28
<211> 29
<212> DNA
<213> artificial sequence

<220>
<223> hairpin probe

<400> 28
gcgagccaat ggcacgttca cttgctcgc          29

<210> 29
<211> 27
<212> DNA
<213> artificial sequence

<220>
<223> hairpin probe

<400> 29
gcgagccaga actgtagcac cgctcgc          27

<210> 30
<211> 27
<212> DNA
<213> Artificial sequence

<220>
<223> hairpin probe

<400> 30
gcgagccaga accgtagcac cgctcgc          27

<210> 31
<211> 26
<212> DNA
<213> artificial sequence

<220>
<223> hairpin probe

<400> 31

gcgacgtcct ggcctgggtg cgtcgc       26

<210> 32
<211> 26
<212> DNA
<213> artificial sequence

<220>
<223> hairpin probe

<400> 32

gcgacgtcct ggcccgggtg cgtcgc       26

<210> 33
<211> 30
<212> DNA
<213> artificial sequence

<220>
<223> oligonucleotide target

<400> 33

atgaagatga ccgagagcca tactcggcca       30

<210> 34
<211> 30
<212> DNA
<213> artificial sequence

<220>
<223> oligonucleotide target

<400> 34

tctaggttgt gtatgacaat ggagccatcc       30

<210> 35
<211> 30
<212> DNA
<213> artificial sequence

<220>
<223> oligonucleotide target

<400> 35

cgtcacaagt gaacgtgcca ttgtcactgt       30

<210> 36
<211> 26
<212> DNA
<213> artificial sequence

<220>
<223> oligonucleotide target

<400> 36

gaagaggtgc tacagttctg ccagag       26

<210> 37
<211> 26
<212> DNA
<213> artificial sequence

<220>
<223> oligonucleotide target

<400> 37
gaaggccacc caggccagga tgtagg          26

<210> 38
<211> 30
<212> DNA
<213> artificial sequence

<220>
<223> oligonucleotide target

<400> 38
atgaagatga ccaagagcca tactcggcca          30

<210> 39
<211> 30
<212> DNA
<213> artificial sequence

<220>
<223> oligonucleotide target

<400> 39
tctaggttgt gtatgaaaat ggagccatcc          30

<210> 40
<211> 26
<212> DNA
<213> artificial sequence

<220>
<223> oligonucleotide target

<400> 40
gaagaggtgc tacggttctg ccagag          26

<210> 41
<211> 26
<212> DNA
<213> artificial sequence

<220>
<223> oligonucleotide target

<400> 41
gaaggccacc cgggccagga tgtagg          26